# EUROPEAN PATENT APPLICATION

(11) **EP 4 818 145 A2**
(43) Date of publication of application: **07.10.2026**
(21) Application number: 26200334.6
(22) Date of filing: 09.07.2024
(51) Int. Cl.: C07C 21/18

(54) **PROCESSES TO PRODUCE 1,1-DIFLUOROPROPENE (HFO-1252ZC), COMPOSITIONS AND INTERMEDIATES THEREOF**

(30) Priority: 17.07.2023 US 202363527096 P; 14.03.2024 US 202463565026 P
(62) Document number(s) of the earlier application(s) in accordance with Art. 76 EPC: 24746895.2 / 4 747 219
(71) Applicant: The Chemours Company FC, LLC, Wilmington, Delaware 19801 (US)
(72) Inventor: SUN, Xuehui, Kennett Square, Pennsylvania 19348 (US); JACKSON, Andrew, Newark, Delaware 19711 (US)
(74) Representative: Mewburn Ellis LLP

(57) **Abstract**

Provided herein are processes for producing difluoroolefins, particularly difluoropropenes such as 1,1-difluoropropene, as well as intermediates, compositions and uses thereof.

## Description

### CROSS REFERENCE TO RELATED APPLICATIONS

This application claims the benefit of priority of U.S. Provisional Application 63/527,096 filed July 17, 2023, and U.S. Provisional Application 63/565,026 filed March 14, 2024, the disclosure of each of which is incorporated herein by reference it its entirety.

### FIELD OF THE INVENTION

The present invention is directed to processes for producing difluoroolefins, particularly difluoropropenes, intermediates, compositions and uses thereof.

### BACKGROUND OF THE INVENTION

The fluorocarbon industry has been working for the past few decades to find replacement refrigerants for the ozone depleting chlorofluorocarbons (CFCs) and hydrochlorofluorocarbons (HCFCs) being phased out as a result of the Montreal Protocol. The solution for many applications has been the commercialization of hydrofluorocarbon (HFC) compounds for use as refrigerants, solvents, fire extinguishing agents, blowing agents and propellants. These new compounds, such as HFC refrigerants, HFC-134a and HFC-125 being the most widely used at this time, have zero ozone depletion potential (ODP) and thus are not affected by the current regulatory phase-out as a result of the Montreal Protocol. In addition to ozone depleting concerns, global warming is another environmental concern in many of these applications. HFC refrigerants such as HFC-134a and HFC-125 respectively have global warming potentials (GWP) of 1,300 and 3,170 according to the UN's IPCC Fifth Assessment Report (AR5).

This regulatory landscape is continuously evolving, taking into consideration properties beyond just ODP and GWP. More particularly, there is a need for refrigerant compositions that not only meet low ODP standards and have low global warming potentials, but that also exhibit low or no flammability, provide superior performance in a variety of applications and which meet the standards of evolving regulations.

There is a need in this art for new refrigerants that meet evolving regulations as well as provide heat transfer and refrigerant characteristics that meet or exceed the effectiveness of conventional refrigerants.

Some fluoropropenes, such as 1,1-difluoropropene (HFO-1252zc), are such potential new refrigerants. There continues to be need for effective and efficient processes for preparing 1,1-difluoropropene (HFO-1252zc) and intermediates and compositions thereof.

### SUMMARY OF THE INVENTION

The present invention relates to processes for producing 1,1-difluoropropene (HFO-1252zc, CF₂=CHCH₃) from 1,1,1,3-tetrachloropropane (HCC-250fb, C₃H₄Cl₄), and compositions thereof.

One embodiment of the invention disclosed herein relates to a process of converting HCC-250fb to HFO-1252zc through intermediates including, but not limited to, one of more of 1,3-dicloro-1,1-difluoropropane (HCFC-252fc, C₃H₄Cl₂F₂); 1-chloro-1,1-difluoropropane (HCFC-262fc, CH₃CH₂CCIF₂); and 3-chloro-3,3-difluoro-1-propene (HCFO-1242zf, C₃H₃ClF₂).

In one embodiment, conversion of HCC-250fb to HFO-1252zc proceeds through the intermediate HCFC-252fc.

In one embodiment, conversion of HCC-250fb to HFO-1252zc proceeds through the intermediate HCFC-262fc.

In one embodiment, conversion of HCC-250fb to HFO-1252zc proceeds through the intermediates HCFC-252fc and HCFC-262fc.

One embodiment of the invention disclosed herein relates to a process of converting HCC-250fb to HCFC-262fc through the intermediate HCFC-252fc.

One embodiment of the invention disclosed herein relates to a process of converting HCFC-252fc to HFO-1252zc through the intermediate HCFC-262fc.

One embodiment of the invention disclosed herein relates to a process of converting HCFC-252fc to HCFC-262fc.

One embodiment of the invention disclosed herein relates to a process of converting HCFC-262fc to HFO-1252zc.

One embodiment of the invention disclosed herein relates to a process of converting HCC-250fb to HCFC-252fc by contacting HCC-250fb with hydrogen fluoride, preferably in the presence of a catalyst and preferably in the liquid phase, to form the HCFC-252fc.

In one embodiment, the present invention provides a process of converting HCC-250fb to HFO-1252zc by contacting HCC-250fb with hydrogen fluoride in the presence of a catalyst to form HCFC-252fc, contacting the HCFC-252fc with hydrogen in the presence or absence of a catalyst to form HCFC-262fc, and dehydrohalogenation the HCFC-262fc in the presence or absence of a catalyst to form HFO-1252zc.

In one embodiment, the present invention provides a process of converting HCC-250fb to HCFC-262fc by contacting HCC-250fb with hydrogen fluoride in the presence of a catalyst to form HCFC-252fc and contacting the HCFC-252fc with hydrogen in the presence or absence of a catalyst to form HCFC-262fc.

In one embodiment, the present invention provides a process of converting HCFC-252fc to HFO-1252zc by contacting HCFC-252fc with hydrogen in the presence or absence of a catalyst to form HCFC-262fc, and dehydrohalogenation (dehydrochlorination) of the HCFC-262fc in the presence of a catalyst to form HFO-1252zc.

In one embodiment, the present invention provides a process of converting HCFC-252fc to HCFC-262fc by contacting the HCFC-252fc with hydrogen in the presence of a catalyst or absence to form HCFC-262fc, in either the liquid or vapor phase.

In one embodiment, the present invention provides a process of converting HCFC-262fc to HFO-1252zc by dehydrohalogenation (dehydrochlorination) of the HCFC-262fc in the presence or absence of a catalyst to form HFO-1252zc, in either the liquid or vapor phase.

In one embodiment, conversion of HCC-250fb to HFO-1252zc proceeds through the intermediate HCFO-1242zf.

In one embodiment, conversion of HCC-250fb to HFO-1252zc proceeds through the intermediates HCFO-1242zf and HCFC-262fc.

One embodiment of the invention disclosed herein relates to a process of converting HCC-250fb to HCFC-262fc through the intermediate HCFO-1242zf.

One embodiment of the invention disclosed herein relates to a process of converting HCFO-1242zf to HFO-1252zc through the intermediate HCFC-262fc.

One embodiment of the invention disclosed herein relates to a process of converting HCFO-1242zf to HCFC-262fc.

One embodiment of the invention disclosed herein relates to a process of converting HCFO-1242zf to HFO-1252zc.

In one embodiment, the present invention provides a process of converting HCC-250fb to HFO-1252zc by contacting HCC-250fb with hydrogen fluoride in the presence or absence of a catalyst to form HCFO-1242zf, contacting the HCFO-1242zf with hydrogen in the presence of a catalyst to form HCFC-262fc, and dehydrochlorinating (dehydrohalogenating) the HCFC-262fc in the presence or absence of a catalyst to form HFO-1252zc.

In one embodiment, the present invention provides a process of converting HCC-250fb to HCFC-262fc by contacting HCC-250fb with hydrogen fluoride in the presence or absence of a catalyst to form HCFO-1242zf, contacting the HCFO-1242zf with hydrogen in the presence of a catalyst to form HCFC-262fc.

In one embodiment, the present invention provides a process of converting HCFO-1242zf to HFO-1252zc by contacting HCFO-1242zf with hydrogen in the presence of a catalyst to form HCFC-262fc, and dehydrochlorinating (dehydrohalogenating) the HCFC-262fc in the presence or absence of a catalyst to form HFO-1252zc.

In one embodiment, the present invention provides a process of converting HCFO-1242zf to HCFC-262fc by contacting the HCFO-1242zf with hydrogen in the presence of a catalyst to form HCFC-262fc, in either the liquid or vapor phase.

In one embodiment, the present invention provides a process of converting HCC-250fb to HCFC-252fc by contacting HCC-250fb with hydrogen fluoride in the liquid phase in the presence of a catalyst.

In one embodiment, the present invention provides a process of converting HCC-250fb to HCFO-1242zf by contacting HCC-250fb with hydrogen fluoride in the vapor phase in the absence or presence of a catalyst.

In one embodiment, the present invention provides a process of converting HCC-250fb to HFO-1243zf by contacting HCC-250fb with hydrogen fluoride in the vapor phase in the absence or presence of a catalyst.

In one embodiment, the present invention provides a process of converting HCC-250fb to HFO-1252zc by contacting HCC-250fb with hydrogen fluoride in the presence or absence of a catalyst to form HCFO-1242zf and dechlorinating the HCFO-1242zf with zinc in the presence or absence of a catalyst to form HFO-1252zc.

In one embodiment, conversion of HCC-250fb to HFO-1252zc first proceeds by contacting HCC-250fb with hydrogen fluoride in the presence or absence of a catalyst to form one of HCFC-252fc or HCFO-1242zf, and then either contacting the HCFC-252fc or HCFO-1242zf with hydrogen in the presence or absence of a catalyst to form HCFC-262fc or contacting the HCFO-1242zf with zinc in the presence or absence of a catalyst to form HFO-1252zc. In one embodiment, where HCFC-262fc is formed, the process further comprises dehydrohalogenation (dehydrochlorination) of the HCFC-262fc in the presence or absence of a catalyst to form HFO-1252zc. In one embodiment, the intermediate formed from the hydrofluorination of HCC-250fb is preferably HCFC-252fc. In another embodiment, the intermediate formed from the hydrofluorination of HCC-250fb is preferably HCFO-1242zf.

In certain embodiments disclosed herein, HFO-1252zc is prepared according to the following Reaction Scheme A:
**Step 1A:**

   CCl₃CH₂CH₂Cl (HCC-250fb) + HF → CClF₂CH₂CH₂Cl (HCFC-252fc) + HCl
**Step 2A:**

   CClF₂CH₂CH₂Cl (HCFC-252fc) + H₂ → CClF₂CH₂CH₃ (HCFC-262fc) + HCl
**Step 3A:**

   CClF₂CH₂CH₃ (HCFC-262fc) → CF₂=CHCH₃ (HFO-1252zc) + HCl

   OR
   Sol

   CClF₂CH₂CH₃ (HCFC-262fc) + Strong Base → CF₂=CHCH₃ (HFO-1252zc)

In certain embodiments, Steps 1A, 2A and 3A comprise an integrated process for producing HFO-1252zc. In some embodiments, the integrated process further comprises separation steps or processes to recover the desired intermediate (e.g., HCFC-252fc and HCFC-262fc) and the desired reaction product HFO-1252zc.

In some embodiments, Step 1A may be omitted in order to prepare HCFC-262fc according to Step 2A and/or HFO-1252zc according to Steps 2A and 3A, utilizing HCFC-252fc as the starting material.

In some embodiments, Steps 1A and 2A may be omitted in order to prepare the HFO-1252zc utilizing HCFC-262fc as the starting material.

In some embodiments, the present invention relates to a process of preparing HCFC-262fc according to (i) Steps 1A and 2A, or (ii) Step 2A.

In certain embodiments disclosed herein, HFO-1252zc is prepared according to the following Reaction Scheme B:
**Step 1B:**

   CCl₃CH₂CH₂Cl (HCC-250fb) + HF → CClF₂CH=CH₂ (HCFO-1242zf) + HCl

   OR

   CClF₂CH₂CH₂Cl (HCFC-252fc) + Caustic → CClF₂CH=CH₂ (HCFO-1242zf) + H2O + Salt
**Step 2B:**

   CClF₂CH=CH₂ (HCFO-1242zf) + H₂ → CClF₂CH₂CH₃ (HCFC-262fc)
**Step 3B:**

   CClF₂CH₂CH₃ (HCFC-262fc) → CF₂=CHCH₃ (HFO-1252zc) + HCl

   OR
   Solv

   CClF₂CH₂CH₃ (HCFC-262fc) + Strong Base → CF₂=CHCH₃ (HFO-1252zc)

In certain embodiments, Steps 1B, 2B and 3B comprise an integrated process for producing HFO-1252zc. In some embodiments, the integrated process further comprises separation steps or processes to recover the desired intermediate (e.g., HCFO-1242zf and HCFC-262fc) and the desired reaction product HFO-1252zc.

In some embodiments, Step 1B may be omitted in order to prepare HCFC-262fc according to Step 2B and/or HFO-1252zc according to Steps 2B and 3B, utilizing HFO-1243zf as the starting material.

In some embodiments, Steps 1B and 2B may be omitted in order to prepare the HFO-1252zc utilizing HCFC-262fc as the starting material.

In some embodiments, the present invention relates to a process of preparing HCFC-262fc according to (i) Steps 1B and 2B, or (ii) Step 2B.

In certain embodiments disclosed herein, HFO-1252zc is prepared according to the following Reaction Scheme C:
**Step 1C:**

   CCl₃CH₂CH₂Cl (HCC-250fb) + HF → CClF₂CH=CH₂ (HCFO-1242zf) + HCl

   OR

   CClF₂CH₂CH₂Cl (HCFC-252fc) + Caustic → CClF₂CH=CH₂ (HCFO-1242zf) + H2O + Salt
**Step 2C:**

   CClF₂CH=CH₂ (HCFO-1242zf) + Zn → CF₂=CHCH₃ (HFO-1252zc) + ZnCl₂

In certain embodiments, Steps 1C and 2C comprise an integrated process for producing HFO-1252zc. In some embodiments, the integrated process further comprises separation steps or processes to recover the desired intermediate (e.g., HCFO-1242zf) and the desired reaction product HFO-1252zc.

In some embodiments, the present invention relates to a process of preparing HFO-1252zc from HCFO-1242zf according to Step 2C.

In one embodiment, one or more of the above steps may be integrated as follows to prepare HFO-1252zc (Reaction Scheme D):
**Step 1D:**

   CCl₃CH₂CH₂Cl (HCC-250fb) + HF → CClF₂CH₂CH₂Cl (HCFC-252fc) + HCl
**Step 2D:**

   CClF₂CH₂CH₂Cl (HCFC-252fc) + Caustic → CClF₂CH=CH₂ (HCFO-1242zf) +H2O +Salt
**Step 3D:**

   CClF₂CH=CH₂ (HCFO-1242zf) + Zn → CF₂=CHCH₃ (HFO-1252zc) + ZnCl₂

   OR

   CClF₂CH=CH₂ (HCFO-1242zf) + H₂ → CClF₂CH₂CH₃ (HCFC-262fc) → CF₂=CHCH₃ (HFO-1252zc) + HCl

   OR
   Base/Solv

   CClF₂CH=CH₂ (HCFO-1242zf) + H₂ → CClF₂CH₂CH₃ (HCFC-262fc) → CF₂=CHCH₃ (HFO-1252zc)

In certain embodiments disclosed herein, such as Reaction Schemes B and C, the HCC-250fb feed is first vaporized prior to hydrofluorination and the hydrofluorination reaction is carried out in the vapor phase. In another embodiment, such as Reaction Scheme A, hydrofluorination of the HCC-250fb is carried out in the liquid phase.

In certain embodiments disclosed herein, HCFC-252fc is converted to HCFC-262fc by hydrogenation in either a liquid phase or vapor phase.

In certain embodiments disclosed herein, HCFO-1242zf is converted to HCFC-262fc by hydrogenation in either a liquid phase or vapor phase.

In certain embodiments disclosed herein, HCFC-262fc is converted to HFO-1252zc by dehydrochlorination in the vapor phase in the presence or absence of a catalyst.

In certain embodiments disclosed herein, HCFC-262fc is converted to HFO-1252zc by dehydrochlorination in the liquid phase in the presence or absence of a catalyst.

In certain embodiments disclosed herein HCFO-1242zf is converted to HFO-1252zc by contact with zinc in the liquid phase.

Certain embodiments of the invention disclosed herein relate to compositions comprising, consisting essentially of, or consisting of HCFC-262fc and one or more additional compounds selected from HCC-250fb, HCFC-252fc, HCFO-1242zf, HFC-263fb, HCFC-272fb and HFO-1252zf. In some embodiments the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from HCC-250fb, HCFC-252fc, HFC-263fb, HCFC-262fa and HCFC-272fb. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from HCC-250fb, HCFO-1242zf, HFC-263fb, HFO-1252zf and HCFC-272fb. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCC-250fb and HCFO-1242zf. In some embodiments, the HCFO-1242zf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from propane, propylene, HCFC-252dc, HCFC-262db, HFC-272fb, HCFO-1232xf, HCFO-1242zf, HFO-1243zf, E-HCFO-1251zd, Z-HCFO-1251zd, HFO-1252zf, E-HFO-1261ze, Z-HFO-1261ze, HCFO-1242 isomer(s) and HCO-1260. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from propane, propylene, HCFC-252dc, HCFC-262db, HFC-272fb, HCFO-1232xf, HCFO-1242zf, HFO-1243zf, E-HCFO-1251zd, Z-HCFO-1251zd, HFO-1252zf, E-HFO-1261ze, Z-HFO-1261ze, and HCO-1260. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from propane, propylene, HCFC-252dc, HCFC-262db, HCFO-1232xf, HCFO-1242zf, HFO-1243zf, E-HCFO-1251zd, Z-HCFO-1251zd, HFO-1252zf, E-HFO-1261ze, Z-HFO-1261ze and HCFO-1242 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from HCFC-262db, HCFO-1232xf, HCFO-1242zf, HFO-1243zf, E-HCFO-1251zd, Z-HCFO-1251zd, HFO-1252zf and E-HFO-1261ze. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from ethane, HFC-152a, HFC-253db, HCFO-1232 isomer(s), HCFO-1242 isomer(s), HCFO-1242zf, HFO-1243zf and HFO-1252zc. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc and one or more additional compounds selected from propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc, HCFO-1242zf and one or more additional compounds selected from propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242 isomer(s), HCFO-1232 isomer(s) and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-262fc, HFC-272fb and one or more additional compounds selected from propane, propylene, E-HFO-1261ze, Z-HFO-1261ze, HFO-1252zc, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFO-1242zf and one or more additional compounds selected from HCC-250fb, HCFC-253fb, HCFC-252fc, HCFC-251fb, HCFC-1243zf, HCFC-1241zf and HCFC-1240za. In some embodiments, the HCFO-1242zf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HCFC-1243zf and one or more additional compounds selected from HCC-250fb, HCFC-253fb, HCFC-252fc, HCFC-251fb, HCFO-1242zf, HCFC-1241zf and HCFC-1240za. In some embodiments, the HCFO-1242zf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

Certain embodiments of the invention disclosed herein relate to compositions comprising, consisting essentially of, or consisting of HFO-1252zc and one or more additional compounds selected from HCC-250fb, HCFC-252fc, HCFC-262fc and HCFO-1242zf. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HFO-1252zc, HCFC-262fc, HCC-250fb and HCFC-252fc. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HFO-1252zc, HCFC-262fc, HCC-250fb and HCFO-1242zf. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HFO-1252zc and one or more additional compounds selected from HCFC-262fc, HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf, and HCFC-252fc. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween. In some embodiments, such compositions are formed by Reaction Schemes A or B.

In one embodiment, compositions according to the present invention comprise, consist essentially of, or consist of HFO-1252zc, HCC-250fb and HCFO-1242zf. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, compositions according to the present invention comprise, consist essentially of, or consist of HFO-1252zc and one or more additional compounds selected from HCFC-262fc, HCC-250fb, HFC-263fb, HCFO-1242zf, HFO-1252zf, HFO-1243zf and HCFC-252fc. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween. In some embodiments, such compositions are formed by Reaction Scheme C.

Certain embodiments of the invention disclosed herein relates to compositions comprising, consisting essentially of, or consisting further include one or more additional members comprising hydrofluorocarbons (HFCs), hydrochlorocarbons (HCCs), hydrofluorochlorocarbons (HCFCs), hydrofluoroolefins (HFOs), hydrochlorofluoroolefins (HCFOs), C₂-C₄ alkanes, C₂-C₄ alkenes and t-butoxy-fluoropropenes.

In a preferred embodiment, compositions according to the present invention are free of or substantially free of Group A Fluorinated Substances, as defined herein.

In a preferred embodiment, degradation products of compositions according to the present invention are free of or substantially free of Group A Fluorinated Substances, as defined herein.

Certain embodiments of the invention disclosed herein relate to compositions comprising, consisting essentially of, or consisting of HFO-1252zc and one or more additional members selected from HFCs, HCCs, HCFCs, HFOs, HCFOs, C₂-C₄ alkanes, C₂-C₄ alkenes and t-butoxy-fluoropropenes. In some embodiments, such compositions are free of or substantially free of Group A Fluorinated Substances and/or degradation products of such compositions are free of or substantially free of Group A Fluorinated Substances, as defined herein.

A further embodiment of the invention disclosed herein is a composition comprising, consisting essentially of, or consisting of HFO-1252zc. In some embodiments, the composition further comprises at least one additional member or compound, wherein the total amount of the additional members or compounds is between greater than 0 and less than about 60%, 55%, 50%, 45%, 40%, 35%, 30%, 25%, 20%, 15%, 14%, 13%, 12%, 11%, 10%, 9%, 8%, 7%, 6%, 5%, 4%, 3%, 2% or 1%, and all values and ranges therebetween.

A still further embodiment of the invention disclosed herein is a composition comprising, consisting essentially of, or consisting of HFO-1252zc. In some embodiments, and the composition further comprises at least one an additional member or compound, wherein the total amount of additional members or compounds is between greater than 0 and less than about 60%, 55%, 50%, 45%, 40%, 35%, 30%, 25%, 20%, 10%, between greater than 0.001% and less than 60%, 55%, 50%, 45%, 40%, 35%, 30%, 25%, 20%, 10%, 9%, 8%, 7%, 6%, 5%, 4%, 3%, 2% or 1%. And all values and ranges therebetween.

One embodiment of the invention disclosed herein is a composition comprising, consisting essentially of, or consisting of HFO-1252zc, wherein HFO-1252zc is present in an amount greater than greater than about 20 wt.%, greater than about 30 wt.%, between about 30 wt.% and about 99 wt.%, between about 40 wt.% and about 99 wt.%, between about 50 wt.% and about 99 wt.%, less than 100 wt.% and greater than about 90 wt.%, greater than about 95 wt.%, greater than about 99 wt.%, greater than about 99.3 wt.%, greater than about 99.5 wt.%, greater than about 99.6 wt.%, greater than 99.7 wt.%, greater than about 99.8 wt.% or greater than about 99.9 wt.% and all values and ranges therebetween.

In certain embodiments, liquid-phase hydrogenation of HCFC-252fc is conducted at a temperature between about 20°C to about 150°C, preferably about 30°C to about 100°C.

In certain embodiments, vapor-phase hydrogenation of HCFC-252fc is conducted at a temperature between about 20°C to about 120°C, preferably about 30°C to about 80°C_{.}

In certain embodiments, liquid-phase hydrogenation of HCFO-1242zf is conducted at a temperature between about 20°C to about 150°C, preferably about 30°C to about 100°C.

In certain embodiments, vapor-phase hydrogenation of HCFO-1242zf is conducted at a temperature between about 20°C to about 120°C, preferably about 30°C to about 80°C.

In certain embodiments, vapor-phase dehydrohalogenation of HCFC-262fc is conducted at a temperature between about 400°C to about 800°C, preferably about 450°C to about 700°C.

Unless otherwise defined, all technical and scientific terms used herein have the same meaning as commonly understood by one of ordinary skill in the art to which this invention belongs. In case of conflict, the present specification, including definitions, will control. Although methods and materials similar or equivalent to those described herein can be used in the practice or testing of embodiments of the present invention, suitable methods and materials are described below. In addition, the materials, methods, and examples are illustrative only and not intended to be limiting.

The various aspects and embodiments of the invention can be used alone or in combinations with each other. Other features and advantages of the present invention will be apparent from the following more detailed description, taken in conjunction with the accompanying drawings which illustrate, by way of example, the principles of the invention.

### BRIEF DESCRIPTION OF THE DRAWINGS

The following detailed description of preferred embodiments of the present invention will be better understood when read in conjunction with the appended drawings. For the purposes of illustrating the invention, there is shown in the drawings embodiments which are presently preferred. It is understood, however, that the invention is not limited to the precise arrangements and instrumentalities shown. In the drawings:
**Fig. 1** illustrates a system according to a first embodiment of the invention.
**Fig. 2** illustrates a system according to second embodiment of the invention.
**Fig. 3** illustrates a system according to third embodiment of the invention.

### DETAILED DESCRIPTION OF THE INVENTION

The present invention provides processes for preparing 1,1-difluoropropene (HFO-1252zc) as well as intermediates and compositions thereof.

As used herein, the terms "comprises," "comprising," "includes," "including," "has," "having" or any other variation thereof, are intended to cover a non-exclusive inclusion. For example, a composition, process, method, article, or apparatus that comprises a list of elements is not necessarily limited to only those elements but may include other elements not expressly listed or inherent to such composition, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

The transitional phrase "consisting of" excludes any element, step, or ingredient not specified. If in the claim such would close the claim to the inclusion of materials other than those recited except for impurities ordinarily associated therewith. When the phrase "consists of" appears in a clause of the body of a claim, rather than immediately following the preamble, it limits only the element set forth in that clause; other elements are not excluded from the claim as a whole.

The transitional phrase "consisting essentially of" is used to define a composition, method that includes materials, steps, features, components, or elements, in addition to those literally disclosed provided that these additional included materials, steps, features, components, or elements do not materially affect the basic and novel characteristic(s) of the claimed invention, especially the mode of action to achieve the desired result of any of the processes of the present invention. The term "consisting essentially of" occupies a middle ground between "comprising" and "consisting of."

Where applicants have defined an invention or a portion thereof with an open-ended term such as "comprising," it should be readily understood that (unless otherwise stated) the description should be interpreted to also include such an invention using the terms "consisting essentially of" or "consisting of."

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

When an amount, concentration, or other value or parameter is given as either a range, preferred range or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

As used herein, the term "about" is meant to account for variations due to experimental error (e.g., plus or minus approximately 10% of the indicated value. ±1%, ± 2%, ± 3, ... ±10%). All measurements reported herein are understood to be modified by the term "about," whether or not the term is explicitly used, unless explicitly stated otherwise.

Compounds referred to in this disclosure may be referred to by code, based on fluorochemical naming convention, chemical structure and/or chemical name. For convenience and reference, selected compounds with codes, structures and chemical names are provided in Table 1.

**TABLE 1**

| **Code** | **Name-ASHRAE Designation** | **Formula** |
|---|---|---|
| R-1252zc | 1,1-difluoropropene (HFO-1252zc) | CH₃CH=CF₂ |
| R-250fb | 1,1,1,3-tetrachloropropane (HCC-250fb) | C₃H₄Cl₄ |
| R-251fb | 1,1,3-trichloro-1-fluoropropane (HCFC-251fb) | CCl₂FCH₂CH₂Cl |
| R-253fb | 3-chloro-1,1,1-trifluoropropane (HCFC-253fb) | CF₃CH₂CH₂Cl |
| R-252fc | 1,3-Dicloro-1,1-difluoropropane (HCFC-252fc) | C₃H₄Cl₂F₂ |
| R-262fc | 1,1-difluoropropane (HCFC-262fc) | CH₃CH₂CClF₂ |
| R-1240za | 1,1,3-trichloropropene (HCO-1240za) | CC₂=CHCH₂Cl |
| R-1241zf | 3,3-dichloro-3-fluoro-1-propene (HCFO-1241zf) | CCl₂FCH=CH₂ |
| R-1242zf | 3-chloro-3,3-difluoro-1-propene (HCFO-1242zf) | CClF₂CH=CH₂ |
| R-1243zf | 3,3,3-Trifluoropropene (HFO-1243zf) | CF₃CH=CH₂ |
| R-1252zf | 3,3-difluoropropene (HFO-1252zc) | CHF₂CH=CH₂ |
| R-262fa | 3-chloro-1,1-difluoropropane (HCFC-262fa) | CHF₂CH₂CH₂Cl |
| R-262db | 2-chloro-1,1-difluoropropane (HCFC-262db) | CH₃CHClCHF₂ |
| R-272fb | 1,1-difluoropropane (HFC-272fb) | CH₃CH₂CF₂H |

Some of the compounds present in the compositions of the present invention may exist as different configurational isomers or stereoisomers. The present invention is intended to include all single configurational isomers, single stereoisomers or any combination or mixture thereof. Single isomers or multiple isomers of the same compound may be used in any proportion.

Embodiments of the invention disclosed herein relates to processes of converting HCC-250fb to HFO-1252zc through intermediates including, but not limited to, one of more of HCFC-252fc, HCFC-262fc, and HCFO-1242zf, as well as processes of preparing the intermediates HCFC-252fc, HCFC-262fc, and HCFO-1242zf.

Reactors suitable for either liquid phase reactions or for vapor phase reactions can be used. In the vapor phase a heated reactor is used and the reactor is provided with suitable heat control. A number of reactor configurations are possible including packed bed tube or column reactors, operated in batch, semi-batch or continuous modes. Liquid phase reactor can be provided with suitable agitation equipment to increase contact between fluids and can be similarly operated in batch, semi-batch and continuous modes. In addition to the reactors disclosed herein, preheaters and vaporizers, heat exchangers, feed and effluent lines, units associated with mass transfer, contacting vessels (pre-mixers), distillation columns, and valving associated with reactors, heat exchangers, vessels, columns, and units that are used in the processes of various embodiments disclosed herein should be constructed of materials resistant to corrosion.

### Fluorination of HCC-250fb

In some embodiments, HCC-250fb may be used to make HCFC-252fc or HCFO-1242zf by hydrofluorination. The hydrofluorination reaction may be carried out in the liquid phase or vapor phase. In one embodiment, the reaction of HCC-250fb with HF may be conducted in the liquid phase to form HCFC-252fc. In another embodiment, the reaction of HCC-250fb with HF may be conducted in the vapor phase to form HCFO-1242zf. In another embodiment, the reaction of HCC-250fb with HF may be conducted in the vapor phase to form HFO-1243zf. In another embodiment, the reaction of HCC-250fb with HF may be conducted in the vapor phase to co-produce HCFO-1242zf and HFO-1243zf.

For liquid phase embodiments of the invention, the reaction of HCC-250fb with HF may be conducted in a liquid-phase reactor operating in batch, semi-batch, semi-continuous, or continuous modes. In the batch mode, HCC-250fb and HF are combined in an autoclave or other suitable reaction vessel and heated to the desired temperature.

In one embodiment, this reaction is carried out in semi-batch mode by feeding HCC-250fb to a liquid-phase reactor containing HF, or by feeding HF to a liquid-phase reactor containing HCC-250fb, or by feeding HCC-250fb to a liquid-phase reactor containing a mixture of HF and reaction products formed by initially heating HCC-250fb and HF, or by feeding HF to a liquid-phase reactor containing a mixture of HCC-250fb and reaction products formed by reacting HF and HCC-250fb. In another embodiment of the liquid-phase process, HF and HCC-250fb may be premixed and fed to the reactor or fed concurrently in the desired stoichiometric ratio to the reactor and mixed upstream of the catalyst bed. In one embodiment, the reactor contains a mixture of HF, HCC-250fb and/or reaction products formed by reacting HF and HCC-250fb.

Suitable temperatures for the reaction of HF with HCC-250fb in the liquid-phase reactor are, in one embodiment, from about 40°C to about 250°C, and in another embodiment, from about 50°C to about 100°C. Higher temperatures typically result in greater conversion of the HCC-250fb.

In one embodiment, a pre-heater may be utilized for pre-heating of the HCC-250fb before being introduced into the liquid phase reactor.

A suitable molar ratio of HF to total amount of HCC-250fb fed to the liquid-phase reactor is, in one embodiment, at least stoichiometric and in another embodiment, is from about 1:1 to about 100:1, or from about 1:1 to about 30:1, or from about 2:1 to about 30:1.

The reactor pressure in the liquid-phase process is not critical and in batch reactions is usually the autogenous pressure of the system at the reaction temperature. The pressure of the system increases as hydrogen chloride is formed by replacement of chlorine in HCC-250fb by fluorine from the HF. In a continuous process, it is possible to set the pressure of the reactor in such a way that the lower boiling products of the reaction are vented from the reactor, optionally through a packed column or condenser. In this manner, higher boiling intermediates remain in the reactor and the volatile products are removed. Typical reactor pressures are from about 20 psig (239 kPa) to about 1,000 psig (6,994 kPa).

Suitable contact times range from 2 minutes to 12 hours, and in some embodiments, 10 minutes to 6 hours.

In some embodiments, the reaction of HCC-250fb with HF to form HCFC-252fc is carried out in the absence of a catalyst.

In some embodiments, the reaction of HCC-250fb with HF to form HCFC-252fc is carried out in the presence of a catalyst. In some embodiments, in which the reaction is conducted using a liquid-phase process, catalysts which may be used include but are not limited to Lewis acid catalysts, transition metal halides, transition metal oxides, Group IVb metal halides, a Group Vb metal halides, or combinations thereof. Non-exclusive examples of liquid phase fluorination catalysts are an antimony halide, a tin halide, a tantalum halide, a titanium halide, a niobium halide, a zirconium halide, a thallium halide, a sodium halide, a molybdenum halide, an iron halide, a fluorinated chrome halide, a fluorinated chrome oxide or combinations thereof. Specific non-exclusive examples of liquid phase fluorination catalysts are include, but are not limited to, SbCl₅, SbCl₃, SbF₃, SbF₅, SbF₄, SnCl₄, TaCl₅, MoCl₆, TiCl₄, TiCl₅, FeCl₃, NaCl₅, NbF₅, ZrCl₄, a fluorinated species of SbCl₅, a fluorinated species of SbCl₃, a fluorinated species of SnCl₄, a fluorinated species of TaCl₅, a fluorinated species of TiCl₄, a fluorinated species of NbCl₅, a fluorinated species of MoCl₆, a fluorinated species of FeCl₃, and the like, as well as combinations of two or more of these. These catalysts can be readily regenerated by any means known in the art if they become deactivated.

Under these conditions, for example, a mixture of HF and HCC-250fb is converted by the catalytic liquid-phase fluorination process to a reaction mixture comprising HCl and a composition including HCFC-252fc. In one embodiment, the composition comprises HCFC-252fc and one or more additional compounds selected from HCFC-253fb, HCFC-251fb and HCC-250fb.

In another embodiment, the reaction of HF with HCC-250fb is carried out in the vapor phase to form HCFO-1242zf. In one embodiment, this reaction of HF with HCC-250fb is carried out in the vapor phase to co-produce HCFO-1242zf and HFO-1243zf. Typically, a heated reactor is used. A number of reactor configurations are possible including horizontal or vertical orientation of the reactor as well as the sequence of reaction of the HCC-250fb with HF. In one embodiment of the invention, the HCC-250fb may be initially vaporized and fed to the reactor as a gas.

In another embodiment of the invention, HCC-250fb may be contacted with HF in a pre-reactor prior to reaction in the vapor-phase reactor. In one embodiment, the pre-reactor may be empty. In another embodiment, the pre-reactor is filled with a suitable packing such as nickel-based alloys such as Hastelloy^{®}, nickel-chromium alloys commercially available from Special Metals Corp. under the trademark Inconel^{®} (hereinafter Inconel^{®}) or nickel-copper alloys commercially available from Special Metals Corp. (New Hartford, N.Y.) under the trademark Monel^{®} or other nickel alloy turnings or wool, or other material inert to HCl and HF which allows efficient mixing of HCC-250fb and HF vapor.

In some embodiments, an inert diluent gas is used as a carrier gas for HCC-250fb. In one embodiment, the carrier gas is selected is nitrogen, argon, helium or carbon dioxide. In some embodiments, the carrier gas is mixed and vaporized with the HCC-250fb and HF in the pre-reactor.

Suitable temperatures for the pre-reactor in one embodiment are from about 20°C to about 375°C, in another embodiment, from about 50°C to about 310°C.

The molar ratio of HF to the total amount of HCC-250fb in the pre-reactor is in one embodiment, from about the stoichiometric ratio of HF to the total amount of HCC-250fb to about 50:1. In another embodiment, the molar ratio of HF to the total amount of HCC-250fb in the pre-reactor is from about 1:1 to about 100:1, or from about 1:1 to about 30:1, or from about 2:1 to about 30:1, preferably about 3:1.

In one embodiment, the HCC-250fb is vaporized, optionally in the presence of HF, and fed to a pre-reactor or to a vapor-phase reactor along with HF.

In some embodiments, the molar ratio of HF to HCC-250fb for the vapor-phase reaction is from about 1:1 to about 100:1, or from about 1:1 to about 30:1, or from about 2:1 to about 30:1, preferably about 3:1.

Suitable temperatures for the vapor-phase reaction are from about 20°C to about 375°C, in another embodiment, from about 50°C to about 310°C.

Suitable reactor pressures for the vapor-phase reactor may be from about 1 to about 30 atmospheres. A pressure of about 15 to about 25 atmospheres may be advantageously employed to facilitate separation of HCl from other reaction products, and the suitable reaction time may vary from about 1 to about 120 seconds, preferably from about 5 to about 60 seconds.

In one embodiment, the vapor-phase fluorination of HCC-250fb to form HFCO-1242zf is carried out in the absence of a catalyst. For example, the hydrofluorination of HCC-250fb may be pyrolized in the absence of a catalyst in a reactor.

In another embodiment, a catalyst is used in the reaction zone for the vapor-phase reaction of HF with HCC-250fb to form HCFO-1242zf. Fluorination catalysts which may be used in the vapor phase reaction include but are not limited to Lewis acid catalysts, transition metal halides, transition metal oxides (preferably partially fluorinated transition metal oxides), Group IVb metal halides, Group Vb metal halides, and metal alloy packing having catalytic activity such as aluminum oxide, nickel-containing alloys such as Hastelloy^{®}, nickel-chromium containing alloys commercially available from Special Metals Corp. under the trademark Inconel^{®}, nickel-copper containing alloys commercially available from Special Metals Corp. (New Hartford, N.Y.) under the trademark Monel^{®}, other nickel alloy turnings, or zinc containing alloys, or combinations thereof.

In one embodiment, a vapor phase reaction of HCC-250fb to HCFO-1242zf may be carried out in the presence of a chromium-based catalyst, a cobalt-based catalyst, a nickel-based catalyst, an aluminum-based catalyst, an iron-based catalyst, or combinations thereof. In one embodiment, the chromium-based catalyst is a chromium oxide (e.g., Cr₂O₃). In one embodiment, the iron-based catalyst may be FeCl₃ on carbon. In one embodiment, the aluminum-based catalyst may be Al₂O₃.

Optionally, the catalysts described above can be pretreated with HF. This pretreatment can be accomplished, for example, by placing the catalyst in a suitable container and thereafter, passing HF over the catalyst. In one embodiment, such container can be the reactor used to perform the hydrofluorination reaction. In one embodiment, the pretreatment time is from about 15 to about 300 minutes, and the pretreatment temperature is from about 200°C to about 450°C.

In some embodiments, under these conditions, for example, a mixture of HF and HCC-250fb is converted by the vapor-phase fluorination process to a reaction mixture comprising HCl and a composition comprising HCFO-1242zf and one or more additional compounds selected from HCFC-253fb, HCFC-252fc, HCFC-251fb, HCC-250fb, HFO-1243zf, HCFO-1241zf and HCO-1240za. In some embodiments, the HCFO-1242zf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween**.**

In some embodiments, under these conditions, for example, a mixture of HF and HCC-250fb is converted by the catalytic vapor-phase fluorination process to a reaction mixture comprising HCl and a composition comprising HCFO-1242zf and HFO-1243zf, and optionally one or more additional compounds selected from HCFC-253fb, HCFC-252fc, HCFC-251fb, HCC-250fb, HCFO-1241zf and HCO-1240za.

In another embodiment, the reaction of HF with HCC-250fb is carried out in the vapor phase to form HFO-1243zf. Typically, a heated reactor is used. A number of reactor configurations are possible including horizontal or vertical orientation of the reactor as well as the sequence of reaction of the HCC-250fb with HF. In one embodiment of the invention, the HCC-250fb may be initially vaporized and fed to the reactor as a gas.

In another embodiment of the invention, HCC-250fb may be contacted with HF in a pre-reactor prior to reaction in the vapor-phase reactor. In one embodiment, the pre-reactor may be empty. In another embodiment, the pre-reactor is filled with a suitable packing such as nickel-based alloys such as Hastelloy^{®}, nickel-chromium alloys commercially available from Special Metals Corp. under the trademark Inconel^{®} (hereinafter Inconel^{®}) or nickel-copper alloys commercially available from Special Metals Corp. (New Hartford, N.Y.) under the trademark Monel^{®} or other nickel alloy turnings or wool, or other material inert to HCl and HF which allows efficient mixing of HCC-250fb and HF vapor.

In some embodiments, an inert diluent gas is used as a carrier gas for HCC-250fb. In one embodiment, the carrier gas is selected is nitrogen, argon, helium or carbon dioxide. In some embodiments, the carrier gas is mixed with the HCC-250fb and HF in the pre-reactor.

Suitable temperatures for the pre-reactor in one embodiment are from about 20°C to about 375°C, in another embodiment, from about 50°C to about 310°C.

The molar ratio of HF to the total amount of HCC-250fb in the pre-reactor is in one embodiment, from about the stoichiometric ratio of HF to the total amount of HCC-250fb to about 50:1. In another embodiment, the molar ratio of HF to the total amount of HCC-250fb in the pre-reactor is from about 1:1 to about 100:1, or from about 1:1 to about 30:1, or from about 2:1 to about 30:1, preferably about 10.2:1 to 26.4:1.

In one embodiment, the HCC-250fb is vaporized, optionally in the presence of HF, and fed to a pre-reactor or to a vapor-phase reactor along with HF.

The molar ratio of HF to the total amount of HCC-250fb for the vapor-phase reaction is, in one embodiment, from about the stoichiometric ratio of HF to the total amount of HCC-250fb to about 50:1 and, in another embodiment, from about 1:1 to about 100:1, or from about 1:1 to about 30:1, or from about 2:1 to about 30:1, preferably about 10.2:1 to 26.4:1.

Suitable temperatures for the vapor-phase reaction are from about 20°C to about 375°C, in another embodiment, from about 50°C to about 310°C.

Suitable reactor pressures for the vapor-phase reactor may be from about 1 to about 30 atmospheres. A pressure of about 15 to about 25 atmospheres may be advantageously employed to facilitate separation of HCl from other reaction products, and the suitable reaction time may vary from about 1 to about 120 seconds, preferably from about 5 to about 60 seconds.

In one embodiment, the vapor-phase fluorination of HCC-250fb to form HFO-1243zf is carried out in the absence of a catalyst. For example, the hydrofluorination of HCC-250fb may be pyrolized in the absence of a catalyst in a reactor.

In another embodiment, a catalyst is used in the reaction zone for the vapor-phase reaction of HF with HCC-250fb to form HFO-1243zf. Fluorination catalysts which may be used in the vapor phase reaction include but are not limited to Lewis acid catalysts, transition metal halides, transition metal oxides (preferably partially fluorinated transition metal oxides), Group IVb metal halides, Group Vb metal halides, and metal alloy packing, such as aluminum oxide, nickel-containing alloys such as Hastelloy^{®}, nickel-chromium containing alloys commercially available from Special Metals Corp. under the trademark Inconel^{®}, nickel-copper containing alloys commercially available from Special Metals Corp. (New Hartford, N.Y.) under the trademark Monel^{®}, other nickel alloy turnings, or zinc containing alloys, or combinations thereof.

In one embodiment, a vapor phase reaction of HCC-250fb to HFO-1243zf may be carried out in the presence of a chromium-based catalyst, a cobalt-based catalyst, a nickel-based catalyst, an aluminum-based catalyst, an iron-based catalyst, or combinations thereof. In one embodiment, the chromium-based catalyst is a chromium oxide (e.g., Cr₂O₃). In one embodiment, the iron-based catalyst may be FeCl₃ on carbon. In one embodiment, the aluminum-based catalyst may be Al₂O₃.

Optionally, the catalysts described above can be pretreated with HF or activated by acid treatment. This pretreatment can be accomplished, for example, by placing the catalyst in a suitable container and thereafter, passing HF or acid over the catalyst. In one embodiment, such container can be the reactor used to perform the hydrofluorination reaction. Where the metal alloy packing of the reactor has catalytic activity, HF or acid may be passed over the metal alloy packing surface for activation thereof. In one embodiment, the pretreatment time is from about 15 to about 300 minutes, and the pretreatment temperature is from about 200°C to about 450°C.

In some embodiments, under these conditions, for example, a mixture of HF and HCC-250fb is converted by the vapor-phase fluorination process to a reaction mixture comprising HCl and a composition comprising HFO-1243zf and one or more additional compounds selected from HCFC-253fb, HCFC-252fc, HCFC-251fb, HCC-250fb, HCFO-1242zf, HCFO-1241zf and HCO-1240za. In some embodiments, the HFO-1243zf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween**.**

In some embodiments, under these conditions, for example, a mixture of HF and HCC-250fb is converted by the vapor-phase fluorination process to a reaction mixture comprising HCl and a composition comprising HFO-1243zf and one or more additional compounds selected from HCFC-253fb, HCFC-252fc, HCFC-251fb, and HCC-250fb.

### Hydrogenation of HCFC-252fc

In some embodiments, HCFC-252fc may be used to make HCFC-262fc by hydrogenation. The hydrogenation reaction of HCFC-252fc may be carried out in the liquid phase or vapor phase. In one embodiment, the HCFC-252fc is produced by the above-described liquid-phase hydrofluorination of HCC-250fb.

For liquid phase embodiments of the invention, the reaction of HCFC-252fc with H₂ may be conducted in a liquid-phase reactor operating in batch, semi-batch, semi-continuous, or continuous modes. In the batch mode, HCFC-252fc and H₂ are combined in an autoclave or other suitable reaction vessel and heated to the desired temperature.

In one embodiment, this reaction is carried out in semi-batch mode by feeding HCFC-252fc to a liquid-phase reactor containing H₂, or by feeding H₂ to a liquid-phase reactor containing HCFC-252fc, or by feeding HCFC-252fc to a liquid-phase reactor containing a mixture of H₂ and reaction products formed by initially heating HCFC-252fc and H₂, or by feeding H₂ to a liquid-phase reactor containing a mixture of HCFC-252fc and reaction products formed by reacting H₂ and HCFC-252fc. In another embodiment of the liquid-phase process, H₂ and HCFC-252fc may be premixed and fed to the reactor or fed concurrently in the desired stoichiometric ratio to the reactor and mixed upstream of the catalyst bed. In one embodiment, the reactor contains a mixture of H₂, HCFC-252fc and/or reaction products formed by reacting H₂ and HCFC-252fc.

A suitable molar ratio of H₂ to total amount of HCFC-252fc fed to the liquid-phase reactor is, in one embodiment, at least stoichiometric and in another embodiment, is from about 0.5:1 to about 100:1, or from about 5:1 to about 100:1, or from about 8:1 to about 50:1, or from about 0.5 to about 30:1, or from about 1:1 to about 30:1, or from about 2:1 to about 30:1.

The reactor pressure in the liquid-phase process is not critical and in batch reactions is usually the autogenous pressure of the system at the reaction temperature. In a continuous process, it is possible to set the pressure of the reactor in such a way that the lower boiling products of the reaction are vented from the reactor, optionally through a packed column or condenser. In this manner, higher boiling intermediates remain in the reactor and the volatile products are removed. Typical reactor pressures are from about 20 psig (239 kPa) to about 1,000 psig (6,994 kPa).

In one embodiment, a catalyst is used in the reaction zone for the liquid phase reaction of H₂ with HCFC-252dc. Hydrogenation catalysts which may be used comprise a group VIII metal or ruthenium. In one embodiment, the metal is carried on a support, for example, Pd is carried on aluminum oxide, aluminum fluoride or carbon). In another embodiment, the metal is carried (for example, Raney nickel). In certain embodiments, the metal catalyst is supported on carbon and the carbon support/carrier is comprises carbon, acid-washed carbon, activated carbon, three-dimensional matrix carbonaceous materials. In one embodiment, the catalyst/catalyzer is Pd/carbon.

Suitable temperatures for the catalytic hydrogenation of HCFC-252fc in the liquid-phase reactor are, in one embodiment, from about 20°C to about 150°C, or about 30°C to about 100°C. Higher temperatures typically result in greater conversion of the HCFC-252fc.

In another embodiment, the hydrogenation of HCFC-252fc to form HCFC-262fc is non-catalytic and suitable temperatures are in the range of about 100°C to about 400°C, or in some embodiments about 200°C or more.

Under these conditions, for example, a mixture of H₂ and HCFC-252fc is converted by the liquid-phase hydrogenation process to a composition comprising HCFC-262fc. In some embodiments, the composition comprises HCFC-262fc and one or more additional compounds selected from HCC-250fb, HCFC-252fc, HFC-263fb, HCFC-262fa and HCFC-272fb.

In another embodiment, the reaction of H₂ with HCFC-252fc is carried out in the vapor phase. Typically, a heated reactor is used. A number of reactor configurations are possible including horizontal or vertical orientation of the reactor as well as the sequence of reaction of the HCFC-252fc with H₂. In one embodiment of the invention, the HCFC-252fc may be initially vaporized and fed to the reactor as a gas.

In another embodiment of the invention, HCFC-252fc may be contacted with H₂ in a pre-reactor prior to reaction in the vapor-phase reactor. In one embodiment, the pre-reactor may be empty. In another embodiment, the reactor is filled with a suitable packing such as nickel-copper alloys commercially available from Special Metals Corp. (New Hartford, New York) under the trademark Monel^{®}, (hereinafter "Monel^{®}") nickel-based alloys commercially available from Haynes International (Kokomo, Indiana) under the trademark Hastelloy^{®}, (hereinafter "Hastelloy^{®}") or other nickel alloy turnings or wool, or other inert material which allows efficient mixing of HCFC-252fc and hydrogen gas.

Suitable temperatures for the pre-reactor in one embodiment are from about 20°C to about 120°C, preferably about 30°C to about 80°C.

In one embodiment, the HCFC-252fc is vaporized, optionally in the presence of hydrogen, and fed to a pre-reactor or to a vapor-phase reactor along with hydrogen.

Suitable temperatures for the vapor-phase reaction are from about 20°C to about 120°C, preferably about 30°C to about 80°C.

Suitable reactor pressures for the vapor-phase reactor may be from about 1 to about 30 atmospheres, and the suitable reaction time may vary from about 1 to about 120 seconds, preferably from about 5 to about 60 seconds.

The molar ratio of H₂ to the total amount of HCFC-252fc for the vapor-phase reaction is, in one embodiment, from about 5:1 to about 100:1, or from about 8:1 to about 50:1, or from about 1:1 to about 30:1, or from about 2:1 to about 30:1.

In one embodiment, a catalyst is used in the reaction zone for the vapor-phase reaction of H₂ with HCFC-252dc. Hydrogenation catalysts which may be used comprise a group VIII metal or ruthenium. In one embodiment, the metal is carried on a support, for example, Pd is carried on aluminum oxide, aluminum fluoride or carbon). In another embodiment, the metal is carried (for example, Raney nickel). In certain embodiments, the metal catalyst is supported on carbon and the carbon support/carrier is comprises carbon, acid-washed carbon, activated carbon, three-dimensional matrix carbonaceous materials. In one embodiment, the catalyst/catalyzer is Pd/carbon.

Under these conditions, for example, a mixture of H₂ and HCFC-252fc is converted by the vapor-phase hydrogenation process to a composition comprising HCFC-262fc and one or more additional compounds selected from HCC-250fb, HFC-252fc, HFC-263fb, HCFC-262fa and HCFC-272fb. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

### Conversion of HCFC-252fc to HCFO-1242zf using a caustic agent

In some embodiments, HCFC-252fc may be used to make HCFO-1242zf by contacting the HCFC-252fc with a caustic agent in an aqueous solvent at a temperature in the range of about 20°C to about 150°C, preferably about 30°C to about 100°C. In one embodiment, the HCFC-252fc is produced by the above-described liquid-phase hydrofluorination of HCC-250fb.

In some embodiments, the caustic agent comprises a base that would dissociate when placed in water or react with water. Examples include an alkali metal oxides, hydroxide, or amide, such as sodium or potassium oxide or sodium or potassium hydroxide or sodium or potassium amide; or alkaline earth metal hydroxide, alkaline earth metal oxide or amide, alkali metal carbonate or alkali metal phosphate or alkali metal carboxylate. Caustic agents include, but are not limited to, NaOH, KOH, LiOH, CsOH, Ca(OH)₂, Zn(OH)₂, Na₂CO₃, K₂CO₃, K₃PO₄, Na₃PO₄, KF, or CsF and the like. In some embodiments, the caustic agent is dissolved in an aqueous solution or present in an aqueous suspension. The caustic agent in the aqueous phase is present in effective amounts for dehydrohalogenation.

The reaction produces an alkali metal halide salt, such as lithium chloride, lithium bromide, lithium iodide, sodium chloride, sodium bromide, sodium iodide, potassium chloride, potassium bromide, potassium iodide, and mixtures thereof. In some embodiments, the alkali metal halide is sodium chloride.

Under these conditions, for example, a mixture of HCFC-252fc is converted by the liquid-phase reaction in the presence of a caustic agent to a product mixture comprising water, a metal salt and a composition comprising HCFO-1242zf and one or more additional compounds selected from HCFC-253fb, HCFC-251fb, HCFC-252fc and HCC-250fb. In some embodiments, the HCFO-1242zf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween**.**

### Hydrogenation of HCFO-1242zf

In some embodiments, HCFO-1242zf may be used to make HCFC-262fc by hydrogenation. The hydrogenation reaction of HCFC-252fc may be carried out in the liquid phase or vapor phase. In one embodiment, the HCFO-1242zf is produced by the above-described vapor-phase hydrofluorination of HCC-250fb.

For liquid phase embodiments of the invention, the reaction of HCFO-1242zf with H₂ may be conducted in a liquid-phase reactor operating in batch, semi-batch, semi-continuous, or continuous modes. In the batch mode, HCFO-1242zf and H₂ are combined in an autoclave or other suitable reaction vessel and heated to the desired temperature.

In one embodiment, this reaction is carried out in semi-batch mode by feeding HCFO-1242zf to a liquid-phase reactor containing H₂, or by feeding H₂ to a liquid-phase reactor containing HCFO-1242zf, or by feeding HCFO-1242zf to a liquid-phase reactor containing a mixture of H₂ and reaction products formed by initially heating HCFO-1242zf and H₂, or by feeding H₂ to a liquid-phase reactor containing a mixture of HCFO-1242zf and reaction products formed by reacting H₂ and HCFO-1242zf. In another embodiment of the liquid-phase process, H₂ and HCFO-1242zf may be premixed and fed to the reactor or fed concurrently in the desired stoichiometric ratio to the reactor and mixed upstream of the catalyst bed. In one embodiment, the reactor contains a mixture of H₂, HCFO-1242zf and/or reaction products formed by reacting H₂ and HCFO-1242zf.

Suitable temperatures for the reaction of H₂ with HCFO-1242zf in the liquid-phase reactor are, in one embodiment, from about 280°C to about 150°C, and in another embodiment, from about 30°C to about 100°C. Higher temperatures typically result in greater conversion of the HCFO-1242zf.

A suitable molar ratio of H₂ to total amount of HCFO-1242zf fed to the liquid-phase reactor is, in one embodiment, at least stoichiometric and in another embodiment, is from about 1:1 to about 5:1, or about 1.2:1 to about 3:1.

The reactor pressure in the liquid-phase process is not critical and in batch reactions is usually the autogenous pressure of the system at the reaction temperature. In a continuous process, it is possible to set the pressure of the reactor in such a way that the lower boiling products of the reaction are vented from the reactor, optionally through a packed column or condenser. In this manner, higher boiling intermediates remain in the reactor and the volatile products are removed. Typical reactor pressures are from about 20 psig (239 kPa) to about 1,000 psig (6,994 kPa).

In some embodiments, in which the reaction is conducted using a liquid-phase process, hydrogenation catalysts which may be used in the liquid-phase comprise a group VIII metal or ruthenium. In one embodiment, the metal is carried on a support, for example, Pd is carried on aluminum oxide, aluminum fluoride or carbon). In another embodiment, the metal is carried (for example, Raney nickel). In certain embodiments, the metal catalyst is supported on carbon and the carbon support/carrier is comprises carbon, acid-washed carbon, activated carbon, three-dimensional matrix carbonaceous materials. In one embodiment, the catalyst/catalyzer is Pd/carbon.

Under these conditions, for example, a mixture of H₂ and HCFO-1242zf is converted by the liquid-phase hydrogenation process to a composition comprising HCFC-262fc and one or more additional compounds selected from HCC-250fb, HFC-263fb, HFC-272fb, HFO-1252zc and HCFO-1242zf, where the HCFC-262fc constitutes about 90 wt.% to 99.9 wt.% based on the total weight of the composition.

In one embodiment, under these conditions, for example, a mixture of H₂ and HCFO-1242zf is converted by the liquid-phase hydrogenation process to a composition comprising HCFC-262fc and one or more additional compounds selected from ethane, HFC-152a, HFC-253db, HCFO-1232 isomer(s), HCFO-1242 isomer(s), HCFO-1242zf, HFO-1243zf and HFO-1252zc. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In some embodiments, the vapor portion of the composition produced by the liquid-phase hydrogenation of HCFO-1242zf comprises HCFC-262fc and one or more additional compounds selected from ethane, HFC-152a, HFC-253db, HCFO-1232 isomer(s), HCFO-1242 isomer(s), HCFO-1242zf, HFO-1243zf and HFO-1252zc. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the vapor portion of the composition, inclusive of all integers and ranges therebetween.

In some embodiments, the liquid portion of the composition produced by the liquid-phase hydrogenation of HCFO-1242zf comprises HCFC-262fc and one or more additional compounds selected from HFC-253db, HCFO-1232 isomer(s), HCFO-1242 isomer(s), HCFO-1242zf and HFO-1252zc. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the liquid portion of the composition, inclusive of all integers and ranges therebetween.

In one embodiment, under these conditions, for example, a mixture of H₂ and HCFO-1242zf is converted by the liquid-phase hydrogenation process to a composition comprising HCFC-262fc and one or more additional compounds selected from HFC-253db, HCFO-1232 isomer(s), HCFO-1242zf and HFO-1252zc. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In another embodiment, the reaction of H₂ with HCFO-1242zf is carried out in the vapor phase. Typically, a heated reactor is used. A number of reactor configurations are possible including horizontal or vertical orientation of the reactor as well as the sequence of reaction of the HCFO-1242zf with H₂. In one embodiment of the invention, the HCFO-1242zf may be initially vaporized and fed to the reactor as a gas.

In another embodiment of the invention, HCFO-1242zf may be contacted with H₂ in a pre-reactor prior to reaction in the vapor-phase reactor. In one embodiment, the pre-reactor may be empty. In another embodiment, the reactor is filled with a suitable packing such as nickel-copper alloys commercially available from Special Metals Corp. (New Hartford, New York) under the trademark Monel^{®}, (hereinafter "Monel^{®}") nickel-based alloys commercially available from Haynes International (Kokomo, Indiana) under the trademark Hastelloy^{®}, (hereinafter "Hastelloy^{®}") or other nickel alloy turnings or wool, or other inert material which allows efficient mixing of HCFO-1242zf and hydrogen gas.

Suitable temperatures for the pre-reactor in one embodiment are from about 20°C to about 120°C, in another embodiment, from about 30°C to about 80°C.

The molar ratio of H₂ to the total amount of HCFO-1242zf in the pre-reactor is in one embodiment, from about the stoichiometric ratio of H₂ to the total amount of HCFO-1242zf to about 10:1. In another embodiment, the molar ratio of H₂ to the total amount of HCFO-1242zf in the pre-reactor is from about 1:1 to about 5:1, or about 1.2:1 to about 3:1.

In one embodiment, the HCFO-1242zf is vaporized, optionally in the presence of hydrogen, and fed to a pre-reactor or to a vapor-phase reactor along with hydrogen.

Suitable temperatures for the vapor-phase reaction are from about 20°C to about 120°C, in another embodiment, from about 30°C to about 80°C.

Suitable reactor pressures for the vapor-phase reactor may be from about 1 to about 30 atmospheres, and the suitable reaction time may vary from about 1 to about 120 seconds, preferably from about 5 to about 60 seconds.

The molar ratio of H₂ to the total amount of HCFO-1242zf for the vapor-phase reaction is, in one embodiment, from about the stoichiometric ratio of H₂ to the total amount of HCFO-1242zf to about 10:1 and, in another embodiment, from about 1:1 to about 5:1, or about 1.2:1 to about 4:1.

In one embodiment, a catalyst is used in the reaction zone for the vapor-phase reaction of H₂ with HCFO-1242zf. Hydrogenation catalysts which may be used in the vapor phase reaction comprises a group VIII metal or ruthenium. In one embodiment, the metal is carried on a support, for example, Pd is carried on aluminum oxide, aluminum fluoride or carbon). In another embodiment, the metal is carried (for example, Raney nickel). In certain embodiments, the metal catalyst is supported on carbon and the carbon support/carrier is comprises carbon, acid-washed carbon, activated carbon, three-dimensional matrix carbonaceous materials. In one embodiment, the catalyst/catalyzer is Pd/carbon.

In one embodiment, under these conditions, for example, a mixture of H₂ and HCFO-1242zf is converted by the vapor-phase hydrogenation process to a composition comprising HCFC-262fc and one or more additional compounds selected from propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomers and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc and one or more compounds selected from HFO-1252zc, HCFC-262db, HFC-272fb, HFO-1243zf and HCFO-1242zf. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc, HCFO-1242zf and one or more additional compounds selected from propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242 isomer(s), HCFO-1232 isomer(s) and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc, HFC-272fb and one or more additional compounds selected from propane, propylene, E-HFO-1261ze, Z-HFO-1261ze, HFO-1252zc, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc and one or more additional compounds selected from HCC-250fb, HFC-263fb, HFC-272fb, HFO-1252zc and HCFO-1242zf. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc and one or more additional compounds selected from propane, propylene, HCFC-252dc, HCFC-262db, HFC-272fb, HCFO-1232xf, HCFO-1242zf, HFO-1243zf, E-HCFO-1251zd, Z-HCFO-1251zd, HFO-1252zf, E-HFO-1261ze, Z-HFO-1261ze, HCFO-1242 isomer(s) and HCO-1260. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc and one or more additional compounds selected from propane, propylene, HCFC-252dc, HCFC-262db, HFC-272fb, HCFO-1232xf, HCFO-1242zf, HFO-1243zf, E-HCFO-1251zd, Z-HCFO-1251zd, HFO-1252zf, E-HFO-1261ze, Z-HFO-1261ze, and HCO-1260. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc and one or more additional compounds selected from propane, propylene, HCFC-252dc, HCFC-262db, HCFO-1232xf, HCFO-1242zf, HFO-1243zf, E-HCFO-1251zd, Z-HCFO-1251zd, HFO-1252zf, E-HFO-1261ze, Z-HFO-1261ze and HCFO-1242 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In one embodiment, under these conditions, for example, a mixture of H₂ and HCFO-1242zf is converted by the vapor-phase hydrogenation process to a composition comprising HCFC-262fc and one or more additional compounds selected from HCFC-262db, HCFO-1232xf, HCFO-1242zf, HFO-1243zf, E-HCFO-1251zd, Z-HCFO-1251zd, HFO-1252zf and E-HFO-1261ze. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

### Dehydrochlorination of HCFC-262fc

In some embodiments, HCFC-262fc may be used to make HFO-1252zc by dehydrohalogenation, and more particularly dehydrochlorination. In one embodiment, the HCFC-262fc is produced by the above-described liquid-phase or vapor-phase hydrogenation of HCFC-252fc or the above-described liquid-phase or vapor-phase hydrogenation of HCFO-1242zf.

In some embodiments, the dehydrohalogenation reaction of HCFC-262fc is preferably carried out in the vapor phase. In one embodiment, vapor phase dehydrochlorination of HCFC-262fc to produce HFO-1252zc is carried out in the presence of catalyst. In one embodiment, the dehydrohalogenation catalysts include, but are not limited to, carbon and/or metal-based catalysts. In one embodiment, the catalyst may be selected from an activated carbon, a nickel-based catalyst, a palladium-based catalyst, or any combination of these catalysts. In one embodiment, the catalyst may be selected from Ni-mesh, palladium on carbon, palladium on aluminum oxide, or combinations thereof. In one embodiment, the catalyst may be a metal alloy, such as nickel-based alloys such as Hastelloy^{®}, nickel-chromium alloys commercially available from Special Metals Corp. under the trademark Inconel^{®} (hereinafter Inconel^{®}) or nickel-copper alloys commercially available from Special Metals Corp. (New Hartford, N.Y.) under the trademark Monel^{®} or other nickel alloy turnings or activated carbon.

In some embodiments of the invention, catalytic dehydrohalogenation of HCFC-262fc to form HFO-1252zc is performed at a temperature in the range of from about 400°C to about 800°C, preferably about 400°C to about 700°C, or preferably greater than about 400°C, all values and ranges therebetween.

In one embodiment, HFO-1252zc is prepared by thermal dehydrochlorination (pyrolysis) of HCFC-262fc. In one embodiment, this reaction occurs in the absence of a catalyst. In one embodiment, HCFC-262fc is introduced into a reaction vessel which temperature is maintained at a temperature high enough to effect the thermal dehydrochlorination of HCFC-262fc. In one embodiment, the temperature is high enough to effect the thermal dehydrochlorination of HCFC-262fc to a percent conversion of at least 10% with a contact time of between about 10 seconds and about 30 minutes.

In some embodiments of the invention, non-catalytic dehydrohalogenation of HCFC-262fc to form HFO-1252zc is performed at a temperature in the range of from about 400°C to about 800°C, preferably about 450°C to about 700°C, or preferably about 450°C or higher, all values and ranges therebetween.

In one embodiment, the reactor is comprised of materials which are resistant to corrosion. In one embodiment, the reactor is filled with a suitable packing such as nickel-copper alloys commercially available from Special Metals Corp. (New Hartford, New York) under the trademark Monel^{®}, (hereinafter "Monel^{®}") nickel-based alloys commercially available from Haynes International (Kokomo, Indiana) under the trademark Hastelloy^{®}, (hereinafter "Hastelloy^{®}") or other nickel alloy turnings or wool, or other inert material.

Suitable reactor pressures for the vapor-phase reactor may be from about 10 to about 200 psig, and the suitable reaction time may vary from 10 seconds to about 30 minutes.

In one embodiment, the HCFC-262fc is preheated in a vaporizer to a temperature of about 200°C.

In some embodiments, an inert diluent gas is used as a carrier gas for HCFC-262fc. In one embodiment, the carrier gas is selected is nitrogen, argon, helium, or carbon dioxide.

In other embodiments, the dehydrohalogenation reaction of HCFC-262fc is preferably carried out in the liquid phase. More particularly, dehydrochlorination of HCFC-262fc is carried out using a strong base in a solvent, such as an aqueous solvent or an organic solvent, in the presence or absence of a catalyst.

Exemplary bases include, but are not limited to, lithium hydroxide, sodium hydroxide, potassium hydroxide, calcium hydroxide, magnesium oxide, calcium oxide, sodium carbonate, potassium carbonate, sodium phosphate, potassium phosphate, and mixtures thereof. Some example strong bases include, but are not limited to, hydroxides, alkoxides, metal amides, metal hydrides, metal dialkylamides and arylamines, wherein; alkoxides include lithium, sodium and potassium salts of methyl, ethyl and t-butyl oxides; metal amides include sodium amide, potassium amide and lithium amide; metal hydrides include sodium hydride, potassium hydride and lithium hydride; and metal dialkylamides include lithium, sodium, and potassium salts of methyl, ethyl, n-propyl, iso-propyl, n-butyl, tert-butyl, trimethylsilyl and cyclohexyl substituted amides.

In some embodiments, the base is selected from lithium hydroxide, sodium hydroxide, potassium hydroxide, calcium hydroxide, magnesium oxide, calcium oxide, sodium carbonate, potassium carbonate, sodium phosphate, potassium phosphate, potassium tert-butoxide, sodium and potassium salts of methyl, ethyl and t-butyl oxides, and mixtures thereof.

In some embodiments, the base is an aqueous basic solution. As used herein, the "basic aqueous solution" is a liquid (e.g., a solution, dispersion, emulsion, or suspension, and the like) that is primarily an aqueous liquid having a pH of over 7.

In some embodiments, the basic aqueous solution contains small amounts of organic liquids which may be miscible or immiscible with water. In some embodiments, the liquid medium in the basic aqueous solution is at least 90% water, for example, at least 95%, at least 97%, at least 98%, at least 99%, at least 99.5%, or at least 99.9%. In some embodiments, the water used in the aqueous basic solution is tap water. In some embodiments, the water is used in the aqueous basic solution deionized water or distilled water.

In some embodiments, examples of solvents include alkyl, dialkyl, and trialkyl linear or cylic amines, N-methylpyrrolidine, N-methylpiperidine, sulfoxides, ethers, pyridine or alkyl-substituted pyridines, pyrazine or pyrimidine, alkyl and aromatic nitriles, hexamethylphosphoramide, alcohols, esters, and mixtures thereof. In one embodiment, an alcohol solvent is methanol. In one embodiment, an ester solvent is methyl formate. In one embodiment, a sulfoxide solvent is dimethylsulfoxide. In one embodiment, an alkyl nitrile solvent is acetonitrile. In one embodiment, an aromatic nitrile solvent is benzonitrile. In another embodiment, the reaction solvent is selected from trialkylamines, N-methylpyrrolidine, N-methylpiperidine, pyridine, alkyl-substituted pyridines, dimethylformamide, pyrazine or pyrimidine, and mixtures thereof. In another embodiment, the reaction solvent is selected from dimethylformamide, tetrahydrofuran, pyridine, dimethylacetamide, 1,4-dioxane, N-methylpyrrolidone, diethyl ether, and mixtures thereof. In yet another embodiment, the reaction solvent is pyridine or alkyl-substituted pyridines, or mixtures thereof. In yet another embodiment, the reaction solvent is a mixture of pyridine or alkyl-substituted pyridines, and dimethylformamide.

Under these conditions, for example, HCFC-262fc is converted by the liquid-phase or vapor-phase dehydrohalogenation process to a composition comprising HFO-1252zc and one or more additional compounds selected from HCFC-262fc, HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf, HFO-1241zf, HFO-1240za and HCFC-252fc. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

### Conversion of HCFO-1242zf to HFO-1252zc by reaction with Zn

In some embodiments, the present invention provides a liquid phase method of dechlorinating the HCFO-1242zf formed by the above-described vapor phase hydrofluorination of HCC-250fb. The dechlorination method involves contacting the HCFO-1242zf with zinc.

The reaction of HCFO-1242zf with Zn may be conducted in a liquid-phase reactor operating in batch, semi-batch, semi-continuous, or continuous modes. In the batch mode, HCFO-1242zf and Zn are combined in an autoclave or other suitable reaction vessel and heated to the desired temperature.

In one embodiment, this reaction is carried out in semi-batch mode by feeding HCFO-1242zf to a liquid-phase reactor containing Zn, or by feeding Zn to a liquid-phase reactor containing HCFO-1242zf, or by feeding HCFO-1242zf to a liquid-phase reactor containing a mixture of Zn and reaction products formed by initially heating HCFO-1242zf and Zn, or by feeding Zn to a liquid-phase reactor containing a mixture of HCFO-1242zf and reaction products formed by reacting Zn and HCFO-1242zf. In another embodiment of the liquid-phase process, Zn and HCFO-1242zf may be premixed and fed to the reactor or fed concurrently in the desired stoichiometric ratio to the reactor and mixed upstream of the catalyst bed. In one embodiment, the reactor contains a mixture of Zn, HCFO-1242zf and/or reaction products formed by reacting Zn and HCFO-1242zf.

Suitable temperatures for the reaction of Zn with HCFO-1242zf in the liquid-phase reactor are, in one embodiment, from about 20°C to about 150°C, and in another embodiment, from about 30°C to about 100°C. Higher temperatures typically result in greater conversion of the HCFO-1242zf.

A suitable molar ratio of Zn to total amount of HCFO-1242zf fed to the liquid-phase reactor is, in one embodiment, at least stoichiometric and in another embodiment, is from about 1:1 to about 30:1, or from about 2:1 to about 30:1.

The reactor pressure in the liquid-phase process is not critical and in batch reactions is usually the autogenous pressure of the system at the reaction temperature. In a continuous process, it is possible to set the pressure of the reactor in such a way that the lower boiling products of the reaction are vented from the reactor, optionally through a packed column or condenser. In this manner, higher boiling intermediates remain in the reactor and the volatile products are removed. Typical reactor pressures are from about 20 psig (239 kPa) to about 1,000 psig (6,994 kPa).

In some embodiments, in which the reaction is conducted using a liquid-phase process, the reaction is carried out by reacting HCFO-1242zf with zinc in an organic solvent in the presence or absence of a catalyst.

Examples of the organic solvent include alcohols such as methanol, ethanol and glycol; organic acids, such as acetic acid, propionic acid, butyric acid, octanoic acid, phthalic acid, benzoic acid; esters of organic acids, such as methyl acetate, ethyl acetate, ethylene glycol diacetate, propylene glycol diacetate, dimethyl adipate, methyl benzoate, ethyl benzoate, dimethyl phthalate, diethyl phthalate, dioctyl phthalate, phenyl acetate, and tolyl acetate; hydrocarbons, such as dodecane, hexadecane, benzene, naphthalene, and biphenyl; esters of inorganic acids, such as triphenyl phosphate, tricresyl phosphate, dibutylphenyl phosphate, silicates such as tetramethyl ortho-silicate, and tetrabutyl silicate; ketones, such as aromatic ethers, such as diphenyl ethers; ketones, such as acetone, methyl ethyl ketone, dibutyl ketone, methyl isobutyl ketone, acetophenone, and benzophenone; polar aprotic solvents such as acetonitrile, propionitrile, N, N-dimethylformamide (DMF); and carboxylic acid anhydrides such as acetic anhydride and propionic anhydride; and mixtures thereof.

In some embodiments, examples of suitable organic solvents include alkyl, dialkyl, and trialkyl linear or cylic amines, N-methylpyrrolidine, N-methylpiperidine, sulfoxides, ethers, pyridine or alkyl-substituted pyridines, pyrazine or pyrimidine, alkyl and aromatic nitriles, hexamethylphosphoramide, alcohols, esters, and mixtures thereof. In one embodiment, an alcohol solvent is methanol. In one embodiment, an ester solvent is methyl formate. In one embodiment, a sulfoxide solvent is dimethylsulfoxide. In one embodiment, an alkyl nitrile solvent is acetonitrile. In one embodiment, an aromatic nitrile solvent is benzonitrile. In another embodiment, the reaction solvent is selected from trialkylamines, N-methylpyrrolidine, N-methylpiperidine, pyridine, alkyl-substituted pyridines, dimethylformamide, pyrazine or pyrimidine, and mixtures thereof. In another embodiment, the reaction solvent is selected from dimethylformamide, tetrahydrofuran, pyridine, dimethylacetamide, 1,4-dioxane, N-methylpyrrolidone, diethyl ether, and mixtures thereof. In yet another embodiment, the reaction solvent is pyridine or alkyl-substituted pyridines, or mixtures thereof. In yet another embodiment, the reaction solvent is a mixture of pyridine or alkyl-substituted pyridines, and dimethylformamide.

In some embodiments, the reaction is carried out by reacting HCFO-1242zf with zinc in an organic solvent in the absence of a catalyst.

In some embodiments, the reaction is carried out by reacting HCFO-1242zf with zinc in an organic solvent in the presence of a catalyst. In some embodiments, the catalyst is a metal salt and/or a phase transfer catalyst. Examples of a metal salt include, but are not limited to, a zinc salt. Suitable zinc salts include zinc acetate, zinc bromide, zinc chloride, zinc citrate, zinc sulfate and mixtures thereof. Suitable phase transfer catalysts include quaternary ammonium halides (e.g., tetrabutylammonium bromide, tetrabutylammonium hydrosulfate, triethylbenzylammonium chloride, dodecyltrimethylammonium chloride, and tricaprylylmethylammonium chloride), quaternary phosphonium halides (e.g., triphenylmethylphosphonium bromide and tetraphenylphosphonium chloride), or cyclic polyether compounds known in the art as crown ethers (e.g., 18-crown-6 and 15-crown-5). In some embodiments, the catalyst is activated by acid treatment. This activation can be accomplished, for example, by placing the catalyst in a suitable container and thereafter, passing HF or acid over the catalyst. In one embodiment, such container can be the reactor used to perform the hydrofluorination reaction.

Under these conditions, for example, a mixture of Zn and HCFO-1242zf is converted by the liquid-phase dechlorination process to a reaction mixture comprising zinc chloride (ZnCl₂) and a composition comprising HFO-1252zc and one or more additional compounds selected from HCC-250fb, HFO-1252zc, HFO-1243zf, HCC-262fc, HCC-263fb and HCFO-1242zf. In some embodiments, the HCFC-1252zc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

### Integrated Processes

In certain embodiments disclosed herein, HFO-1252zc is prepared according to an integrated process comprising Reaction Scheme A:
**Step 1A:**

   CCl₃CH₂CH₂Cl (HCC-250fb) + HF → CClF₂CH₂CH₂Cl (HCFC-252fc) + HCl
**Step 2A:**

   CClF₂CH₂CH₂Cl (HCFC-252fc) + H₂ → CClF₂CH₂CH₃ (HCFC-262fc) + HCl
**Steps 3A:**

   CClF₂CH₂CH₃ (HCFC-262fc) → CF₂=CHCH₃ (HFO-1252zc) + HCl

   OR
   Sol

   CClF₂CH₂CH₃ (HCFC-262fc) + Strong Base → CF₂=CHCH₃ (HFO-1252zc)
where Step 1A is the liquid phase hydrofluorination of HCC-250fb described herein, Step 2A is the liquid phase or vapor phase hydrogenation of HCFC-252fc described herein, and Step 3A is the vapor phase or liquid phase dehydrohalogenation of HCFC-262fc described herein.

In some embodiments, the integrated process further comprises separation and/or purification steps to recover the desired intermediate (e.g., HCFC-252fc and HCFC-262fc) and the desired reaction product HFO-1252zc.

In some embodiments, HCFC-262fc is prepared from HCFC-252fc according to Step 2A, wherein Step 2A is the liquid phase or vapor phase hydrogenation of HCFC-252fc described herein.

In some embodiments, HCFC-262fc is prepared from HCC-250fb according to integrated Steps 1A and 2A, wherein Step 1A is the liquid phase hydrofluorination of HCC-250fb described herein and wherein Step 2A is the liquid phase or vapor phase hydrogenation of HCFC-252fc described herein.

In some embodiments, HFO-1252zc is prepared from HCFC-252fc according to integrated Steps 2A and 3A, where Step 2A is the liquid phase or vapor phase hydrogenation of HCFC-252fc described herein, and Step 3A is the vapor phase dehydrohalogenation of HCFC-262fc described herein.

In some embodiments, HFO-1252zc is prepared from HCFC-262fc according to Step 3A, where Step 3A is the vapor phase dehydrohalogenation of HCFC-262fc described herein.

In certain embodiments disclosed herein, HFO-1252zc is prepared according to an integrated process comprising Reaction Scheme B:
**Step 1B:**

   CCl₃CH₂CH₂Cl (HCC-250fb) + HF → CClF₂CH=CH₂ (HCFO-1242zf) + HCl
**Step 2B:**

   CClF₂CH=CH₂ (HCFO-1242zf) + H₂ → CClF₂CH₂CH₃ (HCFC-262fc)
**Step 3B:**

   CClF₂CH₂CH₃ (HCFC-262fc) → CF₂=CHCH₃ (HFO-1252zc) + HCl
where Step 1B is the vapor phase hydrofluorination of HCC-250fb described herein, Step 2B is the liquid phase or vapor phase hydrogenation of HCFO-1242zf described herein, and Step 3B is the vapor phase dehydrohalogenation of HCFC-262fc described herein.

In some embodiments, the integrated process further comprises separation and/or purification steps to recover the desired intermediate (e.g., HCFO-1242zf and HCFC-262fc) and the desired reaction product HFO-1252zc.

In some embodiments, HCFC-262fc is prepared from HFO-1243zf according to Step 2B, wherein Step 2B is the liquid phase or vapor phase hydrogenation of HCFO-1242zf described herein.

In some embodiments, HFO-1252zc is prepared from HFO-1243zf according to Steps 2B and 3B, wherein Step 2B is the liquid phase or vapor phase hydrogenation of HCFO-1242zf described herein, and Step 3B is the vapor phase dehydrohalogenation of HCFC-262fc described herein.

In some embodiments, HFO-1252zc is prepared from HCFC-262fc according to Step 3B, wherein Step 3B is the vapor phase dehydrohalogenation of HCFC-262fc described herein.

In some embodiments, HCFC-262fc is prepared from HCC-250fb according to Steps 1B and 2B, where Step 1B is the vapor phase hydrofluorination of HCC-250fb described herein, and Step 2B is the liquid phase or vapor phase hydrogenation of HCFO-1242zf described herein.

In some embodiments, HCFC-262fc is prepared from HCFO-1242zf according to Step 2B, where Step 2B is the liquid phase or vapor phase hydrogenation of HCFO-1242zf described herein.

In certain embodiments disclosed herein, HFO-1252zc is prepared according to an integrated process comprising Reaction Scheme C:
**Step 1C:**

   CCl₃CH₂CH₂Cl (HCC-250fb) + HF → CClF₂CH=CH₂ (HCFO-1242zf) + HCl
**Step 2C:**

   CClF₂CH=CH₂ (HCFO-1242zf) + Zn → CF₂=CHCH₃ (HFO-1252zc) + ZnCl₂
where Step 1C is the vapor phase hydrofluorination of HCC-250fb described herein, and Step 2C is the liquid phase dechlorination of HCFO-1242zf described herein.

In some embodiments, the integrated process further comprises separation and/or purification steps to recover the desired intermediate (e.g., HCFO-1242zf) and the desired reaction product HFO-1252zc.

In some embodiments, the present invention relates to a process of preparing HFO-1252zc from HCFO-1242zf according to Step 2C, where Step 2C is the liquid phase dechlorination of HCFO-1242zf described herein.

In certain embodiments disclosed herein, HFO-1252zc is prepared according to an integrated process comprising Reaction Scheme D:
**Step 1D:**

   CCl₃CH₂CH₂Cl (HCC-250fb) + HF → CClF₂CH₂CH₂Cl (HCFC-252fc) + HCl
**Step 2D:**

   CClF₂CH₂CH₂Cl (HCFC-252fc) + Caustic → CClF₂CH=CH₂ (HCFO-1242zf) +H2O +Salt
**Step 3D:**

   CClF₂CH=CH₂ (HCFO-1242zf) + Zn → CF₂=CHCH₃ (HFO-1252zc) + ZnCl₂

   OR

   CClF₂CH=CH₂ (HCFO-1242zf) + H₂ → CClF₂CH₂CH₃ (HCFC-262fc) → CF₂=CHCH₃ (HFO-1252zc) + HCl

   OR
   Base/Solv

   CClF₂CH=CH₂ (HCFO-1242zf) + H₂ → CClF₂CH₂CH₃ (HCFC-262fc) → CF₂=CHCH₃ (HFO-1252zc)
where Step 1D is the liquid phase hydrofluorination of HCC-250fb described herein, Step 2D is the liquid phase dehydrochlorination of HCFC-252fc described herein, and Step 3D is any of (i) the liquid phase reaction of HCFO-1242zf with zinc, (ii) the liquid phase or vapor phase hydrogenation of HCFO-1242zf described herein followed by the vapor phase dehydrohalogenation of HCFC-262fc described herein, or (iii) the liquid phase dehydrochlorination of HCFC-262fc using a strong base described herein.

In some embodiments, the integrated process further comprises separation and/or purification steps to recover the desired intermediate (e.g., HCFO-1242zf and HCFC-262fc) and the desired reaction product HFO-1252zc.

### Compositions

Certain embodiments of the invention disclosed herein relate to compositions comprising, consisting essentially of, or consisting of HCFC-262fc and one or more additional compounds selected from HCC-250fb, HCFC-252fc, HCFO-1242zf, HFC-263fb, HCFC-272fb and HFO-1252zf.

Certain embodiments of the invention disclosed herein relate to compositions comprising, consisting essentially of, or consisting of HFO-1252zc and one or more additional compounds selected from HCFC-262fc, HCC-250fb, HFC-263fb, HFO-1243zf, other HFO-1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf, HCFC-252fc, HFO-1252zf, HCFC-272fb, HFO-1241zf, HFO-1240za, HCFC-253fb and HCFC-251fb.

In one embodiment, compositions according to the present invention comprise, consist essentially of, or consist of (i) HCFC-262fc; (ii) at least one of HCC-250fb and HCFC-252fc; and (iii) one or more additional compounds selected from HFC-263fb and HCFC-272fb. In some embodiments, these compositions are formed by Step 2A or Steps 1A and 2A.

In one embodiment, compositions according to the present invention comprise, consist essentially of, or consist of HCFC-262fc, HCC-250fb, and one or more additional compounds selected from HCFC-252fc, HFC-263fb and HCFC-272fb. In some embodiments, these compositions are formed by Step 2A or Steps 1A and 2A.

In one embodiment, compositions according to the present invention comprise, consist essentially of, or consist of HCFC-262fc, HCFC-252fc and one or more additional compounds selected from HCC-250fb, HFC-263fb and HCFC-272fb. In some embodiments, these compositions are formed by Step 2A or Steps 1A and 2A.

Certain embodiments of the invention disclosed herein relate to compositions comprising, consisting essentially of, or consisting of HFO-1252zc and one or more additional compounds selected from HCFC-262fc, HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf and HCFC-252fc.

In one embodiment, compositions according to the present invention comprise, consist essentially of, or consist of (i) HFO-1252zc; (ii) at least one of HCC-250fb, HCFC-252fc and HCFC-262fc; and (iii) one or more additional compounds selected from HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, and HCFO-1242zf. In some embodiments, these compositions are formed by Steps 1A-3A, or Steps 2A-3A, or Step 3A, or Steps 1B-3B, or Steps 2B-3B, or Step 3B.

In one embodiment, compositions according to the present invention comprise, consist essentially of, or consist of HFO-1252zc, HCFC-262fc, and one or more additional compounds selected from HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf and HCFC-252fc. In some embodiments, these compositions are formed by Steps 1A-3A, or Steps 2A-3A, or Step 3A, or Steps 1B-3B, or Steps 2B-3B, or Step 3B.

In one embodiment, compositions according to the present invention comprise, consist essentially of, or consist of HFO-1252zc, HCFC-262fc, HCFC-252fc and one or more additional compounds selected from HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc and HCFO-1242zf. In some embodiments, these compositions are formed by Steps 1A-3A, or Steps 2A-3A, or Step 3A, or Steps 1B-3B, or Steps 2B-3B, or Step 3B.

Certain embodiments of the invention disclosed herein relate to compositions comprising, consisting essentially of, or consisting of HCFC-262fc and one or more additional compounds selected from HCC-250fb, HCFO-1242zf, HFC-263fb, HFO-1252zf and HCFC-272fb.

In one embodiment, compositions according to the present invention comprise, consist essentially of, or consist of (i) HCFC-262fc; (ii) at least one of HCC-250fb and HCFO-1242zf; and (iii) one or more additional compounds selected from HFC-263fb, HFO-1252zf and HCFC-272fb. In some embodiments, these compositions are formed by Step 2B or Steps 1B and 2B.

In one embodiment, compositions according to the present invention comprise, consist essentially of, or consist of HCFC-262fc, HCC-250fb, and one or more additional compounds selected from HCFO-1242zf, HFC-263fb, HFO-1252zf and HCFC-272fb. In some embodiments, these compositions are formed by Step 2B or Steps 1B and 2B.

In one embodiment, compositions according to the present invention comprise, consist essentially of, or consist of HCFC-262fc, HCFO-1242zf and one or more additional compounds selected from HCC-250fb, HFC-263fb, HFO-1252zf and HCFC-272fb. In some embodiments, these compositions are formed by Step 2B or Steps 1B and 2B.

Certain embodiments of the invention disclosed herein relate to compositions comprising, consisting essentially of, or consisting of HCFC-262fc and one or more additional compounds selected from propane, propylene, HCFC-252dc, HCFC-262db, HFC-272fb, HCFO-1232xf, HCFO-1242zf, HFO-1243zf, E-HCFO-1251zd, Z-HCFO-1251zd, HFO-1252zf, E-HFO-1261ze, Z-HFO-1261ze, HCFO-1242 isomer(s) and HCO-1260. In some embodiments, these compositions are formed by Step 2B or Steps 1B and 2B.

Certain embodiments of the invention disclosed herein relate to compositions comprising, consisting essentially of, or consisting of HCFC-262fc and one or more additional compounds selected from ethane, HFC-152a, HFC-253db, HCFO-1232 isomer(s), HCFO-1242 isomer(s), HCFO-1242zf, HFO-1243zf and HFO-1252zc. In some embodiments, these compositions are formed by Step 2B or Steps 1B and 2B.

Certain embodiments of the invention disclosed herein relate to compositions comprising, consisting essentially of, or consisting of HCFC-262fc and one or more additional compounds selected from propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween. In some embodiments, these compositions are formed by Step 2B or Steps 1B and 2B.

In one embodiment, compositions according to the present invention comprise, consist essentially of, or consist of (i) HCFC-262fc; (ii) at least one of HFO-1252zc, HCFC-262db, HFC-272fb, HFO-1243zf and HCFO-1242zf; and (iii) one or more additional compounds selected from propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HFC-252 isomer(s). In some embodiments, these compositions are formed by Step 2B or Steps 1B and 2B.

In one embodiment, compositions according to the present invention comprise, consist essentially of, or consist of (i) HCFC-262fc; (ii) at least one of HFO-1243zf, HFO-1252zf and HCFO-1242zf; and (iii) one or more additional compounds selected from propane, propylene, HCFC-252dc, HCFC-262db, HFC-272fb, HCFO-1232xf, HCFO-1242zf, HFO-1243zf, E-HCFO-1251zd, Z-HCFO-1251zd, HFO-1252zf, E-HFO-1261ze, Z-HFO-1261ze, and HCO-1260. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween. In some embodiments, these compositions are formed by Step 2B or Steps 1B and 2B.

In one embodiment, compositions according to the present invention comprise, consist essentially of, or consist of (i) HCFC-262fc; (ii) at least one of HFO-1243zf, HFO-1252zf and HCFO-1242zf; and (iii) one or more additional compounds selected from propane, propylene, HCFC-252dc, HCFC-262db, HCFO-1232xf, HCFO-1242zf, HFO-1243zf, E-HCFO-1251zd, Z-HCFO-1251zd, HFO-1252zf, E-HFO-1261ze, Z-HFO-1261ze and HCFO-1242 isomer(s). In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween. In some embodiments, these compositions are formed by Step 2B or Steps 1B and 2B.

In one embodiment, compositions according to the present invention comprise, consist essentially of, or consist of (i) HCFC-262fc; (ii) at least one of HFO-1243zf, HFO-1252zf and HCFO-1242zf; and (iii) one or more additional compounds selected from HCFC-262db, HCFO-1232xf, HCFO-1242zf, HFO-1243zf, E-HCFO-1251zd, Z-HCFO-1251zd, HFO-1252zf and E-HFO-1261ze. In some embodiments, the HCFC-262fc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween. In some embodiments, these compositions are formed by Step 2B or Steps 1B and 2B.

Certain embodiments of the invention disclosed herein relate to compositions comprising, consisting essentially of, or consisting of HFO-1252zc and one or more additional compounds selected from HCFC-262fc, HCC-250fb, HFC-263fb, HCFO-1242zf, HFO-1252zf, HFO-1243zf and HCFC-252fc.

In one embodiment, compositions according to the present invention comprise, consist essentially of, or consist of (i) HFO-1252zc; (ii) at least one of HCC-250fb and HCFO-1242zf; and (iii) one or more additional compounds selected from HCFC-262fc, HFC-263fb, HFO-1252zf, HFO-1243zf and HCFC-252fc. In some embodiments, these compositions are formed by Steps 1C-2C or Step 2C.

In one embodiment, compositions according to the present invention comprise, consist essentially of, or consist of HFO-1252zc, HCFO-1242zf, and one or more additional compounds selected from HCFC-262fc, HCC-250fb, HFC-263fb, HFO-1252zf, HFO-1243zf and HCFC-252fc. In some embodiments, these compositions are formed by Steps 1C-2C or Step 2C.

In one embodiment, compositions according to the present invention comprise, consist essentially of, or consist of HCFC-262fc, HCC-250fb and HCFO-1242zf, and one or more additional compounds selected from HCFC-252fc, HFC-263fb, HCFC-272fb and HFO-1252zf.

Certain embodiments of the invention disclosed herein relate to compositions comprising, consisting essentially of, or consisting of HFO-1252zc, one or more of HCC-250fb, HCFC-252fc, HCFC-262fc and HCFO-1242zf, and one or more additional compounds selected from HFC-263fb, other 1252 isomers, ethane, ethylene, HFC-23, HCFC-22, HFO-1132a, propane, HFC-143a, HCFC-142b, HFO-1234yf, HFO-1234ze, HFO-1225zc and HCFO-1242zf.

In one embodiment, compositions according to the present invention comprise, consist essentially of, or consist of HFO-1252zc, HCFC-262fc, HCC-250fb and HCFC-252fc, and one or more additional compounds selected from HFC-263fb, HCFO-1242zf, HFO-1252zf, HFO-1243zf and HCFC-252fc.

In one embodiment, compositions according to the present invention comprise, consist essentially of, or consist of HFO-1252zc, HCFC-262fc, HCC-250fb and HCFO-1242zf, and one or more additional compounds selected from HCFC-252fc, HFC-263fb, other 1252 isomers, ethane, ethylene, HFC-23, HCFC-22, HFO-1132a, propane, HFC-143a, HCFC-142b, HFO-1234yf, HFO-1234ze, HFO-1241zf, HFO-1240za and HFO-1225zc.

In one embodiment, compositions according to the present invention comprise, consist essentially of, or consist of HFO-1252zc, HCC-250fb and HCFO-1242zf, and one or more additional compounds selected from HFC-263fb, other 1252 isomers, ethane, ethylene, HFC-23, HCFC-22, HFO-1132a, propane, HFC-143a, HCFC-142b, HFO-1234yf, HFO-1234ze HFO-1241zf, HFO-1240za and HFO-1225zc.

In one embodiment, compositions according to the present invention comprise, consist essentially of, or consist of HFO-1252zc and one or more additional compounds selected from HFC-263fb, HCFC-262fc, E-HFO-1251zb, Z-HFO-1251zb, HCFC-272fb, methane, HFC-32, ethylene, ethane, HFO-1132a, HCC-40, HCFC-22, ethylene oxide, E-HFO-1261ze, HFO-1252 isomers, HFC-281fa, HCC-30, C₄H₃ClF₄, C₄H₆F₄ (I), C₄H₆F₄ (II), C₆F₁₂, HFO-1252zc dimer (I) and HFO-1252zc dimer (II) (HFO-1252zc dimers). In some embodiments, the one or more additional compounds comprise at least HFO-1252zc dimer.

In one embodiment, compositions according to the present invention comprise, consist essentially of, or consist of HFO-1252zc and one or more additional compounds selected from HFC-263fb, HCFC-262fc, E-HFO-1251zb, Z-HFO-1251zb, HCFC-272fb, methane, HFC-32, ethylene, ethane, HFO-1132a, HCC-40, HCFC-22, E-HFO-1261ze, HFO-1252 isomers, HFC-281fa, HCC-30, C₄H₃ClF₄, C₄H₆F₄ (I), C₄H₆F₄ (II), C₆F₁₂, HFO-1252zc dimer (I) and HFO-1252zc dimer (II). In some embodiments, the one or more additional compounds comprise at least HFO-1252zc dimer.

In one embodiment, compositions according to the present invention comprise, consist essentially of, or consist of HFO-1252zc and one or more additional compounds selected from HFC-263fb, HCFC-262fc, E-HFO-1251zb, Z-HFO-1251zb, HCFC-272fb, HFO-1252zc dimer (I) and HFO-1252zc dimer (II). In some embodiments, the one or more additional compounds comprise at least HFO-1252zc dimer.

In one embodiment, compositions according to the present invention comprise, consist essentially of, or consist of HCFO-1242zf and one or more additional compounds selected from HCC-250fb, HCFC-253fb, HCFC-252fc, HCFC-251fb, HCFC-1243zf, HCFC-1241zf and HCFC-1240za. In one embodiment, the additional compound HCFC-1243zf may constitute about 30 wt.% to about 60 wt.%, or about 30 wt.% to about 50 wt.%, or about 30 wt.% to about 45 wt.%, based on the total weight of the composition.

In one embodiment, compositions according to the present invention comprise, consist essentially of, or consist of HCFC-1243zf and one or more additional compounds selected from HCC-250fb, HCFC-253fb, HCFC-252fc, HCFC-251fb, HCFO-1242zf, HCFC-1241zf and HCFC-1240za.

The one or more additional members of the compositions disclosed herein may be selected from one or more HFCs, HCCs, HCFCs, HFOs, HCFOs, C₂-C₄ alkanes, C₂-C₄ alkenes and t-butoxy-fluoropropenes.

In any of the composition embodiments disclosed herein, the total amount of additional compounds may be between greater than 0 and about 15 wt.% expressed by GC-FID peak area percent, e.g., the total amount of the composition, and all values and ranges therebetween. In any of the composition embodiments disclosed herein, the total amount of additional compounds may be between greater than 0 and less than one of 15 percent, 14 percent, 13 percent, 12 percent, 11 percent, 10 percent, 9 percent, 9 percent, 7 percent, 6 percent, 5 percent, 4 percent, 3 percent, 2 percent, or 1 percent and all values and ranges therebetween.

In any of the composition embodiments disclosed herein, the total amount of additional compounds may be between greater than 0 and less than 0.1 percent, greater than 0 and less than 0.01 percent, between greater than 0.0001 and less than 0.3 percent, greater than 0.0001 and less than 0.2 percent, greater than 0.0001 and less than 0.1 percent, greater than 0.0001 and less than 0.01 percent, or greater than 0.0001 and less than 0.001 percent based on the total amount of the composition and all values and ranges therebetween.

In any of the composition embodiments disclosed herein, each additional compound may be present in an amount of between:
a) greater than 0 and less than 4 percent,
b) greater than 0 and less than 3 percent,
c) greater than 0 and less than 2 percent,
d) greater than 0 and less than 1 percent,
e) greater than 0 and less than 0.5 percent,
f) greater than 0 and less than 0.1 percent,
g) greater than 0 and less than 0.01 percent,
h) greater than 0 and less than 0.005 percent,
i) greater than 0.001 and less than 4 percent,
j) greater than 0.001 and less than 3 percent,
k) greater than 0.001 and less than 2 percent,
l) greater than 0.001 and less than 1 percent,
m) greater than 0.001 and less than 0.5 percent,
n) greater than 0.001and less than 0.1 percent,
o) greater than 0.001 and less than 0.01 percent, or
p) greater than 0.001 and less than 0.005 percent,
based on the total amount of the composition *with the proviso* that the total amount of the additional compounds is greater than 0.0001 and less than 15%, greater than 0.0001 and less than 10%, greater than 0.0001 and less than 8%, greater than 0.0001 and less than 7%, greater than 0.0001 and less than 6%, greater than 0.0001 and less than 5%, greater than 0.0001 and less than 4%, greater than 0.0001 and less than 3%, greater than 0.0001 and less than 2%, greater than 0.0001 and less than 1%, greater than 0.0001 and less than 0.5%, or greater than 0.0001 and less than 0.1.

In a preferred embodiment, compositions according to the present invention are free of or substantially free of Group A Fluorinated Substances. In one embodiment, as used herein, "Group A Fluorinated Substances" includes any substance that (i) contains at least one fully fluorinated methyl (-CF₃) or methylene (-CF₂-) carbon atom (without any H/Cl/Br/I attached to it); and (ii) meets the criterion for persistence in soil/sediment and water established in Annex XIII (Section 1.1.1) of the European Union's REACH Regulation (https://reachonline.eu/reach/en/annex-xiii-1-1.1-1.1.1.html as accessed on May 2, 2023) and referenced in the Annex XV Restriction Report dated March 22, 2023, the disclosure of which is hereby incorporated by reference (https://echa.europa.eu/documents/10162/f605d4b5-7c17-7414-8823-b49b9fd43aea as accessed on May 2, 2023).

In another embodiment, as used herein, "Group A Fluorinated Substances" includes any substance that has a Henry's Law constant < 250 Pa*m³/mol and contains at least one fully fluorinated methyl (-CF₃) or methylene (-CF₂-) carbon atom (without any H/Cl/Br/I attached to it).

In embodiments, Group A Fluorinated Substances include, but are not limited to, TFA.

The phrase "free of" as used herein with respect to the presence of Group A Fluorinated Substances in the present compositions means that the amount of such substances in the compositions is sufficiently low so as to not be detectable, including but not limited to 0%, when measured by gas chromatography with a flame ionization detector, gas chromatography with a mass detector by analysis of a gas sample or liquid sample, and/or ion chromatography by analysis of a water sample after bubbling the thermal fluid through water. Such methodologies are well known to those skilled in the art. The phrase "substantially free of" as used herein with respect to the presence of Group A Fluorinated Substances in the present compositions means that the amount of such substances in the compositions is > 0 wt.% and < 5 wt.%, or > 0 wt.% and < 4 wt.%, or > 0 wt.% and < 3 wt.%, or > 0 wt.% and < 2 wt.%, or > 0 wt.% and < 1 wt.%, and all values and ranges therebetween, when measured by gas chromatographic (GC) techniques, for example gas chromatography (GC) with a flame ionization or electron-capture detector, or GC coupled with a mass detector (gas chromatography/mass spectral (GC/MS) method), by ion chromatograph(IC) or ion chromatography mass spectrometry (IC-MS) techniques, or by high-performance liquid chromatography (HPLC) or high-performance liquid chromatography mass spectrometry (HPLC-MS) techniques. The TFA analytical standard may be used in either gas chromatography or ion chromatography and is available from, for example, Sigma Aldrich.

In a preferred embodiment, degradation products of compositions according to the present invention are free of or substantially free of Group A Fluorinated Substances. The phrase "free of" as used herein with respect to the formation of Group A Fluorinated Substances as degradation products of the present compositions means that the theoretical molar yield of such substances in environmental compartments of air, soil/sediment and water produced during tropospheric degradation of the compositions is sufficiently low so as to not be detectable, including but not limited to 0%, when measured by GC techniques, for example GC with a flame ionization or electron-capture detector or GC/MS method, by IC or IC-MS techniques, or by HPLC or HPLC-MS techniques. The phrase "substantially free of" as used herein with respect to the formation of Group A Fluorinated Substances by the present compositions means that the theoretical molar yield of such substances in environmental compartments of air, soil/sediment and water produced during tropospheric degradation of the compositions is > 0% and < 5%, or > 0% and < 4%, or > 0% and < 3%, or > 0% and < 2%, or > 0% and < 1%, and all values and ranges therebetween, when measured by GC techniques, for example GC with a flame ionization or electron-capture detector or GC/MS method, by IC or IC-MS techniques, or by HPLC or HPLC-MS techniques.

Certain embodiments of the invention disclosed herein relate to compositions comprising, consisting essentially of, or consisting of HFO-1252zc, and are free of or substantially free of Group A Fluorinated Substances. In some embodiments, compositions of the present invention comprise, consist essentially of, or consist of HFO-1252zc, and degradation products of such compositions are free of or substantially free of Group A Fluorinated Substances, as defined herein.

Certain embodiments of the invention disclosed herein relate to compositions comprising, consisting essentially of, or consisting of HFO-1252zc and one or more additional members selected from HFCs, HCCs, HCFCs, HFOs, HCFOs, C₂-C₄ alkanes, C₂-C₄ alkenes and t-butoxy-fluoropropenes. In some embodiments, such compositions are free of or substantially free of Group A Fluorinated Substances and/or degradation products of such compositions are free of or substantially free of Group A Fluorinated Substances, as defined herein.

Certain embodiments of the invention disclosed herein relate to blend compositions comprising, consisting essentially of, or consisting of HFO-1252zc and one or more refrigerant compounds selected from HFCs, HCCs, HCFCs, HFOs, HFCOs, C₂-C₄ alkanes, C₂-C₄ alkenes and t-butoxy-fluoropropenes. In some embodiments, such blend compositions are free of or substantially free of Group A Fluorinated Substances and/or degradation products of such blend compositions are free of or substantially free of Group A Fluorinated Substances, as defined herein.

In some embodiments, at least a portion of any of the compositions disclosed herein comprises reclaimed materials.

### Systems and Processes

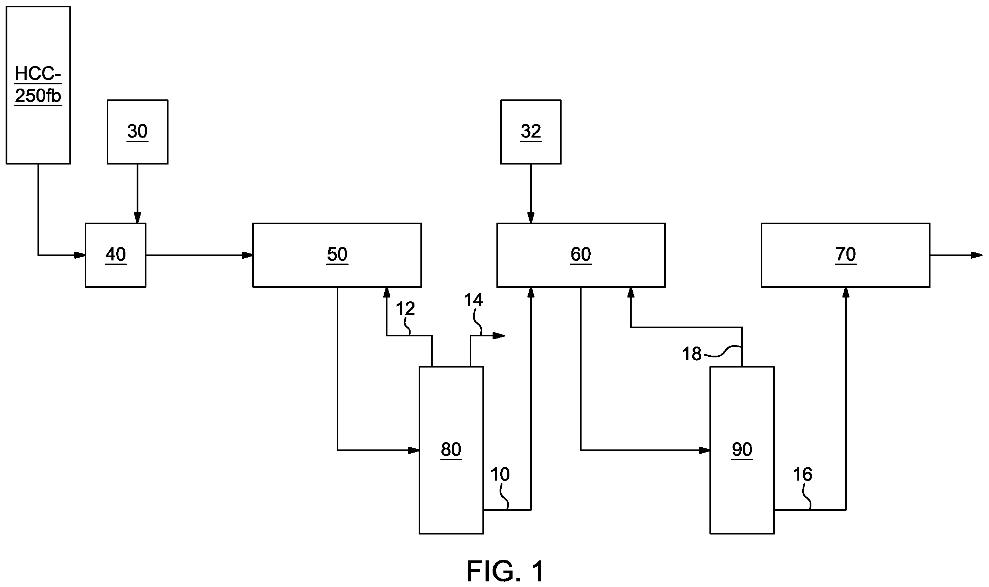

Fig. 1 illustrates a first embodiment of the present invention for the production of HCFC-262fc and/or HFO-1252zc according to one or more Steps of Reaction Scheme A. Referring to Fig. 1, in one embodiment, three reactors or reaction zones 50, 60, 70, as well as first and second separators (e.g., distillation columns) 80, 90 are depicted. The process can be operated in batch mode, semi-continuously or continuously. The starting feed includes HCC-250fb and is introduced to the first reactor 50 for contact with HF to produce the HCFC-252fc intermediate. In some embodiments, the HF is stored in a pressurized vessel 30. In some embodiments, the HCC-250fb and HF may be pre-mixed in a mixer 40 and fed to the reactor 50. In some embodiments, the HCC-250fb and HF may be co-fed and mixed in the reactor 50 upstream of the catalyst bed.

Preferably, the first reactor 50 is configured for a liquid phase hydrofluorination reaction. The first reactor 50 is heated and the reaction proceeds to produce a product mixture containing HCFC-252fc. The HCFC-252fc reactor product mixture is withdrawn and preferably further processed to purify the HCFC-252fc, such as using conventional separation techniques, recycling of unreacted HCC-250fb and/or HF to the first reactor 50, removal of byproduct HCl, and the like. For example, in some embodiments, the HCFC-252fc reactor product mixture is fed from the first reactor 50 to a first separator system 80 for recovery of the HCFC-252fc (stream 10), separation and recycling of the unreacted HCC-250fb and optionally unreacted HF (stream 12), and removal of the HCl (stream 14). Removal of unreacted HCC-250fb from the product mixture increases the relative concentration/amount of HCFC-252fc.

The HCFC-252fc (stream 10) is then fed to a second reactor or reaction zone 60. The second reactor is preferably suitable for hydrogenation of the HCFC-252fc, namely to convert the HCFC-252fc to HCFC-262fc. The second reactor 60 may be configured for either liquid phase reactions or for vapor phase reactions. HCFC-252fc and H₂ can be premixed and fed to the second reaction zone 60 or mixed in the reaction zone 60 upstream of the catalyst bed. In some embodiments, the H₂ is stored in a pressurized vessel 32. In some embodiments, a single pressurized vessel serves as a source for both the HF and H₂. The second reactor 60 is heated and the reaction proceeds to produce a product mixture of a second intermediate comprising HCFC-262fc. The product mixture is withdrawn from the second reaction zone 60 and preferably further processed to purify the HCFC-262fc, such as using conventional separation techniques, recycling of unreacted HCFC-252fc to the second reactor 60, and the like. For example, in some embodiments, the HCFC-262fc reactor product mixture is fed from the second reactor 60 to a second separator system 90 for recovery of the HCFC-262fc (stream 16), and separation and recycling of the unreacted HCFC-252fc and optionally unreacted hydrogen (stream 18). Removal of unreacted HCFC-252fc from the product mixture increases the relative concentration/amount of HCFC-262fc.

The HCFC-262fc intermediate (stream 16) can then be converted to HFO-1252zc in a third reaction zone or reactor 70 by dehydrohalogenation. The third reactor 70 is preferably configured for vapor phase reactions. HCFC-262fc is fed to the third reaction zone 70 for dehydrochlorination. The third reactor 70 may be heated and the reaction proceeds to produce a product mixture comprising HFO-1252zc. The product mixture is withdrawn from the third reactor 70 and preferably further processed to purify the HFO-1252zc, such as using conventional separation techniques, recycling of unreacted HCFC-262fc to the third reactor 70, and the like. Removal of unreacted HCFC-262fc from the product mixture increases the relative concentration/amount of HFO-1252zc.

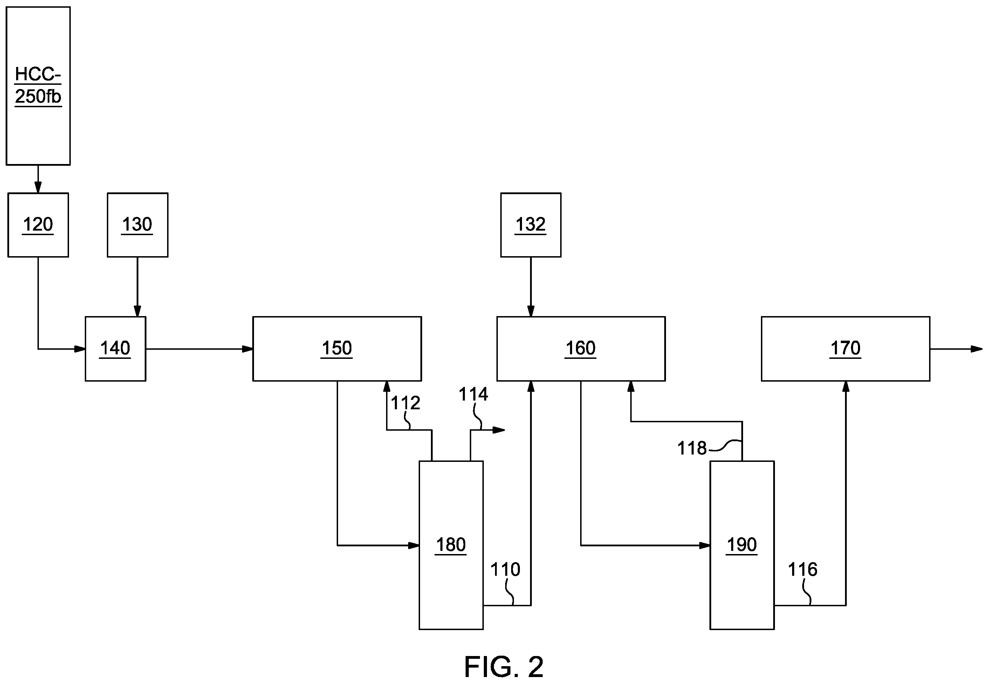

Fig. 2 illustrates a first embodiment of the present invention for the production of HCFC-262fc and/or HFO-1252zc according to one or more Steps of Reaction Scheme B. Referring to Fig. 2, in one embodiment, three reactors or reaction zones 150, 160, 170, as well as first and second separators (e.g., distillation columns) 180, 190, are depicted. The process can be operated in batch mode, semi-continuously or continuously. The starting feed includes HCC-250fb and is introduced to the first reactor 150 for contact with HF to produce the HCFO-1242zf intermediate. In some embodiments, the HF is stored in a pressurized vessel 130. In some embodiments, the HCC-250fb and HF may be pre-mixed in a mixer 140 and fed to the reactor 150. In some embodiments, the HCC-250fb and HF may be co-fed and mixed in the reactor 150 upstream of the catalyst bed. In some embodiments, the HCC-250fb feed is preferably first vaporized in a vaporizer 120, and then mixed with HF in the mixer 140 and fed to the first reaction zone 150 for contact with the catalyst bed.

Preferably, the first reactor 150 is configured for a vapor phase hydrofluorination reaction. The first reactor 150 is heated and the reaction proceeds to produce a product mixture containing HCFO-1242zf. The HCFO-1242zf reactor product mixture is withdrawn and preferably further processed to purify the HCFO-1242zf, such as using conventional separation techniques, recycling of unreacted HCC-250fb and/or HF to the first reactor 150, removal of byproduct HCl, and the like. For example, in some embodiments, the HCFO-1242zf reactor product mixture is fed from the first reactor 150 to a first separator system 180 for recovery of the HCFO-1242zf (stream 110), separation and recycling of the unreacted HCC-250fb and optionally unreacted HF (stream 112), and removal of the HCl (stream 114). Removal of unreacted HCC-250fb from the product mixture increases the relative concentration/amount of HCFO-1242zf.

The HCFO-1242zf (stream 110) is then fed to a second reactor or reaction zone 160. The second reactor is preferably suitable for hydrogenation of the HCFO-1242zf, namely to convert the HCFO-1242zf to HCFC-262fc. The second reactor 160 may be configured for either liquid phase reactions or for vapor phase reactions. HCFO-1242zf and H₂ can be premixed and fed to the second reaction zone 160 or mixed in the reaction zone 160 upstream of the catalyst bed. In some embodiments, the H₂ is stored in a pressurized vessel 132. In some embodiments, a single pressurized vessel serves as a source for both the HF and H₂. The second reactor 160 is heated and the reaction proceeds to produce a product mixture of a second intermediate comprising HCFC-262fc. The product mixture is withdrawn from the second reaction zone 160 and preferably further processed to purify the HCFC-262fc, such as using conventional separation techniques, recycling of unreacted HCFO-1242zf to the second reactor 60, and the like. For example, in some embodiments, the HCFC-262fc reactor product mixture is fed from the second reactor 160 to a second separator system 190 for recovery of the HCFC-262fc (stream 116), and separation and recycling of the unreacted HCFC-252fc and optionally unreacted hydrogen (stream 118). Removal of unreacted HCFO-1242zf from the product mixture increases the relative concentration/amount of HCFC-262fc.

The HCFC-262fc intermediate (stream 116) can then be converted to HFO-1252zc in a third reaction zone or reactor 170 by dehydrohalogenation. The third reactor 170 is preferably configured for vapor phase reactions. HCFC-262fc is fed to the third reaction zone 170 for dehydrochlorination. The third reactor 170 may be heated and the reaction proceeds to produce a product mixture comprising HFO-1252zc. The product mixture is withdrawn from the third reactor 170 and preferably further processed to purify the HFO-1252zc, such as using conventional separation techniques, recycling of unreacted HCFC-262fc to the third reactor 170, and the like. Removal of unreacted HCFC-262fc from the product mixture increases the relative concentration/amount of HFO-1252zc.

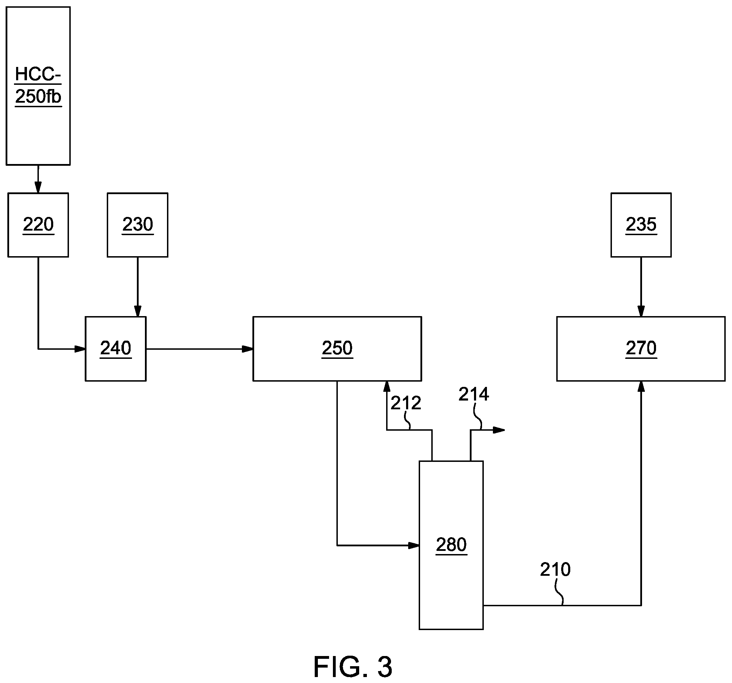

Fig. 3 illustrates a first embodiment of the present invention for the production of HFO-1252zc according to one or more Steps of Reaction Scheme C. Referring to Fig. 3, in one embodiment, two reactors or reaction zones 250, 270, as well as a separator (e.g., distillation column) 280, are depicted. The process can be operated in batch mode, semi-continuously or continuously. The starting feed includes HCC-250fb and is introduced to the first reactor 250 for contact with HF to produce the HCFO-1242zf intermediate. In some embodiments, the HF is stored in a pressurized vessel 230. In some embodiments, the HCC-250fb and HF may be pre-mixed in a mixer 240 and fed to the reactor 250. In some embodiments, the HCC-250fb and HF may be co-fed and mixed in the reactor 250 upstream of the catalyst bed. In some embodiments, the HCC-250fb feed is preferably first vaporized in a vaporizer 220, and then mixed with HF in the mixer 240 and fed to the first reaction zone 250 for contact with the catalyst bed.

Preferably, the first reactor 250 is configured for a vapor phase hydrofluorination reaction. The first reactor 250 is heated and the reaction proceeds to produce a product mixture containing HCFO-1242zf. The HCFO-1242zf reactor product mixture is withdrawn and preferably further processed to purify the HCFO-1242zf, such as using conventional separation techniques, recycling of unreacted HCC-250fb and/or HF to the first reactor 250, removal of byproduct HCl, and the like. For example, in some embodiments, the HCFO-1242zf reactor product mixture is fed from the first reactor 250 to a first separator system 280 for recovery of the HCFO-1242zf (stream 210), separation and recycling of the unreacted HCC-250fb and optionally unreacted HF (stream 212), and removal of the HCl (stream 214). Removal of unreacted HCC-250fb from the product mixture increases the relative concentration/amount of HCFO-1242zf.

The HCFO-1242zf (stream 210) is then fed to a second reactor or reaction zone 270. The second reactor 270 is preferably suitable for dechlorination of the HCFO-1242zf with zinc, namely to convert the HCFO-1242zf to HFO-1252zc. Zinc is supplied to the second reactor 270 from a source 235. The second reactor 270 is preferably configured for liquid phase reactions. HCFO-1242zf and zinc can be premixed and fed to the second reaction zone 270 or mixed in the reaction zone 270 upstream of the catalyst bed. The second reactor 270 is heated and the reaction proceeds to produce a product mixture comprising HFO-1252zc. The product mixture is withdrawn from the second reactor 270 and preferably further processed to purify the HFO-1252zc, such as using conventional separation techniques, recycling of unreacted HCFO-1242zf to the second reactor 270, and the like. Removal of unreacted HCFO-1242zf from the product mixture increases the relative concentration/amount of HFO-1252zc.

Certain embodiments described herein relate to a system comprising a self-contained source of HCC-250fb, pressurized source containers of hydrogen and/or hydrogen fluoride, one or more vaporizer, at least one mixer, at least first and second and optionally third serially arranged reactors respectfully producing intermediate product mixtures, and the most downstream reactor providing a final product mixture, and one or more separator systems. In some embodiments, the first reactor contains a flow through bed of fluorination catalyst. In some embodiments, the second reactor contains a flow through bed of hydrogenation catalyst. In some embodiments, each upstream reactor includes a discharge line to convey intermediate product mixtures to respective separator systems to recover the desired intermediate products, which, in turn, are respectively conveyed to a downstream reactor.

Aspects of the present invention will now be described with reference to the following Examples.

### EXAMPLES

### Example 1: Vapor Phase Hydrogenation of 252fc to 262fc

Hydrogenation of 252fc to 262fc in vapor phase by 0.02% Pd/Al2O3: In a 12-inch long ½" OD Monel reactor, 5ml 0.02% Pd/Al2O3 is loaded. The catalyst is treated at 200C by flow H2 for 1 hour. Then the reactor is heated to 60C. 252fc is fed at 2ml/hr rate, H2 and N2 are fed at 11sccm and 10 sccm, respectively. The reactor effluent is analyzed by on-line GC-MS-FID. The results of analysis show >30% conversion of 262fc and 85% selectivity to 263fb. 262fa (CHF2CH2CH2Cl) and 272fb (CHF2CH2CH3) are also found in the product.

### Example 2: Pyrolysis of 262fc to 1252zc in an empty gold-lined reactor tube

HCFC-262fc was fed by pump into an empty 10 inches long ½" OD gold-lined tube reactor. The reaction test conditions are listed on Table 2 below. The reactor effluent was analyzed by online GC-MS-FID at each test condition. The results of the analysis are listed in Tables 2 and 3 below.

**Table 2**

| **T** | **262fc** | **P** | **N2** | **GC-FID** | | | | | | |
|---|---|---|---|---|---|---|---|---|---|---|
| **C** | **cc/hr** | **psig** | **Sccm** | **1252zc** | **263fb** | **262fc** | **E-1251zb** | **Z-1251zb** | **272fb** | **others** |
| 100 | 0.50 | 5.0 | 4.97 | 3.13% | 1.47% | 91.80% | 0.99% | 1.17% | 0.86% | 0.58% |
| 100 | 0.50 | 5.1 | 4.96 | 3.12% | 1.48% | 91.91% | 0.98% | 1.16% | 0.83% | 0.52% |
| 460 | 0.50 | 4.9 | 4.97 | 7.96% | 2.22% | 85.27% | 1.44% | 1.66% | 0.96% | 0.48% |
| 460 | 0.50 | 5.0 | 5.00 | 6.71% | 1.89% | 87.35% | 1.26% | 1.44% | 0.88% | 0.47% |
| 460 | 0.50 | 5.0 | 5.00 | 6.52% | 1.97% | 87.24% | 1.32% | 1.51% | 0.89% | 0.54% |
| 460 | 0.50 | 5.0 | 4.97 | 6.04% | 1.75% | 88.56% | 1.08% | 1.23% | 0.84% | 0.50% |
| 480 | 0.50 | 5.0 | 5.00 | 9.38% | 1.89% | 84.89% | 1.17% | 1.33% | 0.83% | 0.51% |
| 480 | 0.50 | 5.0 | 5.00 | 9.24% | 1.77% | 85.44% | 1.04% | 1.18% | 0.77% | 0.56% |
| 480 | 0.50 | 4.7 | 4.98 | 8.85% | 1.71% | 86.03% | 1.00% | 1.13% | 0.75% | 0.54% |
| 500 | 0.50 | 5.2 | 5.00 | 17.19% | 1.86% | 77.28% | 1.13% | 1.27% | 0.67% | 0.59% |
| 500 | 0.50 | 5.2 | 5.00 | 17.63% | 1.93% | 76.75% | 1.15% | 1.29% | 0.68% | 0.57% |
| 500 | 0.50 | 5.1 | 5.00 | 16.50% | 1.86% | 78.16% | 1.07% | 1.21% | 0.68% | 0.52% |
| 509 | 0.50 | 5.0 | 5.01 | 23.51% | 1.90% | 71.12% | 1.08% | 1.21% | 0.62% | 0.58% |
| 507 | 0.50 | 5.3 | 5.01 | 20.99% | 1.72% | 74.26% | 0.91% | 1.02% | 0.59% | 0.51% |
| 503 | 0.50 | 5.0 | 5.01 | 18.34% | 1.76% | 76.86% | 0.91% | 1.04% | 0.62% | 0.47% |
| 450 | 1.00 | 30.0 | 0.00 | 7.54% | 1.38% | 87.97% | 0.74% | 0.87% | 0.70% | 0.81% |
| 450 | 1.00 | 30.0 | 0.00 | 6.83% | 1.26% | 89.09% | 0.65% | 0.76% | 0.66% | 0.75% |
| 450 | 1.00 | 30.0 | 0.00 | 6.57% | 1.26% | 89.32% | 0.68% | 0.78% | 0.63% | 0.76% |
| 460 | 1.00 | 30.0 | 0.00 | 8.76% | 1.32% | 86.98% | 0.69% | 0.79% | 0.61% | 0.84% |
| 460 | 1.00 | 30.0 | 0.00 | 8.07% | 1.25% | 87.95% | 0.62% | 0.71% | 0.60% | 0.81% |
| 470 | 1.00 | 30.0 | 0.00 | 11.34% | 1.31% | 84.50% | 0.63% | 0.72% | 0.57% | 0.95% |
| 470 | 1.00 | 30.0 | 0.00 | 10.74% | 1.29% | 85.17% | 0.61% | 0.70% | 0.56% | 0.93% |
| 480 | 1.00 | 30.0 | 0.00 | 12.29% | 1.35% | 83.53% | 0.56% | 0.66% | 0.62% | 0.99% |
| 459 | 1.50 | 60.0 | 0.00 | 18.46% | 1.59% | 74.90% | 0.44% | 0.51% | 0.53% | 3.59% |
| 459 | 1.50 | 60.0 | 0.00 | 9.56% | 0.88% | 87.60% | 0.31% | 0.35% | 0.40% | 0.89% |
| 459 | 1.50 | 60.0 | 0.00 | 7.20% | 0.82% | 90.19% | 0.31% | 0.35% | 0.38% | 0.75% |
| 459 | 1.50 | 60.0 | 0.00 | 7.64% | 0.76% | 89.88% | 0.28% | 0.32% | 0.35% | 0.76% |
| 470 | 1.50 | 60.0 | 0.00 | 14.57% | 0.87% | 82.11% | 0.30% | 0.34% | 0.33% | 1.49% |
| 470 | 1.50 | 60.0 | 0.00 | 12.81% | 0.81% | 84.09% | 0.29% | 0.33% | 0.31% | 1.35% |
| 470 | 1.50 | 60.0 | 0.00 | 12.39% | 0.77% | 84.60% | 0.26% | 0.31% | 0.28% | 1.39% |
| 480 | 1.50 | 60.0 | 0.00 | 17.73% | 0.82% | 78.24% | 0.27% | 0.32% | 0.27% | 2.35% |
| 480 | 1.50 | 60.0 | 0.00 | 17.80% | 0.86% | 78.32% | 0.27% | 0.31% | 0.25% | 2.19% |
| 480 | 1.50 | 60.0 | 0.00 | 17.78% | 0.80% | 78.20% | 0.25% | 0.29% | 0.24% | 2.43% |
| 490 | 1.50 | 60.0 | 0.00 | 22.80% | 0.87% | 72.28% | 0.26% | 0.30% | 0.23% | 3.26% |
| 490 | 1.50 | 60.0 | 0.00 | 21.78% | 0.81% | 73.69% | 0.24% | 0.28% | 0.22% | 2.97% |

**Table 3: Detailed GC analysis of product produced at 490°C**

| **Compounds** | **GC-FID area%** |
|---|---|
| Methane | 0.0771% |
| HFC-32 | 0.0008% |
| Ethylene | 0.0425% |
| Ethane | 0.0097% |
| 1132a | 0.0026% |
| HC-40 | 0.0048% |
| HCFC-22 | 0.0098% |
| ethylene oxide | 0.0017% |
| HFO-1261ze-E | 0.0278% |
| 1252zc | 23.1407% |
| 263fb | 0.2173% |
| 1252 isomer | 0.1365% |
| 281fa | 0.2447% |
| HC-30 | 0.0282% |
| C4H3ClF4 | 0.0168% |
| 262fc | 73.3758% |
| E-1251zb | 0.1832% |
| Z-1251zb | 0.1794% |
| 272fb | 0.1632% |
| C4H6F4 | 0.0248% |
| C4H6F4 | 0.0501% |
| C6F12 | 0.0458% |
| 1252-dimer | 0.7631% |
| 1252dimer | 1.0084% |
| Others | 0.2454% |

### Example 3: Pyrolysis of 262fc to 1252zc in empty Inconel 625 reactor tube

HCFC-262fc was fed by pump into an empty 10 inches long ½" OD Inconel 625 tube reactor. The reaction test conditions are listed on Table 4 below. The reactor effluent was analyzed by online GC-MS-FID at each test condition. The results of the analysis are listed in Tables 4 and 5 below.

**Table 4**

| **T** | **P** | **262fc** | **GC-FID area%** | | | | | | | | |
|---|---|---|---|---|---|---|---|---|---|---|---|
| **C** | **psig** | **cc/hr** | **1252zc %** | **263fb %** | **262fc %** | **E-1251zb %** | **Z-1251zb %** | **272fb %** | **1252-dimer %** | **1252-dimer %** | **Others %** |
| 460 | 49.9 | 1.50 | 14.40 | 0.82 | 83.08 | 0.35 | 0.39 | 0.17 | 0.31 | 0.49 | 0.00 |
| 460 | 50.0 | 1.50 | 12.25 | 0.72 | 85.61 | 0.32 | 0.37 | 0.16 | 0.24 | 0.34 | 0.00 |
| 470 | 50.1 | 1.50 | 13.95 | 0.69 | 84.11 | 0.30 | 0.35 | 0.15 | 0.21 | 0.24 | 0.00 |
| 470 | 50.4 | 1.50 | 13.21 | 0.65 | 84.60 | 0.28 | 0.33 | 0.15 | 0.34 | 0.43 | 0.00 |
| 470 | 50.0 | 1.50 | 14.02 | 0.66 | 84.06 | 0.27 | 0.31 | 0.14 | 0.25 | 0.30 | 0.00 |
| 480 | 49.8 | 1.50 | 17.72 | 0.63 | 79.59 | 0.26 | 0.30 | 0.13 | 0.63 | 0.74 | 0.00 |
| 480 | 50.0 | 1.50 | 19.21 | 0.65 | 78.24 | 0.25 | 0.29 | 0.13 | 0.57 | 0.66 | 0.00 |
| 480 | 49.8 | 1.50 | 18.12 | 0.63 | 79.24 | 0.22 | 0.22 | 0.12 | 0.67 | 0.78 | 0.00 |
| 490 | 50.1 | 1.50 | 22.08 | 0.69 | 75.45 | 0.21 | 0.21 | 0.12 | 0.60 | 0.64 | 0.00 |
| 490 | 50.0 | 1.50 | 24.01 | 0.66 | 72.98 | 0.21 | 0.21 | 0.12 | 0.81 | 0.85 | 0.15 |
| 490 | 49.8 | 1.50 | 25.07 | 0.65 | 71.48 | 0.21 | 0.21 | 0.12 | 1.03 | 1.06 | 0.18 |
| 469 | 33.0 | 1.09 | 16.47 | 0.83 | 81.59 | 0.26 | 0.27 | 0.15 | 0.21 | 0.22 | 0.00 |
| 469 | 33.0 | 1.09 | 14.85 | 0.65 | 82.84 | 0.21 | 0.21 | 0.11 | 0.35 | 0.38 | 0.39 |
| 469 | 33.0 | 1.09 | 15.49 | 0.68 | 82.02 | 0.18 | 0.19 | 0.10 | 0.42 | 0.49 | 0.43 |
| 480 | 33.0 | 1.09 | 18.64 | 0.65 | 78.33 | 0.18 | 0.18 | 0.10 | 0.61 | 0.71 | 0.58 |
| 480 | 33.0 | 1.09 | 18.34 | 0.63 | 78.59 | 0.17 | 0.17 | 0.10 | 0.64 | 0.78 | 0.58 |
| 480 | 33.0 | 1.09 | 18.09 | 0.61 | 78.64 | 0.20 | 0.22 | 0.09 | 0.68 | 0.84 | 0.63 |
| 490 | 33.0 | 1.09 | 25.32 | 0.69 | 70.46 | 0.18 | 0.18 | 0.08 | 1.04 | 1.11 | 0.94 |
| 490 | 33.0 | 1.09 | 25.45 | 0.68 | 70.33 | 0.18 | 0.17 | 0.08 | 1.05 | 1.11 | 0.95 |
| 500 | 33.0 | 1.09 | 33.11 | 0.71 | 60.21 | 0.19 | 0.20 | 0.08 | 1.84 | 1.96 | 1.69 |
| 500 | 33.0 | 1.09 | 32.55 | 0.69 | 60.99 | 0.18 | 0.19 | 0.08 | 1.78 | 1.89 | 1.64 |

**Table 5: Detailed GC analysis of product produced at 490°C**

| **Compounds** | **GC-FID area%** |
|---|---|
| methane | 0.0183% |
| HFC-32 | 0.0006% |
| ethylene | 0.0612% |
| ethane | 0.0147% |
| 1132a | 0.0161 % |
| HCC-40 | 0.0035% |
| HCFC-22 | 0.0081% |
| HFO-1261ze-E | 0.0286% |
| 1252zc | 25.2162% |
| 263fb | 0.3565% |
| 1252 isomer | 0.3004% |
| 281fa | 0.2090% |
| HC-30 | 0.0306% |
| C4H3ClF4 | 0.0260% |
| 262fc | 70.3866% |
| E-1251zb | 0.2122% |
| Z-1251zb | 0.2319% |
| 272fb | 0.0931% |
| C4H6F4 | 0.0281% |
| C4H6F4 | 0.0422% |
| C6F12 | 0.0521% |
| 1252-dimer | 1.0604% |
| 1252-dimer | 1.1227% |
| others | 0.4806% |

### Example 4: Liquid Phase Dehydrohalogenation of 262fc to 1252zc

A mixture of 262fc (6.2 g, 63 mmol) and t-BuOK (7.1 g, 6.3 mmol) in dry DMF (30 ml) is stirred in a 100 mL flask at 0C with an overhead condenser, and the product of reaction is continuously taken off from reactor. Gas chromatography is used to monitor the reaction. After 1 hr, 1.3 g product CF2=CH-CH2 (conversion 30%, selectivity 86%) is collected in a dry ice trap.

### Example 5: Vapor Phase Hydrofluorination of 250fb to 1242zf without a catalyst

Fluorination of 250fb is carried out in an 10 inches long empty Inconel (0.5-inch OD) tube reactor. The reaction was run by feeding liquid 250fb into a heated chamber where it vaporized and mixed with HF and N₂. The reaction mixture was then allowed to pass through the reactor at the conditions listed in Table 6 below. Part of the reactor effluent was passed through a series of valves and analyzed by GCMS. Results are shown in Table 6 below.

**Table 6**

| **Time** | **T** | **250fb** | **N2** | **HF** | **HF/250fb** | **GC FID area%** | | | | |
|---|---|---|---|---|---|---|---|---|---|---|
| **hr** | **C** | **ml/hr** | **sccm** | **sccm** | **mol ratio** | **1243zf %** | **1242zf %** | **1241 %** | **1240za %** | **Others %** |
| 1.5 | 220 | 0.19 | 45 | 5.87 | 3.00 | 20.82 | 18.75 | 3.68 | 50.07 | 6.22 |
| 3 | 240 | 0.19 | 45 | 5.84 | 3.00 | 24.32 | 19.48 | 2.79 | 48.83 | 4.57 |
| 4.5 | 240 | 0.19 | 45 | 5.86 | 3.00 | 25.00 | 18.44 | 6.26 | 49.18 | 1.11 |
| 6 | 260 | 0.19 | 45 | 5.90 | 3.00 | 26.82 | 16.91 | 3.00 | 48.61 | 4.67 |
| 7.5 | 260 | 0.19 | 45 | 5.95 | 3.00 | 27.11 | 16.99 | 3.12 | 48.06 | 4.72 |
| 9 | 280 | 0.19 | 45 | 5.89 | 3.00 | 26.01 | 16.66 | 3.13 | 49.75 | 4.44 |
| 10.5 | 280 | 0.19 | 45 | 5.90 | 3.00 | 27.09 | 17.29 | 6.54 | 48.59 | 0.49 |
| 12 | 300 | 0.19 | 45 | 5.83 | 3.00 | 25.67 | 16.68 | 3.03 | 50.49 | 4.13 |
| 13.5 | 300 | 0.19 | 45 | 5.84 | 3.00 | 24.46 | 16.25 | 6.06 | 51.57 | 1.65 |
| 15 | 280 | 0.31 | 45 | 5.81 | 3.00 | 21.44 | 17.01 | 1.38 | 54.44 | 5.74 |

### Example 6: Vapor Phase Hydrofluorination of 250fb to 1242zf and 1243zf

Flurorination of 250fb over metal packings: Into an Inconel (0.5-inch OD) tube reactor was added 6 cc of Monel packing. The reaction was run by feeding liquid 250fb into a heated chamber where it vaporized and mixed with HF and N2. The reaction mixture was then allowed to pass through the reactor. The N2:HF:liquid feed ratio is 10.0:3.0:1. Part of the reactor effluent was passed through a series of valves and analyzed by GCMS. Results are shown in Table 7 below.

**Table 7**

| | **GC area%** | | | | | **Furnace** | **C T-Rxct** | **Ratio** |
|---|---|---|---|---|---|---|---|---|
| **Inj** | **1243zf** | **1242zf** | **1241zf** | **252fc** | **1240za** | **Temp °C** | **Temp (sec)** | **N2:HF:liquid** |
| 1 | 38.87% | 41.01% | 5.94% | 0.00% | 14.18% | 200 | 2.6 | 10.0:3.0:1 |
| 2 | 38.03% | 43.38% | 6.79% | 0.00% | 11.81% | 200 | 2.6 | 10.0:3.0:1 |
| 3 | 41.06% | 43.11% | 6.22% | 0.00% | 9.61% | 200 | 2.6 | 10.0:3.0:1 |
| 4 | 39.52% | 44.07% | 5.97% | 0.00% | 10.44% | 200 | 2.6 | 10.0:3.0:1 |
| 5 | 41.35% | 43.43% | 5.81% | 0.00% | 9.40% | 200 | 2.6 | 10.0:3.0:1 |
| 6 | 41.32% | 43.11% | 5.95% | 0.00% | 9.61% | 200 | 2.6 | 10.0:3.0:1 |
| 7 | 39.20% | 44.06% | 5.94% | 0.00% | 10.81% | 200 | 2.6 | 10.0:3.0:1 |
| 8 | 38.66% | 44.16% | 6.06% | 0.00% | 11.11% | 225 | 2.5 | 10.0:3.0:1 |
| 9 | 40.12% | 43.72% | 5.94% | 0.00% | 10.23% | 225 | 2.5 | 10.0:3.0:1 |
| 10 | 39.53% | 43.93% | 6.07% | 0.00% | 10.48% | 225 | 2.5 | 10.0:3.0:1 |
| 11 | 38.57% | 44.43% | 5.94% | 0.00% | 11.05% | 225 | 2.5 | 10.0:3.0:1 |
| 12 | 38.56% | 43.32% | 6.27% | 0.00% | 11.85% | 225 | 2.5 | 10.0:3.0:1 |
| 13 | 41.50% | 42.97% | 5.46% | 0.00% | 10.08% | 225 | 2.5 | 10.0:3.0:1 |
| 14 | 42.16% | 42.24% | 5.84% | 0.00% | 9.76% | 225 | 2.5 | 10.0:3.0:1 |
| 15 | 40.90% | 42.53% | 6.13% | 0.00% | 10.44% | 225 | 2.5 | 10.0:3.0:1 |
| 16 | 40.17% | 42.56% | 6.29% | 0.00% | 10.98% | 225 | 2.5 | 10.0:3.0:1 |
| 17 | 37.11% | 43.49% | 6.37% | 0.00% | 13.02% | 250 | 2.3 | 10.0:3.0:1 |
| 18 | 40.90% | 42.34% | 6.06% | 0.00% | 10.70% | 250 | 2.3 | 10.0:3.0:1 |
| 19 | 38.07% | 42.68% | 6.55% | 0.00% | 12.70% | 250 | 2.3 | 10.0:3.0:1 |
| 20 | 30.41% | 41.54% | 8.13% | 0.00% | 19.92% | 175 | 2.7 | 10.0:3.0:1 |
| 21 | 36.46% | 43.47% | 6.40% | 0.00% | 13.67% | 175 | 2.7 | 10.0:3.0:1 |
| 22 | 36.91% | 42.32% | 6.43% | 1.26% | 13.08% | 175 | 2.7 | 10.0:3.0:1 |
| 23 | 33.24% | 42.43% | 6.60% | 1.22% | 16.52% | 175 | 2.7 | 10.0:3.0:1 |
| 24 | 37.05% | 42.00% | 5.95% | 1.17% | 13.83% | 175 | 2.7 | 10.0:3.0:1 |
| 25 | 37.66% | 41.79% | 6.17% | 1.17% | 13.21% | 175 | 2.7 | 10.0:3.0:1 |
| 26 | 34.56% | 41.42% | 6.93% | 1.24% | 15.85% | 175 | 2.7 | 10.0:3.0:1 |
| 27 | 38.00% | 43.39% | 5.69% | 0.00% | 12.92% | 175 | 2.7 | 10.0:3.0:1 |
| 29 | 45.20% | 39.48% | 4.46% | 1.43% | 9.43% | 175 | 2.7 | 10.0:3.0:1 |

### Example 7: Hydrogenation of 1242zf to 262fc in vapor phase by 0.02% Pd/Al₂O₃

In a 12-inch long ½" OD Monel reactor, 6ml 0.02% Pd/Al2O3 was loaded. The catalyst was treated at 200°C by the flow of H₂ for 1 hour. Then, the reactor was heated to 50°C. HFO-1243zf, H₂ and N₂ feeds were controlled by a mass flow controller. The reaction test conditions are provided in Table 8. The reactor effluent was analyzed by on-line GC-MS-FID at the times indicated in Table 8. The results of analysis are provided in Tables 8 to 11, and show high conversion of HCFO-1242zf to HCFC-262fc and that selectivity of HCFC-262fc can reach 93%.

**Table 8**

| **Hours** | **C** | **psig** | **cc/hr** | **sccm** | **sccm** | **conversion** | **selectivity** |
|---|---|---|---|---|---|---|---|
| 1.0 | 50 | 25.0 | 0.60 | 10.32 | 8.05 | 100% | 91% |
| 2.0 | 50 | 25.0 | 0.60 | 10.32 | 8.05 | 100% | 91% |
| 3.0 | 50 | 25.0 | 0.60 | 10.32 | 8.05 | 100% | 91% |
| 4.0 | 60 | 25.0 | 0.60 | 10.32 | 8.05 | 100% | 90% |
| 5.0 | 60 | 25.0 | 0.60 | 10.32 | 8.05 | 100% | 91% |
| 6.0 | 70 | 25.0 | 0.60 | 10.32 | 8.10 | 100% | 90% |
| 7.0 | 70 | 25.0 | 0.60 | 10.32 | 8.10 | 100% | 89% |
| 8.0 | 80 | 25.0 | 0.60 | 10.32 | 8.10 | 100% | 88% |
| 9.0 | 80 | 25.0 | 0.60 | 10.32 | 8.10 | 100% | 89% |
| 10.0 | 40 | 25.0 | 0.60 | 10.34 | 8.09 | 100% | 93% |
| 11.0 | 40 | 25.0 | 0.60 | 10.34 | 8.09 | 94% | 91% |
| 12.5 | 40 | 25.0 | 0.60 | 10.34 | 8.09 | 92% | 90% |
| 13.5 | 40 | 25.0 | 0.60 | 10.34 | 8.09 | 97% | 93% |
| 14.5 | 40 | 25.0 | 0.60 | 10.33 | 8.04 | 100% | 92% |
| 15.5 | 40 | 25.0 | 0.60 | 10.33 | 8.04 | 94% | 93% |
| 16.5 | 45 | 25.0 | 1.00 | 10.33 | 8.04 | 85% | 89% |
| 17.5 | 45 | 25.0 | 0.60 | 10.33 | 8.04 | 95% | 93% |
| 18.5 | 45 | 25.0 | 0.60 | 10.33 | 8.04 | 91% | 92% |
| 19.5 | 45 | 25.0 | 0.60 | 10.33 | 8.04 | 89% | 92% |

**Table 9**

| **Detail GC analysis of product at 6^{th} hr of reaction time:** | |
|---|---|
| **Compounds** | **GC FID area%** |
| Propane | 0.1118% |
| Propylene | 0.5485% |
| 1261ze | 0.0219% |
| 1252zc | 4.5950% |
| 272fb | 0.6380% |
| 262fc | 90.9433% |
| E-1251zd | 0.4455% |
| Z-1251zd | 0.4488% |
| 262db | 1.3037% |
| 252dc | 0.6352% |
| 1250 | 0.0301% |
| 260 | 0.2284% |
| Others | 0.0497% |

**Table 10**

| **Detail GC analysis of product at 11^{th} hour of reaction time** | |
|---|---|
| **Compounds** | **FID area%** |
| propane | 0.0369% |
| propylene | 0.0833% |
| 1243zf | 2.5384% |
| 1252zf | 2.5912% |
| E-1261ze | 0.2409% |
| Z-1261ze | 0.0060% |
| 1242zf | 5.7972% |
| 262fc | 85.3762% |
| E-1251zd | 0.4880% |
| Z-1251zd | 0.6567% |
| 1242 isomer | 0.2742% |
| 262db | 1.0887% |
| 1232xf | 0.5607% |
| 252dc | 0.1249% |
| others | 0.1366% |

**Table 11**

| **Detail GC analysis of product at 19.5hr of reaction time** | |
|---|---|
| **Compounds** | **GC FID area%** |
| propane | 0.0295% |
| 1243zf | 2.2838% |
| 1252zc | 1.7754% |
| E-1261ze | 0.1237% |
| 1242zf | 11.0944% |
| 262fc | 81.6127% |
| E-1251zd | 0.3956% |
| Z-1251zd | 0.5558% |
| 1242 isomer | 0.5186% |
| 262db | 0.7944% |
| 1232 isomers | 0.5621% |
| 252 isomers | 0.0269% |
| 252dc | 0.0414% |
| 1250 | 0.0695% |
| 260 | 0.0741% |
| others | 0.0422% |

### Example 8: Liquid Phase Hydrogenation of 1242zf

0.25g of 0.5%Pd/C was loaded into a 10ml Hastelloy C shaker tube. Then, the shaker tube was chilled to -30°C and vacuumed. 5g of HCFO-1242zf was charged into the shaker tube, and heated back to a temperature of 65°C. At a temperature of 65°C, H₂ was added slowly to 300 psig pressure. The pressure drop quickly indicated the reaction happened at this condition. H₂ was further added until the pressure of reactor did not drop anymore. Both the vapor phase and liquid phase of reaction mixture were analyzed by GC-MS-FID, and the contents are identified in Tables 12 and 13.

**Table 12**

| **GC analysis of Vapor phase of reaction mixture** | |
|---|---|
| **Compounds** | **GC FID area%** |
| ethane | 0.0453% |
| 152a | 0.1359% |
| 1243zf | 0.4647% |
| 1252zc | 11.8858% |
| 1242zf | 79.7319% |
| 253db | 0.5287% |
| 262fc | 6.9624% |
| 1242 isomer | 0.0824% |
| 1232 isomer | 0.1630% |

**Table 13**

| **GC analysis of Liquid phase of reaction mixture** | |
|---|---|
| **Compounds** | **GC FID area%** |
| 1252zc | 0.1286% |
| 1242zf | 83.5625% |
| 253db | 1.6333% |
| 262fc | 12.5316% |
| 1242 isomer | 0.4363% |
| 1232 isomer | 1.7078% |

### Claim Embodiments

Embodiment 1. A method comprising contacting 1,3-dicloro-1,1-difluoropropane (HCFC-252fc) and hydrogen to form 1-chloro-1,1-difluoropropane (HCFC-262fc).
Embodiment 2. A method comprising converting 1-chloro-1,1-difluoropropane (HCFC-262fc) to 1,1-difluoropropene (HFO-1252zc).
Embodiment 3. A method comprising the steps of: (i) contacting 1,1,1,3-tetrachloropropane (HCC-250fb) and hydrogen fluoride in a liquid phase reaction to form 1,3-dicloro-1,1-difluoropropane (HCFC-252fc); and (ii) contacting the HCFC-252fc and hydrogen to form 1-chloro-1,1-difluoropropane (HCFC-262fc).
Embodiment 4. A method comprising the steps of: (i) contacting 1,3-dicloro-1,1-difluoropropane (HCFC-252fc) and hydrogen to form 1-chloro-1,1-difluoropropane (HCFC-262fc); and (ii) converting the HCFC-262fc to 1,1-difluoropropene (HFO-1252zc).
Embodiment 5. A method comprising the steps of: (i) contacting 1,1,1,3-tetrachloropropane (HCC-250fb) and hydrogen fluoride in a liquid phase reaction to form 1,3-dicloro-1,1-difluoropropane (HCFC-252fc); (ii) contacting the HCFC-252fc and hydrogen to form 1-chloro-1,1-difluoropropane (HCFC-262fc); and (iii) converting the HCFC-262fc to 1,1-difluoropropene (HFO-1252zc).
Embodiment 6. A method comprising contacting 1,1,1,3-tetrachloropropane (HCC-250fb) and hydrogen fluoride in a vapor phase reaction in the absence or presence of a catalyst in a reactor to form 3-chloro-3,3-difluoro-1-propene (HCFO-1242zf), wherein the catalyst, if present, is a metal alloy packing of the reactor, the metal alloy packing having catalytic activity.
Embodiment 7. A method comprising contacting 3-chloro-3,3-difluoro-1-propene (HCFO-1242zf) and hydrogen to form 1-chloro-1,1-difluoropropane (HCFC-262fc).
Embodiment 8. A method comprising the steps of: (i) contacting 1,1,1,3-tetrachloropropane (HCC-250fb) and hydrogen fluoride in a vapor phase reaction to form 3-chloro-3,3-difluoro-1-propene (HCFO-1242zf); and (ii) contacting the HCFO-1242zf and hydrogen to form 1-chloro-1,1-difluoropropane (HCFC-262fc).
Embodiment 9. A method comprising the steps of: (i) contacting 1,3-dicloro-1,1-difluoropropane (HCFC-252fc) with a caustic agent in an aqueous solvent to form 3-chloro-3,3-difluoro-1-propene (HCFO-1242zf); and (ii) contacting the HCFO-1242zf and hydrogen to form 1-chloro-1,1-difluoropropane (HCFC-262fc).
Embodiment 10. A method comprising the steps of: (i) contacting 3-chloro-3,3-difluoro-1-propene (HCFO-1242zf) and hydrogen to form 1-chloro-1,1-difluoropropane (HCFC-262fc); and (ii) converting the HCFC-262fc to 1,1-difluoropropene (HFO-1252zc).
Embodiment 11. A method comprising the steps of: (i) contacting 1,1,1,3-tetrachloropropane (HCC-250fb) and hydrogen fluoride in a vapor phase reaction to form 3-chloro-3,3-difluoro-1-propene (HCFO-1242zf); (ii) contacting the HCFO-1242zf and hydrogen to form 1-chloro-1,1-difluoropropane (HCFC-262fc); and (iii) converting the HCFC-262fc to 1,1-difluoropropene (HFO-1252zc).
Embodiment 12. A method comprising the steps of: (i) contacting 1,3-dicloro-1,1-difluoropropane (HCFC-252fc) with a caustic agent in an aqueous solvent to form 3-chloro-3,3-difluoro-1-propene (HCFO-1242zf); (ii) contacting the HCFO-1242zf and hydrogen to form 1-chloro-1,1-difluoropropane (HCFC-262fc); and (iii) converting the HCFC-262fc to 1,1-difluoropropene (HFO-1252zc).
Embodiment 13. A method comprising the steps of: (i) contacting 1,1,1,3-tetrachloropropane (HCC-250fb) and hydrogen fluoride in a liquid phase reaction to form 1,3-dicloro-1,1-difluoropropane (HCFC-252fc); (ii) contacting the HCFC-252fc with a caustic agent in an aqueous solvent to form 3-chloro-3,3-difluoro-1-propene (HCFO-1242zf); (iii) contacting the HCFO-1242zf and hydrogen to form 1-chloro-1,1-difluoropropane (HCFC-262fc); and (iv) converting the HCFC-262fc to 1,1-difluoropropene (HFO-1252zc).
Embodiment 14. A method comprising the steps of:
   (i) contacting 1,1,1,3-tetrachloropropane (HCC-250fb) and hydrogen fluoride in a vapor phase reaction to form 3-chloro-3,3-difluoro-1-propene (HCFO-1242zf); and
   (ii) contacting the HCFO-1242zf with zinc to form 1,1-difluoropropene (HFO-1252zc).
Embodiment 15. A method comprising: (i) contacting 1,3-dicloro-1,1-difluoropropane (HCFC-252fc) with a caustic agent in an aqueous solvent to form 3-chloro-3,3-difluoro-1-propene (HCFO-1242zf); and (ii) contacting the HCFO-1242zf with zinc to form 1,1-difluoropropene (HFO-1252zc).
Embodiment 16. A method comprising: (i) contacting 1,1,1,3-tetrachloropropane (HCC-250fb) and hydrogen fluoride in a liquid phase reaction to form 1,3-dicloro-1,1-difluoropropane (HCFC-252fc); (ii) contacting the HCFC-252fc with a caustic agent in an aqueous solvent to form 3-chloro-3,3-difluoro-1-propene (HCFO-1242zf); and (iii) contacting the HCFO-1242zf with zinc to form 1,1-difluoropropene (HFO-1252zc).
Embodiment 17. The method of any of Embodiments 1, 3, 4, and 5, wherein the contacting of the HCFC-252fc and hydrogen to form HCFC-262fc occurs in the vapor phase.
Embodiment 18. The method of any one of Embodiments 7 to 13, wherein the contacting of the HCFO-1242zf and hydrogen to form HCFC-262fc occurs in the vapor phase.
Embodiment 19. The method of any of Embodiments 1, 3, 4, and 5, wherein the contacting of the HCFC-252fc and hydrogen to form HCFC-262fc occurs in the liquid phase.
Embodiment 20. The method of any one of Embodiments 7 to 13, wherein the contacting of the HCFO-1242zf and hydrogen to form HCFC-262fc occurs in the liquid phase.
Embodiment 21. The method of either Embodiment 19 or Embodiment 20, wherein the contacting is in the presence of a catalyst selected from the group consisting of a group VIII metal, Pd, Pt, Ni, Cu, and combination of two or more thereof, preferably Pd.
Embodiment 22. The method of Embodiment 21, wherein the catalyst is unsupported or is supported.
Embodiment 23. The method of Embodiment 22, wherein the support is a carbon or aluminum oxide support.
Embodiment 24. The method of Embodiment 23, wherein the carbon support comprises one of carbon, acid-washed carbon, activated carbon, and three-dimensional matrix carbonaceous materials.
Embodiment 25. The method of any of Embodiments 21 to 24, wherein loading of the Pd catalyst is between 0.5% and 0.01%, or wherein loading of the Pd catalyst for a vapor phase reaction is between 0.1% and 0.01%.
Embodiment 26. The method of any of Embodiments 21 to 25, wherein the catalyst is Pd/Al₂O₃ or Pd/C.
Embodiment 27. The method of any of Embodiments 2, 4, 5, and 10 to 13 wherein the conversion of HCFC-262fc to HFO-1252zc comprises dehydrohalogenation of the HCFC-262fc.
Embodiment 28. The method of Embodiment 27, wherein the conversion of HCFC-262fc to HFO-1252zc by dehydrohalogenation of the HCFC-262fc occurs in the vapor phase or in the liquid phase using a strong base.
Embodiment 29. The method of any of Embodiments 6, 8, 11 and 14, wherein the contacting HCC-250fb with hydrogen fluoride to form HCFO-1242zf is in the absence of a catalyst.
Embodiment 30. The method of any of Embodiments 8, 11 and 14, wherein contacting HCC-250fb with hydrogen fluoride is in the presence of a catalyst selected from the group consisting of Lewis acid catalysts, transition metal halides, transition metal oxides (preferably partially fluorinated transition metal oxides), Group IVb metal halides, Group Vb metal halides, and metal alloy packing having catalytic activity.
Embodiment 31. The method of any of Embodiments 3 to 5, 8 to16, wherein separate reactors are used for each of the reaction steps.
Embodiment 32. The method of any of Embodiments 9, 12, 13, 15 and 16, wherein the caustic agent is selected from the group consisting of alkali metal oxides, hydroxides, amides, alkaline earth metal hydroxides, alkaline earth metal oxides or amides, alkali metal carbonates, alkali metal phosphate and alkali metal carboxylate.
Embodiment 33. The method of any of Embodiments 9, 12, 13, 15, 16 and 32, wherein the caustic agent is selected from the group consisting of NaOH, KOH, LiOH, CsOH, Ca(OH)₂, Zn(OH)₂, Na₂CO₃, K₂CO₃, K₃PO₄, Na₃PO₄, KF, and CsF.
Embodiment 34. The method of any of Embodiments 9, 12, 13, 15, 16, 32 and 33, wherein the HCFC-252fc is contacted with the caustic agent at a temperature in the range of about 20°C to about 150°C, preferably about 30°C to about 100°C.
Embodiment 35. The method of any of Embodiments 2, 4, 5, 10 to 13, wherein converting the HCFC-262fc to HFO-1252zc comprises contacting the HCFC-262fc with a strong base in a solvent.
Embodiment 36. The method of Embodiment 35, wherein the strong base is selected from the group consisting of hydroxides, alkoxides, metal amides, metal hydrides, metal dialkylamides and arylamines.
Embodiment 37. The method of any of Embodiments 35 to 36, wherein the strong base is selected from the group consisting of alkoxides including lithium, sodium and potassium salts of methyl, ethyl and t-butyl oxides; metal amides including sodium amide, potassium amide and lithium amide; metal hydrides including sodium hydride, potassium hydride and lithium hydride; and metal dialkylamides including lithium, sodium, and potassium salts of methyl, ethyl, n-propyl, iso-propyl, n-butyl, tert-butyl, trimethylsilyl and cyclohexyl substituted amides.
Embodiment 38. The method of any of Embodiments 6, 8, 11 and 14, wherein the HCC-250fb is contacted with hydrogen fluoride in the vapor phase in the presence of a catalyst to co-produce 3,3,3-trifluoropropene (HFO-1243zf, CF₃CH=CH₂) and the HCFO-1242zf.
Embodiment 39. The method of Embodiment 38, wherein the catalyst is a metal surface.
Embodiment 40. The method of Embodiment 39, wherein the metal surface is the surface of a metal alloy packing.
Embodiment 41. The method of any of Embodiments 38 to 40, wherein the catalyst is selected from the group consisting of Lewis acid catalysts, transition metal halides, transition metal oxides (preferably partially fluorinated transition metal oxides), Group IVb metal halides, Group Vb metal halides, chromium-based catalyst, cobalt-based catalyst, nickel-based catalyst, aluminum-based catalyst, iron-based catalyst, and combinations thereof.
Embodiment 42. A method comprising contacting 1,1,1,3-tetrachloropropane (HCC-250fb) with hydrogen fluoride in the vapor phase in the absence or presence of a catalyst to form 3,3,3-trifluoropropene (HFO-1243zf).
Embodiment 43. The method of Embodiment 42, wherein the catalyst is selected from the group consisting of Lewis acid catalysts, transition metal halides, transition metal oxides (preferably partially fluorinated transition metal oxides), Group IVb metal halides, Group Vb metal halides, and metal alloy packing.
Embodiment 44. The method of Embodiment 43, wherein the catalyst is selected from the group consisting of a chromium-based catalyst, a cobalt-based catalyst, a nickel-based catalyst, an aluminum-based catalyst, an iron-based catalyst, and combinations thereof.
Embodiment 45. A system comprising: a self-contained source of 1,1,1,3-tetrachloropropane (HCC-250fb); a pressurized source container of hydrogen and/or hydrogen fluoride; at least first, second and third serially arranged reactors, the first and second reactors respectively producing first and second intermediate product mixtures, and the third reactor producing a final product mixture comprising 1,1-difluoropropene (HFO-1252zc), wherein the first reactor contains a flowthrough bed of fluorination catalyst, the second reactor contains a flowthrough bed of hydrogenation catalyst, and the third reactor is configured for a vapor phase reaction to convert a hydrochlorofluorocarbon produced in the second reactor to HFO-1252zc.
Embodiment 46. The system of Embodiment 45, wherein the first reactor is configured for a liquid phase fluorination reaction to convert the HCC-250fb to 1,3-dicloro-1,1-difluoropropane (HCFC-252fc).
Embodiment 47. The system of any of Embodiments 45 to 46, wherein the second reactor is configured for a liquid phase or vapor phase hydrogenation reaction to convert the HCFC-252fc to 1-chloro-1,1-difluoropropane (HCFC-262fc).
Embodiment 48. The system of any of Embodiments 45 to 47, wherein the first reactor is configured for a vapor phase fluorination reaction to convert the HCC-250fb to 3-chloro-3,3-difluoro-1-propene (HCFO-1242zf).
Embodiment 49. The system of any of Embodiments 45 to 48, wherein the second reactor is configured for a liquid phase or vapor phase hydrogenation reaction to convert the HCFO-1242zf to the hydrochlorofluorocarbon HCFC-262fc.
Embodiment 50. The system of any of Embodiments 45 to 49, further comprising one or more separator systems.
Embodiment 51. The system of Embodiment 50, further comprising a first discharge line configured to supply the first intermediate product mixture from the first reactor to a first separator system, the first separator system being configured to recover a first intermediate product and convey the first intermediate product to the second reactor.
Embodiment 52. The system of Embodiment 51, further comprising a second discharge line configured to supply the second intermediate product mixture from the second reactor to a second separator system, the second separator system being configured to recover the hydrochlorofluorocarbon and convey the hydrochlorofluorocarbon to the third reactor.
Embodiment 53. The system of any of Embodiments 45 to 52, further comprising a vaporizer and a mixer.
Embodiment 54. The system of Embodiment 53, wherein the vaporizer and mixer are connected in series and arranged downstream of said source and upstream of said first reactor.
Embodiment 55. The system of any of Embodiments 45 to 54, wherein said pressurized source of hydrogen fluoride is connected to said mixer.
Embodiment 56. The system of any of Embodiments 45 to 55, wherein said pressurized source of hydrogen fluoride is connected to directly to said first reactor.
Embodiment 57. The system of Embodiment 51, wherein unreacted HCFC-252fc from the first separator system is recycled to the first reactor.
Embodiment 58. The system of Embodiment 52, wherein unreacted HCFC-262fc from the second separator system is recycled to the second reactor.
Embodiment 59. The system of Embodiment 51, wherein unreacted HCFO-1242zf from the first separator system is recycled to the first reactor.
Embodiment 60. A system comprising: a self-contained source of 1,1,1,3-tetrachloropropane (HCC-250fb); a pressurized source container of hydrogen fluoride; at least first and second serially arranged reactors, the first reactor producing a first intermediate product mixture comprising a hydrochlorofluoroolefin, and the second reactor producing a final product mixture comprising 1,1-difluoropropene (HFO-1252zc), wherein the first reactor contains a flowthrough bed of fluorination catalyst, and the second reactor is configured for a liquid phase reaction to convert the hydrochlorofluoroolefin produced in the first reactor to HFO-1252zc.
Embodiment 61. The system of Embodiment 60, wherein the first reactor is configured for a vapor phase fluorination reaction to convert the HCC-250fb to the hydrochlorofluoroolefin HCFO-1242zf, and wherein the second reactor is configured for liquid phase conversion of the hydrochlorofluoroolefin to HFO-1252zc.
Embodiment 62. The system of any of Embodiments 60 to 61, further comprising one or more separator systems.
Embodiment 63. The system of Embodiment 62, further comprising a first discharge line configured to supply the first intermediate product mixture from the first reactor to a first separator system, the first separator system being configured to recover the hydrochlorofluoroolefin and convey the hydrochlorofluoroolefin to the second reactor.
Embodiment 64. The system of any of Embodiments 60 to 63, further comprising a vaporizer and a mixer.
Embodiment 65. The system of Embodiment 64, wherein the vaporizer and mixer are connected in series and arranged downstream of said source and upstream of said first reactor.
Embodiment 66. The system of any of Embodiments 60 to 65, wherein said pressurized source of hydrogen fluoride is connected to said mixer.
Embodiment 67. The system of any of Embodiments 60 to 66, wherein said pressurized source of hydrogen fluoride is connected to directly to said first reactor.
Embodiment 68. The system of Embodiment 63, wherein unreacted HCFO-1242zf from the first separator system is recycled to the first reactor.
Embodiment 69. A process for producing HFO-1252zc comprising using the system of any of Embodiments 45 to 68.
Embodiment 70. A composition comprising, consisting essentially of, or consisting of 1-chloro-1,1-difluoropropane (HCFC-262fc) and one or more additional compounds selected from the group consisting of propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s).
Embodiment 71. The composition of Embodiment 70, the composition being formed by the method of Embodiment 18.
Embodiment 72. A composition comprising, consisting essentially of, or consisting of 1-chloro-1,1-difluoropropane (HCFC-262fc), 3-chloro-3,3-difluoro-1-propene (HCFO-1242zf) and one or more additional compounds selected from the group consisting of propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242 isomer(s), HCFO-1232 isomer(s) and HCFC-252 isomer(s).
Embodiment 73. The composition of Embodiment 72, the composition being formed by the method of Embodiment 18.
Embodiment 74. A composition comprising, consisting essentially of, or consisting of 1-chloro-1,1-difluoropropane (HCFC-262fc), 1,1-difluoropropane (HFC-272fb), and one or more additional compounds selected from the group consisting of propane, propylene, E-HFO-1261ze, Z-HFO-1261ze, HFO-1252zc, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s).
Embodiment 75. The composition of Embodiment 74, the composition being formed by the method of Embodiment 18.
Embodiment 76. A composition comprising, consisting essentially of, or consisting of 1-chloro-1,1-difluoropropane (HCFC-262fc) and one or more additional compounds selected from the group consisting of ethane, HFC-152a, HFC-253db, HCFO-1232 isomer(s), HCFO-1242 isomer(s), HCFO-1242zf, HFO-1243zf and HFO-1252zc.
Embodiment 77. The composition of Embodiment 76, the composition being formed by the method of Embodiment 20.
Embodiment 78. The composition of any of Embodiments 76 or 77, wherein a vapor portion of the composition comprises, consists essentially of, or consists of HCFC-262fc and one or more additional compounds selected from the group consisting of ethane, HFC-152a, HFC-253db, HCFO-1232 isomer(s), HCFO-1242 isomer(s), HCFO-1242zf, HFO-1243zf and HFO-1252zc, and wherein a liquid portion of the composition comprises, consists essentially of, or consists of HCFC-262fc and one or more additional compounds selected from the group consisting of HFC-253db, HCFO-1232 isomer(s), HCFO-1242 isomer(s), HCFO-1242zf and HFO-1252zc.
Embodiment 79. A composition comprising, consisting essentially of, or consisting of HCFO-1242zf and one or more additional compounds selected from the group consisting of HCC-250fb, HCFC-253fb, HCFC-252fc, HCFC-251fb, HCFC-1243zf, HCFC-1241zf and HCFC-1240za.
Embodiment 80. A composition comprising, consisting essentially of, or consisting of HCFC-1243zf and one or more additional compounds selected from the group consisting of HCC-250fb, HCFC-253fb, HCFC-252fc, HCFC-251fb, HCFO-1242zf, HCFC-1241zf and HCFC-1240za.
Embodiment 81. A composition comprising, consisting essentially of, or consisting of HFO-1252zc and one or more additional compounds selected from the group consisting of HCC-250fb, HCFC-252fc, HCFC-262fc and HCFO-1242zf.
Embodiment 82. A composition comprising, consisting essentially of, or consisting of HFO-1252zc and one or more additional compounds selected from the group consisting of HCFC-262fc, HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf, and HCFC-252fc.
Embodiment 83. The composition of Embodiment 82, the composition being formed by the method of any of Embodiments 2, 4, 5 and 10 to 13.
Embodiment 84. A composition comprising, consisting essentially of, or consisting of (i) HFO-1252zc; (ii) at least one of HCC-250fb, HCFC-252fc and HCFC-262fc; and (iii) one or more additional compounds selected from the group consisting of HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, and HCFO-1242zf.
Embodiment 85. The composition of Embodiment 84, the composition being formed by the method of any of Embodiments 2, 4, 5 and 10 to 13.
Embodiment 86. A composition comprising, consisting essentially of, or consisting of HFO-1252zc, HCFC-262fc, and one or more additional compounds selected from the group consisting of HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf and HCFC-252fc.
Embodiment 87. The composition of Embodiment 86, the composition being formed by the method of any of Embodiments 2, 4, 5 and 10 to 13.
Embodiment 88. A composition comprising, consisting essentially of, or consisting of HFO-1252zc, HCFC-262fc, HCFC-252fc and one or more additional compounds selected from HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc and HCFO-1242zf.
Embodiment 89. The composition of Embodiment 88, the composition being formed by the method of any of Embodiments 2, 4, 5 and 10 to 13.
Embodiment 90. A composition comprising, consisting essentially of, or consisting of HFO-1252zc and one or more additional compounds selected from the group consisting of HCFC-262fc, HCC-250fb, HFC-263fb, HCFO-1242zf, HFO-1252zf, HFO-1243zf and HCFC-252fc.
Embodiment 91. A composition comprising, consisting essentially of, or consisting of (i) HFO-1252zc; (ii) at least one of HCC-250fb and HCFO-1242zf; and (iii) one or more additional compounds selected from HCFC-262fc, HFC-263fb, HFO-1252zf, HFO-1243zf and HCFC-252fc.
Embodiment 92. The composition of Embodiment 90 or Embodiment 91, wherein the composition is formed by contacting 3-chloro-3,3-difluoro-1-propene (HCFO-1242zf) with zinc to form the composition comprising, consisting essentially of or consisting of HFO-1252zc and the one or more additional compounds.
Embodiment 93. The composition of Embodiment 92, wherein contacting HCFO-1242zf with zinc occurs in the liquid phase.
Embodiment 94. The composition of Embodiment 93, wherein the HCFO-1242zf is reacted with zinc in an organic solvent.
Embodiment 95. The composition of Embodiment 94, wherein the organic solvent is selected from the group consisting of alcohols, organic acids; polar aprotic solvents; carboxylic acid anhydrides; and mixtures thereof.
Embodiment 96. The composition of any of Embodiments 94 to 95, wherein the organic solvent is selected from the group consisting of acetic acid, N, N-dimethylformamide (DMF); acetic anhydride; and mixtures thereof.
Embodiment 97. The composition of any of Embodiments 94 to 96, wherein the HCFO-1242zf is reacted with zinc in an organic solvent in the presence of a catalyst.
Embodiment 98. The composition of Embodiment 97, wherein the catalyst is selected from the group consisting of a phase transfer catalyst, a metal salt and combinations thereof.
Embodiment 99. The composition of Embodiment 98, wherein the metal salt is a zinc salt.
Embodiment 100. The composition of Embodiment 99, wherein the zinc salt is selected from the group consisting of zinc acetate, zinc bromide, zinc chloride, zinc citrate, zinc sulfate and mixtures thereof.
Embodiment 101. The composition of Embodiment 98, wherein the phase transfer catalyst is selected from the group consisting of quaternary ammonium halides, quaternary phosphonium halides and cyclic polyether compounds.
Embodiment 102. The composition of any of Embodiments 97 to 101, wherein the catalyst has been activated by an acid wash.
Embodiment 103. The composition of any of Embodiments 90 to 91 and 97 to 102, wherein the zinc has been activated by an acid wash.
Embodiment 104. A composition comprising, consisting essentially of, or consisting of (i) HFO-1252zc and one or more additional compounds selected from the group consisting of HFC-263fb, HCFC-262fc, E-HFO-1251zb, Z-HFO-1251zb, HCFC-272fb, methane, HFC-32, ethylene, ethane, HFO-1132a, HCC-40, HCFC-22, ethylene oxide, E-HFO-1261ze, HFO-1252 isomers, HFC-281fa, HCC-30, C₄H₃ClF₄, C₄H₆F₄ (I), C₄H₆F₄ (II), C₆F₁₂, HFO-1252zc dimer (I) and HFO-1252zc dimer (II).
Embodiment 105. A composition comprising, consisting essentially of, or consisting of (i) HFO-1252zc and one or more additional compounds selected from the group consisting of HFC-263fb, HCFC-262fc, E-HFO-1251zb, Z-HFO-1251zb, HCFC-272fb, methane, HFC-32, ethylene, ethane, HFO-1132a, HCC-40, HCFC-22, E-HFO-1261ze, HFO-1252 isomers, HFC-281fa, HCC-30, C₄H₃ClF₄, C₄H₆F₄ (I), C₄H₆F₄ (II), C₆F₁₂, HFO-1252zc dimer (I) and HFO-1252zc dimer (II).
Embodiment 106. A composition comprising, consisting essentially of, or consisting of (i) HFO-1252zc and one or more additional compounds selected from the group consisting of HFC-263fb, HCFC-262fc, E-HFO-1251zb, Z-HFO-1251zb, HCFC-272fb, HFO-1252zc dimer (I) and HFO-1252zc dimer (II).
Embodiment 107. The composition according to any of Embodiments 104 to 106, wherein the one or more additional compounds comprise at least HFO-1252zc dimers.
Embodiment 108. The composition according to any of Embodiments 104 to 107, wherein the composition is formed by the method of any of Embodiments 2, 4, 5, 10 to 13, 27 and 28.
Embodiment 109. The composition of any of Embodiments 70 to 108, wherein the composition is free of or substantially free of Group A Fluorinated Substances.
Embodiment 110. The composition of any of Embodiments 70 to 109, wherein degradation products of the composition are free of or substantially free of Group A Fluorinated Substances.

Although certain aspects, embodiments and principals have been described above, it is understood that this description is made only way of example and not as limitation of the scope of the invention or appended claims. The foregoing various aspects, embodiments and principals can be used alone and in combinations with each other.

While the invention has been described with reference to one or more embodiments, it will be understood by those skilled in the art that various changes may be made, and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims. In addition, all numerical values identified in the detailed description shall be interpreted as though the precise and approximate values are both expressly identified.
The following numbered clauses, describing aspects of the invention, are part of the description.
Clause 1. A method comprising contacting 1,3-dicloro-1,1-difluoropropane (HCFC-252fc) and hydrogen to form 1-chloro-1,1-difluoropropane (HCFC-262fc).
Clause 2. A method comprising converting 1-chloro-1,1-difluoropropane (HCFC-262fc) to 1,1-difluoropropene (HFO-1252zc).
Clause 3. A method comprising the steps of:
   (i) contacting 1,1,1,3-tetrachloropropane (HCC-250fb) and hydrogen fluoride in a liquid phase reaction to form 1,3-dicloro-1,1-difluoropropane (HCFC-252fc); and
   (ii) contacting the HCFC-252fc and hydrogen to form 1-chloro-1,1-difluoropropane (HCFC-262fc).
Clause 4. A method comprising the steps of:
   (i) contacting 1,3-dicloro-1,1-difluoropropane (HCFC-252fc) and hydrogen to form 1-chloro-1,1-difluoropropane (HCFC-262fc); and
   (ii) converting the HCFC-262fc to 1,1-difluoropropene (HFO-1252zc).
Clause 5. A method comprising the steps of:
   (i) contacting 1,1,1,3-tetrachloropropane (HCC-250fb) and hydrogen fluoride in a liquid phase reaction to form 1,3-dicloro-1,1-difluoropropane (HCFC-252fc);
   (ii) contacting the HCFC-252fc and hydrogen to form 1-chloro-1,1-difluoropropane (HCFC-262fc); and
   (iii) converting the HCFC-262fc to 1,1-difluoropropene (HFO-1252zc).
Clause 6. A method comprising contacting 1,1,1,3-tetrachloropropane (HCC-250fb) and hydrogen fluoride in a vapor phase reaction in the absence or presence of a catalyst in a reactor to form 3-chloro-3,3-difluoro-1-propene (HCFO-1242zf), wherein the catalyst, if present, is a metal alloy packing of the reactor, the metal alloy packing having catalytic activity.
Clause 7. A method comprising contacting 3-chloro-3,3-difluoro-1-propene (HCFO-1242zf) and hydrogen to form 1-chloro-1,1-difluoropropane (HCFC-262fc).
Clause 8. A method comprising the steps of:
   (i) contacting 1,1,1,3-tetrachloropropane (HCC-250fb) and hydrogen fluoride in a vapor phase reaction to form 3-chloro-3,3-difluoro-1-propene (HCFO-1242zf); and
   (ii) contacting the HCFO-1242zf and hydrogen to form 1-chloro-1,1-difluoropropane (HCFC-262fc).
Clause 9. A method comprising the steps of:
   (i) contacting 1,3-dicloro-1,1-difluoropropane (HCFC-252fc) with a caustic agent in an aqueous solvent to form 3-chloro-3,3-difluoro-1-propene (HCFO-1242zf); and
   (ii) contacting the HCFO-1242zf and hydrogen to form 1-chloro-1,1-difluoropropane (HCFC-262fc).
Clause 10. A method comprising the steps of:
   (i) contacting 3-chloro-3,3-difluoro-1-propene (HCFO-1242zf) and hydrogen to form 1-chloro-1,1-difluoropropane (HCFC-262fc); and
   (ii) converting the HCFC-262fc to 1,1-difluoropropene (HFO-1252zc).
Clause 11. A method comprising the steps of:
   (i) contacting 1,1,1,3-tetrachloropropane (HCC-250fb) and hydrogen fluoride in a vapor phase reaction to form 3-chloro-3,3-difluoro-1-propene (HCFO-1242zf);
   (ii) contacting the HCFO-1242zf and hydrogen to form 1-chloro-1,1-difluoropropane (HCFC-262fc); and
   (iii) converting the HCFC-262fc to 1,1-difluoropropene (HFO-1252zc).
Clause 12. A method comprising the steps of:
   (i) contacting 1,3-dicloro-1,1-difluoropropane (HCFC-252fc) with a caustic agent in an aqueous solvent to form 3-chloro-3,3-difluoro-1-propene (HCFO-1242zf);
   (ii) contacting the HCFO-1242zf and hydrogen to form 1-chloro-1,1-difluoropropane (HCFC-262fc); and
   (iii) converting the HCFC-262fc to 1,1-difluoropropene (HFO-1252zc).
Clause 13. A method comprising the steps of:
   (i) contacting 1,1,1,3-tetrachloropropane (HCC-250fb) and hydrogen fluoride in a liquid phase reaction to form 1,3-dicloro-1,1-difluoropropane (HCFC-252fc);
   (ii) contacting the HCFC-252fc with a caustic agent in an aqueous solvent to form 3-chloro-3,3-difluoro-1-propene (HCFO-1242zf);
   (iii) contacting the HCFO-1242zf and hydrogen to form 1-chloro-1,1-difluoropropane (HCFC-262fc); and
   (iv) converting the HCFC-262fc to 1,1-difluoropropene (HFO-1252zc).
Clause 14. A method comprising the steps of:
   (i) contacting 1,1,1,3-tetrachloropropane (HCC-250fb) and hydrogen fluoride in a vapor phase reaction to form 3-chloro-3,3-difluoro-1-propene (HCFO-1242zf); and
   (ii) contacting the HCFO-1242zf with zinc to form 1,1-difluoropropene (HFO-1252zc).
Clause 15. A method comprising:
   (i) contacting 1,3-dicloro-1,1-difluoropropane (HCFC-252fc) with a caustic agent in an aqueous solvent to form 3-chloro-3,3-difluoro-1-propene (HCFO-1242zf); and
   (ii) contacting the HCFO-1242zf with zinc to form 1,1-difluoropropene (HFO-1252zc).
Clause 16. A method comprising:
   (i) contacting 1,1,1,3-tetrachloropropane (HCC-250fb) and hydrogen fluoride in a liquid phase reaction to form 1,3-dicloro-1,1-difluoropropane (HCFC-252fc);
   (ii) contacting the HCFC-252fc with a caustic agent in an aqueous solvent to form 3-chloro-3,3-difluoro-1-propene (HCFO-1242zf); and
   (iii) contacting the HCFO-1242zf with zinc to form 1,1-difluoropropene (HFO-1252zc).
Clause 17. The method of any of clauses 1, 3, 4, and 5, wherein the contacting of the HCFC-252fc and hydrogen to form HCFC-262fc occurs in the vapor phase.
Clause 18. The method of any one of clauses 7 to 13, wherein the contacting of the HCFO-1242zf and hydrogen to form HCFC-262fc occurs in the vapor phase.
Clause 19. The method of any of clauses 1, 3, 4, and 5, wherein the contacting of the HCFC-252fc and hydrogen to form HCFC-262fc occurs in the liquid phase.
Clause 20. The method of any one of clauses 7 to 13, wherein the contacting of the HCFO-1242zf and hydrogen to form HCFC-262fc occurs in the liquid phase.
Clause 21. The method of either clause 19 or clause 20, wherein the contacting is in the presence of a catalyst selected from the group consisting of a group VIII metal, Pd, Pt, Ni, Cu, and combination of two or more thereof, preferably Pd.
Clause 22. The method of clause 21, wherein the catalyst is unsupported or is supported.
Clause 23. The method of clause 22, wherein the support is a carbon or aluminum oxide support.
Clause 24. The method of clause 23, wherein the carbon support comprises one of carbon, acid-washed carbon, activated carbon, and three-dimensional matrix carbonaceous materials.
Clause 25. The method of any of clauses 21 to 24, wherein loading of the Pd catalyst is between 0.5% and 0.01%, or wherein loading of the Pd catalyst for a vapor phase reaction is between 0.1% and 0.01%.
Clause 26. The method of any of clauses 21 to 25, wherein the catalyst is Pd/Al₂O₃ or Pd/C.
Clause 27. The method of any of clauses 2, 4, 5, and 10 to 13 wherein the conversion of HCFC-262fc to HFO-1252zc comprises dehydrohalogenation of the HCFC-262fc.
Clause 28. The method of clause 27, wherein the conversion of HCFC-262fc to HFO-1252zc by dehydrohalogenation of the HCFC-262fc occurs in the vapor phase or in the liquid phase using a strong base.
Clause 29. The method of any of clauses 6, 8, 11 and 14, wherein the contacting HCC-250fb with hydrogen fluoride to form HCFO-1242zf is in the absence of a catalyst.
Clause 30. The method of any of clauses 8, 11 and 14, wherein contacting HCC-250fb with hydrogen fluoride is in the presence of a catalyst selected from the group consisting of Lewis acid catalysts, transition metal halides, transition metal oxides (preferably partially fluorinated transition metal oxides), Group IVb metal halides, Group Vb metal halides, and metal alloy packing having catalytic activity.
Clause 31. The method of any of clauses 3 to 5, 8 to16, wherein separate reactors are used for each of the reaction steps.
Clause 32. The method of any of clauses 9, 12, 13, 15 and 16, wherein the caustic agent is selected from the group consisting of alkali metal oxides, hydroxides, amides, alkaline earth metal hydroxides, alkaline earth metal oxides or amides, alkali metal carbonates, alkali metal phosphate and alkali metal carboxylate.
Clause 33. The method of any of clauses 9, 12, 13, 15, 16 and 32, wherein the caustic agent is selected from the group consisting of NaOH, KOH, LiOH, CsOH, Ca(OH)₂, Zn(OH)₂, Na₂CO₃, K₂CO₃, K₃PO₄, Na₃PO₄, KF, and CsF.
Clause 34. The method of any of clauses 9, 12, 13, 15, 16, 32 and 33, wherein the HCFC-252fc is contacted with the caustic agent at a temperature in the range of about 20°C to about 150°C, preferably about 30°C to about 100°C.
Clause 35. The method of any of clauses 2, 4, 5, 10 to 13, wherein converting the HCFC-262fc to HFO-1252zc comprises contacting the HCFC-262fc with a strong base in a solvent.
Clause 36. The method of clause 35, wherein the strong base is selected from the group consisting of hydroxides, alkoxides, metal amides, metal hydrides, metal dialkylamides and arylamines.
Clause 37. The method of any of clauses 35 to 36, wherein the strong base is selected from the group consisting of alkoxides including lithium, sodium and potassium salts of methyl, ethyl and t-butyl oxides; metal amides including sodium amide, potassium amide and lithium amide; metal hydrides including sodium hydride, potassium hydride and lithium hydride; and metal dialkylamides including lithium, sodium, and potassium salts of methyl, ethyl, n-propyl, iso-propyl, n-butyl, tert-butyl, trimethylsilyl and cyclohexyl substituted amides.
Clause 38. The method of any of clauses 6, 8, 11 and 14, wherein the HCC-250fb is contacted with hydrogen fluoride in the vapor phase in the presence of a catalyst to co-produce 3,3,3-trifluoropropene (HFO-1243zf, CF₃CH=CH₂) and the HCFO-1242zf.
Clause 39. The method of clause 38, wherein the catalyst is a metal surface.
Clause 40. The method of clause 39, wherein the metal surface is the surface of a metal alloy packing.
Clause 41. The method of any of clauses 38 to 40, wherein the catalyst is selected from the group consisting of Lewis acid catalysts, transition metal halides, transition metal oxides (preferably partially fluorinated transition metal oxides), Group IVb metal halides, Group Vb metal halides, chromium-based catalyst, cobalt-based catalyst, nickel-based catalyst, aluminum-based catalyst, iron-based catalyst, and combinations thereof.
Clause 42. A method comprising contacting 1,1,1,3-tetrachloropropane (HCC-250fb) with hydrogen fluoride in the vapor phase in the absence or presence of a catalyst to form 3,3,3-trifluoropropene (HFO-1243zf).
Clause 43. The method of clause 42, wherein the catalyst is selected from the group consisting of Lewis acid catalysts, transition metal halides, transition metal oxides (preferably partially fluorinated transition metal oxides), Group IVb metal halides, Group Vb metal halides, and metal alloy packing.
Clause 44. The method of clause 43, wherein the catalyst is selected from the group consisting of a chromium-based catalyst, a cobalt-based catalyst, a nickel-based catalyst, an aluminum-based catalyst, an iron-based catalyst, and combinations thereof.
Clause 45. A system comprising:
   a self-contained source of 1,1,1,3-tetrachloropropane (HCC-250fb);
   a pressurized source container of hydrogen and/or hydrogen fluoride;
   at least first, second and third serially arranged reactors, the first and second reactors respectively producing first and second intermediate product mixtures, and the third reactor producing a final product mixture comprising 1,1-difluoropropene (HFO-1252zc),
   wherein the first reactor contains a flowthrough bed of fluorination catalyst, the second reactor contains a flowthrough bed of hydrogenation catalyst, and the third reactor is configured for a vapor phase reaction to convert a hydrochlorofluorocarbon produced in the second reactor to HFO-1252zc.
Clause 46. The system of clause 45, wherein the first reactor is configured for a liquid phase fluorination reaction to convert the HCC-250fb to 1,3-dicloro-1,1-difluoropropane (HCFC-252fc).
Clause 47. The system of any of clauses 45 to 46, wherein the second reactor is configured for a liquid phase or vapor phase hydrogenation reaction to convert the HCFC-252fc to 1-chloro-1,1-difluoropropane (HCFC-262fc).
Clause 48. The system of any of clauses 45 to 47, wherein the first reactor is configured for a vapor phase fluorination reaction to convert the HCC-250fb to 3-chloro-3,3-difluoro-1-propene (HCFO-1242zf).
Clause 49. The system of any of clauses 45 to 48, wherein the second reactor is configured for a liquid phase or vapor phase hydrogenation reaction to convert the HCFO-1242zf to the hydrochlorofluorocarbon HCFC-262fc.
Clause 50. The system of any of clauses 45 to 49, further comprising one or more separator systems.
Clause 51. The system of clause 50, further comprising a first discharge line configured to supply the first intermediate product mixture from the first reactor to a first separator system, the first separator system being configured to recover a first intermediate product and convey the first intermediate product to the second reactor.
Clause 52. The system of clause 51, further comprising a second discharge line configured to supply the second intermediate product mixture from the second reactor to a second separator system, the second separator system being configured to recover the hydrochlorofluorocarbon and convey the hydrochlorofluorocarbon to the third reactor.
Clause 53. The system of any of clauses 45 to 52, further comprising a vaporizer and a mixer.
Clause 54. The system of clause 53, wherein the vaporizer and mixer are connected in series and arranged downstream of said source and upstream of said first reactor.
Clause 55. The system of any of clauses 45 to 54, wherein said pressurized source of hydrogen fluoride is connected to said mixer.
Clause 56. The system of any of clauses 45 to 55, wherein said pressurized source of hydrogen fluoride is connected to directly to said first reactor.
Clause 57. The system of clause 51, wherein unreacted HCFC-252fc from the first separator system is recycled to the first reactor.
Clause 58. The system of clause 52, wherein unreacted HCFC-262fc from the second separator system is recycled to the second reactor.
Clause 59. The system of clause 51, wherein unreacted HCFO-1242zf from the first separator system is recycled to the first reactor.
Clause 60. A system comprising:
   a self-contained source of 1,1,1,3-tetrachloropropane (HCC-250fb);
   a pressurized source container of hydrogen fluoride;
   at least first and second serially arranged reactors, the first reactor producing a first intermediate product mixture comprising a hydrochlorofluoroolefin, and the second reactor producing a final product mixture comprising 1,1-difluoropropene (HFO-1252zc),
   wherein the first reactor contains a flowthrough bed of fluorination catalyst, and the second reactor is configured for a liquid phase reaction to convert the hydrochlorofluoroolefin produced in the first reactor to HFO-1252zc.
Clause 61. The system of clause 60, wherein the first reactor is configured for a vapor phase fluorination reaction to convert the HCC-250fb to the hydrochlorofluoroolefin HCFO-1242zf, and wherein the second reactor is configured for liquid phase conversion of the hydrochlorofluoroolefin to HFO-1252zc.
Clause 62. The system of any of clauses 60 to 61, further comprising one or more separator systems.
Clause 63. The system of clause 62, further comprising a first discharge line configured to supply the first intermediate product mixture from the first reactor to a first separator system, the first separator system being configured to recover the hydrochlorofluoroolefin and convey the hydrochlorofluoroolefin to the second reactor.
Clause 64. The system of any of clauses 60 to 63, further comprising a vaporizer and a mixer.
Clause 65. The system of clause 64, wherein the vaporizer and mixer are connected in series and arranged downstream of said source and upstream of said first reactor.
Clause 66. The system of any of clauses 60 to 65, wherein said pressurized source of hydrogen fluoride is connected to said mixer.
Clause 67. The system of any of clauses 60 to 66, wherein said pressurized source of hydrogen fluoride is connected to directly to said first reactor.
Clause 68. The system of clause 63, wherein unreacted HCFO-1242zf from the first separator system is recycled to the first reactor.
Clause 69. A process for producing HFO-1252zc comprising using the system of any of clauses 45 to 68.
Clause 70. A composition comprising, consisting essentially of, or consisting of 1-chloro-1,1-difluoropropane (HCFC-262fc) and one or more additional compounds selected from the group consisting of propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s).
Clause 71. The composition of clause 70, the composition being formed by the method of clause 18.
Clause 72. A composition comprising, consisting essentially of, or consisting of 1-chloro-1,1-difluoropropane (HCFC-262fc), 3-chloro-3,3-difluoro-1-propene (HCFO-1242zf) and one or more additional compounds selected from the group consisting of propane, propylene, E-HFO-1261ze, Z-HFO-1261ze, HFO-1252zc, HFC-272fb, E-HCFO-1251zd, Z-HCFO-1251zd, HCFC-262db, HCFC-252dc, HCO-1250, HCC-260, HFO-1243zf, HCFO-1242 isomer(s), HCFO-1232 isomer(s) and HCFC-252 isomer(s).
Clause 73. The composition of clause 72, the composition being formed by the method of clause 18.
Clause 74. A composition comprising, consisting essentially of, or consisting of 1-chloro-1,1-difluoropropane (HCFC-262fc), 1,1-difluoropropane (HFC-272fb), and one or more additional compounds selected from the group consisting of propane, propylene, E-HFO-1261ze, Z-HFO-1261ze, HFO-1252zc, E-HCFO-1251zd, Z-HCFO-1251zd, HCFC-262db, HCFC-252dc, HCO-1250, HCC-260, HFO-1243zf, HCFO-1242zf, HCFO-1242 isomer(s), HCFO-1232 isomer(s) and HCFC-252 isomer(s).
Clause 75. The composition of clause 74, the composition being formed by the method of clause 18.
Clause 76. A composition comprising, consisting essentially of, or consisting of 1-chloro-1,1-difluoropropane (HCFC-262fc) and one or more additional compounds selected from the group consisting of ethane, HFC-152a, HFC-253db, HCFO-1232 isomer(s), HCFO-1242 isomer(s), HCFO-1242zf, HFO-1243zf and HFO-1252zc.
Clause 77. The composition of clause 76, the composition being formed by the method of clause 20.
Clause 78. The composition of any of clauses 76 or 77, wherein a vapor portion of the composition comprises, consists essentially of, or consists of HCFC-262fc and one or more additional compounds selected from the group consisting of ethane, HFC-152a, HFC-253db, HCFO-1232 isomer(s), HCFO-1242 isomer(s), HCFO-1242zf, HFO-1243zf and HFO-1252zc, and wherein a liquid portion of the composition comprises, consists essentially of, or consists of HCFC-262fc and one or more additional compounds selected from the group consisting of HFC-253db, HCFO-1232 isomer(s), HCFO-1242 isomer(s), HCFO-1242zf and HFO-1252zc.
Clause 79. A composition comprising, consisting essentially of, or consisting of HCFO-1242zf and one or more additional compounds selected from the group consisting of HCC-250fb, HCFC-253fb, HCFC-252fc, HCFC-251fb, HCFC-1243zf, HCFC-1241zf and HCFC-1240za.
Clause 80. A composition comprising, consisting essentially of, or consisting of HCFC-1243zf and one or more additional compounds selected from the group consisting of HCC-250fb, HCFC-253fb, HCFC-252fc, HCFC-251fb, HCFO-1242zf, HCFC-1241zf and HCFC-1240za.
Clause 81. A composition comprising, consisting essentially of, or consisting of HFO-1252zc and one or more additional compounds selected from the group consisting of HCC-250fb, HCFC-252fc, HCFC-262fc and HCFO-1242zf.
Clause 82. A composition comprising, consisting essentially of, or consisting of HFO-1252zc and one or more additional compounds selected from the group consisting of HCFC-262fc, HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf, and HCFC-252fc.
Clause 83. The composition of clause 82, the composition being formed by the method of any of clauses 2, 4, 5 and 10 to 13.
Clause 84. A composition comprising, consisting essentially of, or consisting of (i) HFO-1252zc; (ii) at least one of HCC-250fb, HCFC-252fc and HCFC-262fc; and (iii) one or more additional compounds selected from the group consisting of HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, and HCFO-1242zf.
Clause 85. The composition of clause 84, the composition being formed by the method of any of clauses 2, 4, 5 and 10 to 13.
Clause 86. A composition comprising, consisting essentially of, or consisting of HFO-1252zc, HCFC-262fc, and one or more additional compounds selected from the group consisting of HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc, HCFO-1242zf and HCFC-252fc.
Clause 87. The composition of clause 86, the composition being formed by the method of any of clauses 2, 4, 5 and 10 to 13.
Clause 88. A composition comprising, consisting essentially of, or consisting of HFO-1252zc, HCFC-262fc, HCFC-252fc and one or more additional compounds selected from HCC-250fb, HFC-263fb, HFO-1243zf, other 1252 isomers, ethane, ethylene, HFC-23, HCFC-22, HFO-1132a, propane, HFC-143a, HCFC-142b, HFO-1234yf, HFO-1234ze, HFO-1225zc and HCFO-1242zf.
Clause 89. The composition of clause 88, the composition being formed by the method of any of clauses 2, 4, 5 and 10 to 13.
Clause 90. A composition comprising, consisting essentially of, or consisting of HFO-1252zc and one or more additional compounds selected from the group consisting of HCFC-262fc, HCC-250fb, HFC-263fb, HCFO-1242zf, HFO-1252zf, HFO-1243zf and HCFC-252fc.
Clause 91. A composition comprising, consisting essentially of, or consisting of (i) HFO-1252zc; (ii) at least one of HCC-250fb and HCFO-1242zf; and (iii) one or more additional compounds selected from HCFC-262fc, HFC-263fb, HFO-1252zf, HFO-1243zf and HCFC-252fc.
Clause 92. The composition of clause 90 or clause 91, wherein the composition is formed by contacting 3-chloro-3,3-difluoro-1-propene (HCFO-1242zf) with zinc to form the composition comprising, consisting essentially of or consisting of HFO-1252zc and the one or more additional compounds.
Clause 93. The composition of clause 92, wherein contacting HCFO-1242zf with zinc occurs in the liquid phase.
Clause 94. The composition of clause 93, wherein the HCFO-1242zf is reacted with zinc in an organic solvent.
Clause 95. The composition of clause 94, wherein the organic solvent is selected from the group consisting of alcohols, organic acids; polar aprotic solvents; carboxylic acid anhydrides; and mixtures thereof.
Clause 96. The composition of any of clauses 94 to 95, wherein the organic solvent is selected from the group consisting of acetic acid, N, N-dimethylformamide (DMF); acetic anhydride; and mixtures thereof.
Clause 97. The composition of any of clauses 94 to 96, wherein the HCFO-1242zf is reacted with zinc in an organic solvent in the presence of a catalyst.
Clause 98. The composition of clause 97, wherein the catalyst is selected from the group consisting of a phase transfer catalyst, a metal salt and combinations thereof.
Clause 99. The composition of clause 98, wherein the metal salt is a zinc salt.
Clause 100. The composition of clause 99, wherein the zinc salt is selected from the group consisting of zinc acetate, zinc bromide, zinc chloride, zinc citrate, zinc sulfate and mixtures thereof.
Clause 101. The composition of clause 98, wherein the phase transfer catalyst is selected from the group consisting of quaternary ammonium halides, quaternary phosphonium halides and cyclic polyether compounds.
Clause 102. The composition of any of clauses 97 to 101, wherein the catalyst has been activated by an acid wash.
Clause 103. The composition of any of clauses 90 to 91 and 97 to 102, wherein the zinc has been activated by an acid wash.
Clause 104. A composition comprising, consisting essentially of, or consisting of (i) HFO-1252zc and one or more additional compounds selected from the group consisting of HFC-263fb, HCFC-262fc, E-HFO-1251zb, Z-HFO-1251zb, HCFC-272fb, methane, HFC-32, ethylene, ethane, HFO-1132a, HCC-40, HCFC-22, ethylene oxide, E-HFO-1261ze, HFO-1252 isomers, HFC-281fa, HCC-30, C₄H₃ClF₄, C₄H₆F₄ (I), C₄H₆F₄ (II), C₆F₁₂, HFO-1252zc dimer (I) and HFO-1252zc dimer (II).
Clause 105. A composition comprising, consisting essentially of, or consisting of (i) HFO-1252zc and one or more additional compounds selected from the group consisting of HFC-263fb, HCFC-262fc, E-HFO-1251zb, Z-HFO-1251zb, HCFC-272fb, methane, HFC-32, ethylene, ethane, HFO-1132a, HCC-40, HCFC-22, E-HFO-1261ze, HFO-1252 isomers, HFC-281fa, HCC-30, C₄H₃ClF₄, C₄H₆F₄ (I), C₄H₆F₄ (II), C₆F₁₂, HFO-1252zc dimer (I) and HFO-1252zc dimer (II).
Clause 106. A composition comprising, consisting essentially of, or consisting of (i) HFO-1252zc and one or more additional compounds selected from the group consisting of HFC-263fb, HCFC-262fc, E-HFO-1251zb, Z-HFO-1251zb, HCFC-272fb, HFO-1252zc dimer (I) and HFO-1252zc dimer (II).
Clause 107. The composition according to any of clauses 104 to 106, wherein the one or more additional compounds comprise at least HFO-1252zc dimers.
Clause 108. The composition according to any of clauses 104 to 107, wherein the composition is formed by the method of any of clauses 2, 4, 5, 10 to 13, 27 and 28.
Clause 109. The composition of any of clauses 70 to 108, wherein the composition is free of or substantially free of Group A Fluorinated Substances.
Clause 110. The composition of any of clauses 70 to 109, wherein degradation products of the composition are free of or substantially free of Group A Fluorinated Substances.

## Claims

1. A method comprising contacting 1,3-dichloro-1,1-difluoropropane (HCFC-252fc) and hydrogen to form 1-chloro-1,1-difluoropropane (HCFC-262fc).

2. The method of claim 1, wherein the contacting of the HCFC-252fc and hydrogen to form HCFC-262fc occurs in the vapor phase.

3. The method of claim 1, wherein the contacting of the HCFC-252fc and hydrogen to form HCFC-262fc occurs in the liquid phase.

4. The method of claim 3, wherein the contacting is in the presence of a catalyst selected from the group consisting of a group VIII metal, Pd, Pt, Ni, Cu, and combination of two or more thereof, preferably Pd;
optionally wherein the catalyst is unsupported or is supported.

5. The method of claim 4, wherein the catalyst is supported and wherein the support is a carbon or aluminum oxide support;
optionally wherein the carbon support comprises one of carbon, acid-washed carbon, activated carbon, and three-dimensional matrix carbonaceous materials.

6. The method of claim 4 or 5, wherein loading of the Pd catalyst is between 0.5% and 0.01%, or wherein loading of the Pd catalyst for a vapor phase reaction is between 0.1% and 0.01%; and/or
wherein the catalyst is Pd/Al₂O₃ or Pd/C.

7. The method of any one of claims 1 to 6, further comprising contacting 1,1,1,3-tetrachloropropane (HCC-250fb) and hydrogen fluoride in a liquid phase reaction to form the 1,3-dichloro-1,1-difluoropropane (HCFC-252fc).

8. The method of any one of claims 1 to 7, further comprising converting the HCFC-262fc to 1,1-difluoropropene (HFO-1252zc).

9. The method of claim 8, wherein the conversion of HCFC-262fc to HFO-1252zc comprises dehydrohalogenation of the HCFC-262fc.

10. The method of claim 9, wherein the conversion of HCFC-262fc to HFO-1252zc by dehydrohalogenation of the HCFC-262fc occurs in the vapor phase or in the liquid phase using a strong base.

11. The method of claim 7 or 8, wherein separate reactors are used for each of the reaction steps.

12. The method of claim 8, wherein converting the HCFC-262fc to HFO-1252zc comprises contacting the HCFC-262fc with a strong base in a solvent;
optionally wherein the strong base is selected from the group consisting of hydroxides, alkoxides, metal amides, metal hydrides, metal dialkylamides and arylamines;
optionally wherein the strong base is selected from the group consisting of alkoxides selected from lithium, sodium and potassium salts of methyl, ethyl and t-butyl oxides; metal amides selected from sodium amide, potassium amide and lithium amide; metal hydrides selected from sodium hydride, potassium hydride and lithium hydride; and metal dialkylamides selected from lithium, sodium, and potassium salts of methyl, ethyl, n-propyl, iso-propyl, n-butyl, tert-butyl, trimethylsilyl and cyclohexyl substituted amides.

13. A system comprising:
a self-contained source of 1,1,1,3-tetrachloropropane (HCC-250fb);
a pressurized source container of hydrogen and/or hydrogen fluoride;
at least first, second and third serially arranged reactors, the first and second reactors respectively producing first and second intermediate product mixtures, and the third reactor producing a final product mixture comprising 1,1-difluoropropene (HFO-1252zc),
wherein the first reactor contains a flowthrough bed of fluorination catalyst and is configured for a liquid phase fluorination reaction to convert the HCC-250fb to 1,3-dichloro-1,1-difluoropropane (HCFC-252fc), the second reactor contains a flowthrough bed of hydrogenation catalyst and is configured for a liquid phase or vapor phase hydrogenation reaction to convert the HCFC-252fc to 1-chloro-1,1-difluoropropane (HCFC-262fc), and the third reactor is configured for a vapor phase reaction to convert a hydrochlorofluorocarbon produced in the second reactor to HFO-1252zc.

14. The system of claim 13, further comprising one or more separator systems;
the system optionally further comprising a first discharge line configured to supply the first intermediate product mixture from the first reactor to a first separator system, the first separator system being configured to recover a first intermediate product and convey the first intermediate product to the second reactor; and
the system optionally further comprising a second discharge line configured to supply the second intermediate product mixture from the second reactor to a second separator system, the second separator system being configured to recover the hydrochlorofluorocarbon and convey the hydrochlorofluorocarbon to the third reactor.

15. A process for producing HFO-1252zc comprising using the system of claim 13 or 14.
